(19) Europäisches Patentamt
European Patent Office
Office européen des brevets

(11) Veröffentlichungsnummer : **0 344 232 B1**

# (12) EUROPÄISCHE PATENTSCHRIFT

(45) Veröffentlichungstag der Patentschrift :
**24.03.93 Patentblatt 93/12**

(51) Int. Cl.$^5$ : **C07D 239/54,** A01N 43/54

(21) Anmeldenummer : **88908651.8**

(22) Anmeldetag : **21.10.88**

(86) Internationale Anmeldenummer :
**PCT/CH88/00197**

(87) Internationale Veröffentlichungsnummer :
**WO 89/03825 05.05.89 Gazette 89/10**

## (54) 3-Aryluracile zur Unkrautbekämpfung.

Die Akte enthält technische Angaben, die nach dem Eingang der Anmeldung eingereicht wurden und die nicht in dieser Patentschrift enthalten sind.

(30) Priorität : **22.10.87 CH 4132/87**

(43) Veröffentlichungstag der Anmeldung :
**06.12.89 Patentblatt 89/49**

(45) Bekanntmachung des Hinweises auf die Patenterteilung :
**24.03.93 Patentblatt 93/12**

(84) Benannte Vertragsstaaten :
**AT BE CH DE FR GB IT LI LU NL SE**

(56) Entgegenhaltungen :
**EP-A- 0 195 346**

(73) Patentinhaber : **CIBA-GEIGY AG**
**Klybeckstrasse 141**
**CH-4002 Basel (CH)**

(72) Erfinder : **SUCHY, Milos**
**Grossplatzstrasse 3**
**CH-8122 Pfaffhausen (CH)**
Erfinder : **WENGER, Jean**
**Brunnenwiesenstrasse 1**
**CH-8610 Uster (CH)**
Erfinder : **WINTERNITZ, Paul**
**Am Pfisterhölzli 50**
**CH-8606 Greifensee (CH)**
Erfinder : **ZELLER, Martin**
**Zwinggartenstrasse 51**
**CH-8600 Dübendorf (CH)**

EP 0 344 232 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft heterocyclische Verbindungen, und zwar 3-Aryluracile der allgemeinen Formel

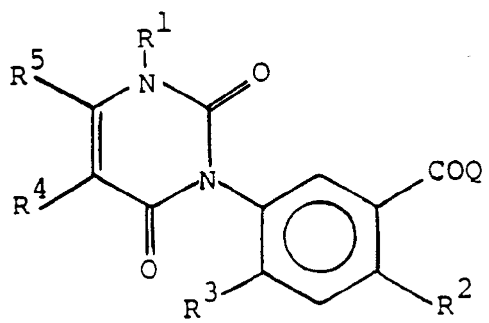

I

worin

$R^1$ Wasserstoff, $C_{1-4}$-Alkyl, $C_{1-4}$-Halogenalkyl, $C_{2-5}$-Alkenyl oder $C_{3-5}$-Alkinyl,

$R^2$ Halogen oder Cyano,

$R^3$ Wasserstoff oder Halogen,

$R^4$ Wasserstoff, Halogen oder $C_{1-4}$-Alkyl,

$R^5$ $C_{1-4}$-Alkyl oder, im Falle, dass $R^1$ verschieden von $C_{1-4}$-Halogenalkyl ist, auch $C_{1-4}$-Halogenalkyl

und

Q eine der Gruppen (a) bis (d) (falls $R^1$ verschieden von Wasserstoff ist) bzw. eine Gruppe (c) oder (d) (falls $R^1$ für Wasserstoff steht) bedeuten

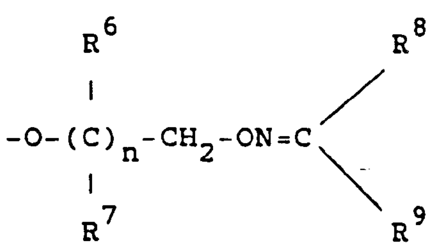

(a)

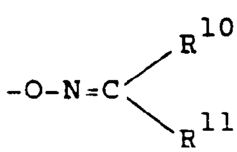

(b)

$-O-R^{12}$ (c)

$-S-R^{13}$ (d)

worin

$R^6$ Wasserstoff oder $C_{1-4}$-Alkyl,

$R^7$ Wasserstoff, $C_{1-4}$-Alkyl, Phenyl oder Benzyl,

$R^8$ Wasserstoff oder $C_{1-6}$-Alkyl,

$R^9$ $C_{1-6}$-Alkyl, $C_{3-6}$-Cycloalkyl oder Phenyl,

oder

$R^8$ und $R^9$ zusammen Tri-, Tetra-, Penta- oder Hexamethylen,

n 0 oder 1,

$R^{10}$ $C_{1-6}$-Alkyl, $C_{1-6}$-Alkylthio, $C_{2-7}$-Alkoxycarbonyl oder $C_{2-7}$-Alkoxycarbonyl-$C_{1-4}$-alkyl,

$R^{11}$ $C_{1-6}$-Alkyl, Trifluormethyl, $C_{1-6}$-Alkoxy-$C_{1-4}$-alkyl, $C_{2-7}$-Alkoxycarbonyl-$C_{1-4}$-alkyl, Di($C_{2-7}$-alkoxycarbonyl)-$C_{1-4}$-alkyl, $C_{3-6}$-Cycloalkyl, $C_{1-6}$-Alkoxy, $C_{1-6}$-Alkylthio, $C_{2-7}$-Alkanoyl, $C_{2-7}$-Alkoxycarbonyl, Phenyl oder

2-Furyl,
oder

$R^{10}$ und $R^{11}$ zusammen mit dem Kohlenstoffatom, an das sie geknüpft sind, einen gegebenenfalls mit 1 bis 3 $C_{1-4}$-Alkylgruppen substituierten Cyclopentan- oder Cyclohexanring,

$R^{12}$ $C_{1-8}$-Halogenalkyl, $C_{3-5}$-Halogenalkenyl oder $C_{3-5}$-Halogenalkinyl,

und

$R^{13}$ Wasserstoff, $C_{1-8}$-Alkyl, $C_{3-8}$-Alkenyl, $C_{3-8}$-Alkinyl, $C_{2-6}$-Alkoxyalkyl oder gegebenenfalls mit 1 bis 3 $C_{1-4}$-Alkylgruppen substituiertes $C_{3-8}$-Cycloalkyl, Phenyl oder Benzyl bedeuten,

und die Enoläther derjenigen Verbindungen der Formel I, in denen $R^1$ $C_{1-4}$-Alkyl, $C_{2-5}$-Alkenyl oder $C_{3-5}$-Alkinyl und Q eine Gruppe (b), (c) oder (d) bedeuten, sowie Salze derjenigen Verbindungen der Formel I bzw. Enoläther, in denen $R^1$ und/oder $R^{13}$ Wasserstoff bedeutet.

Unter den obenerwähnten Enoläthern sind also die Verbindungen der Formel

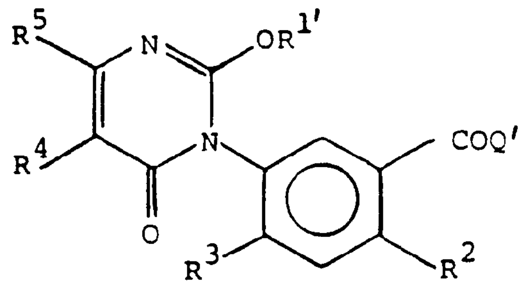

Ia

und auch die Verbindungen der Formel

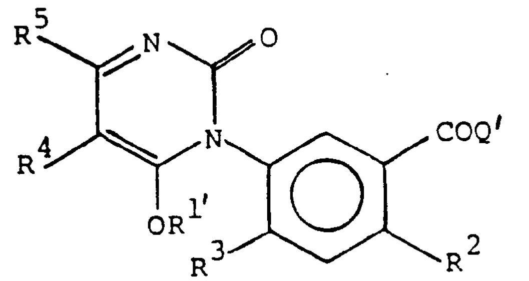

Ib

worin $R^2$, $R^3$, $R^4$ und $R^5$ die oben angegebenen Bedeutungen besitzen, $R^{1'}$ $C_{1-4}$-Alkyl, $C_{2-5}$-Alkenyl oder $C_{3-5}$-Alkinyl bedeutet und Q' eine Gruppe (b), (c) oder (d) bedeutet,
zu verstehen. Bei deren Salzen handelt es sich um Salze derjenigen Enoläther Ia und Ib, in denen Q' Mercapto bedeutet, also die Gruppe (d), in der $R^{13}$ Wasserstoff bedeutet.

Die erfindungsgemässen Verbindungen, also die Verbindungen der Formel I und deren Enoläther und die Salze davon, sind herbizid wirksam und eignen sich als Wirkstoffe von Unkrautbekämpfungsmitteln. Somit umfasst die Erfindung auch Unkrautbekämpfungsmittel, welche erfindungsgemässe Verbindungen als Wirkstoffe enthalten, Verfahren zur Herstellung dieser Verbindungen sowie die Verwendung der Verbindungen bzw. Mittel zur Bekämpfung von Unkräutern.

In der obigen Formel I umfasst "Halogen" Fluor, Chlor, Brom und Jod. Die Alkyl-, Alkenyl- und Alkinylreste können geradkettig oder verzweigt sein, wobei dies auch für den bzw. jeden Alkyl-, Alkenyl- oder Alkinylteil der Halogenalkyl-, Alkylthio-, Alkoxycarbonyl-, Alkoxycarbonylalkyl-, Alkoxyalkyl-, Dialkoxycarbonylalkyl-, Alkoxy-, Alkanoyl-, Halogenalkenyl- und Halogenalkinylgruppen gilt. Eine Halogenalkyl-, Halogenalkenyl- oder Halogenalkinylgruppe kann ein oder mehrere (gleiche oder verschiedene) Halogenatome aufweisen. Ebenfalls kann eine mehrfach mit $C_{1-4}$-Alkylgruppen substituierte Cyclopentyl-, Cyclohexyl-, $C_{3-8}$-Cycloalkyl-, Phenyl oder Benzylgruppe gleiche oder verschiedene Alkylsubstituenten aufweisen.

Bei den Salzen der Verbindungen der Formel I und der Enoläther der Formeln Ia und Ib handelt es sich insbesondere um Alkalimetallsalze, z.B. Natrium- und Kaliumsalze; Erdalkalimetallsalze, z.B. Calcium- und Magnesiumsalze; Ammoniumsalze, d.h. unsubstituierte Ammoniumsalze und mono- oder mehrfach substituierte Ammoniumsalze, z.B. Triäthylammonium- und Methylammoniumsalze, sowie um Salze mit anderen organischen Basen, z.B. mit Pyridin.

Das Vorhandensein mindestens eines asymmetrischen Kohlenstoffatoms in den Verbindungen I und in deren Enoläthern Ia und Ib hat zur Folge, dass die Verbindungen in optisch isomeren Formen auftreten können. Durch das Vorliegen einer allfälligen aliphatischen C=C- oder C=N-Doppelbindung kann auch geometrische Isomerie auftreten. Zudem kann bei denjenigen Verbindungen der Formel I, in denen $R^1$ Wasserstoff bedeutet, Keto-Enol-Tautomerie [-NH-CO-. $\rightleftarrows$ -N=C(OH)-] auftreten. Die Formel I soll all diese möglichen isomeren Formen sowie Gemische davon umfassen.

Bedeutet $R^1$ oder $R^{13}$ jeweils Alkenyl oder Alkinyl, ist dieser Rest vorzugsweise Allyl bzw. Propargyl. Eine allfällig vorhandene Halogenalkylgruppe ist vorzugsweise $C_{1-4}$-Fluoralkyl, insbesondere im Falle von $R^1$ Difluormethyl und im Falle von $R^5$ Trifluormethyl oder Pentafluoräthyl. Im allgemeinen ist ein allfällig vorkommendes Halogenatom vorzugsweise Fluor, Chlor oder Brom.

Eine besondere Gruppe von Verbindungen der Formel I besteht aus denjenigen Verbindungen I, in denen $R^1$ $C_{1-4}$-Alkyl oder $C_{1-4}$-Halogenalkyl, $R^2$ Halogen, $R^4$ Wasserstoff und Q eine Gruppe (a), in der $R^6$ Wasserstoff, $R^7$ Wasserstoff, $C_{1-4}$-Alkyl oder Phenyl, $R^8$ $C_{1-6}$-Alkyl, $R^9$ $C_{1-6}$-Alkyl, $C_{3-6}$-Cycloalkyl oder Phenyl, oder $R^8$ und $R^9$ zusammen $-(CH_2)_{3-6}-$, und n 1 bedeuten, oder Q eine Gruppe (b), in der $R^{10}$ $C_{1-6}$-Alkyl oder $C_{2-7}$-Alkoxycarbonyl-$C_{1-4}$-alkyl und $R^{11}$ $C_{1-6}$-Alkyl, Trifluormethyl, $C_{1-6}$-Alkoxy-$C_{1-4}$-alkyl, $C_{2-7}$-Alkoxycarbonyl-$C_{1-4}$-alkyl, $C_{3-7}$-Cycloalkyl, $C_{1-6}$-Alkoxy, $C_{2-7}$-Alkanoyl, $C_{2-7}$-Alkoxycarbonyl, Phenyl oder 2-Furyl, oder $CR^{10}R^{11}$ einen Cyclopentan- oder Cyclohexanring bedeuten, oder Q eine Gruppe (c), wie oben definiert, bedeutet, oder Q eine Gruppe (d), in der $R^{13}$ $C_{1-8}$-Alkyl, $C_{3-8}$-Alkenyl, $C_{3-8}$-Cycloalkyl, Phenyl oder Benzyl bedeutet, bedeuten, und aus deren Enoläthern.

Unabhängig voneinander bedeuten $R^1$ vorzugsweise geradkettiges $C_{1-4}$-Alkyl, insbesondere Methyl, oder Difluormethyl; $R^2$ vorzugsweise Chlor oder Brom; $R^3$ vorzugsweise Wasserstoff oder Fluor; $R^4$ vorzugsweise Wasserstoff, Fluor oder Methyl; und $R^5$ vorzugsweise Methyl, Trifluormethyl oder Pentafluoräthyl.

Besonders bevorzugte einzelne erfindungsgemässe Verbindungen sind:

2-Chlor-5-[3,6-dihydro-2,6-dioxo-3-methyl-4-trifluormethyl-1(2H)-pyrimidinyl]-4-fluorbenzoesäure-{2-[(isopropylidenamino)oxy]-äthyl}ester,

2-Chlor-5-[3,6-dihydro-2,6-dioxo-3-methyl-4-trifluormethyl-1(2H)-pyrimidinyl]-4-fluorbenzoesäure-{2-[(cyclohexylidenamino)oxy]-äthyl}ester,

2-Chlor-5-[3,6-dihydro-2,6-dioxo-3-methyl-4-trifluormethyl-1(2H)-pyrimidinyl]-4-fluorbenzoesäure-[α-{[(isopropylidenamino)oxy]methyl}-benzyl]ester,

1-Cyclopropyl-1-äthanon-O-{2-chlor-5-[3,6-dihydro-2,6-dioxo-3-methyl-4-trifluormethyl-1(2H)-pyrimidinyl]-4-fluorbenzoyl}oxim,

1-[4-Chlor-2-fluor-5-{[(isopropylidenamino)oxy]carbonyl}-phenyl]-3-methyl-4-trifluormethyl-2,6(1H, 3H)-pyrimidindion,

2-Furylmethylketon-O-{2-chlor-5-[3,6-dihydro-2,6-dioxo-3-methyl-4-trifluormethyl-1(2H)-pyrimidinyl]-4-fluorbenzoyl}oxim,

2,3-Butandion-2-[O-{2-chlor-5-[3,6-dihydro-2,6-dioxo-3-methyl-4-trifluormethyl-1(2H)-pyrimidinyl]-4-fluorbenzoyl}]oxim,

2-Chlor-4-fluor-5-[2-methoxy-6-oxo-4-trifluormethyl-1(6H)-pyrimidinyl}-benzoesäure-(2-fluor-1-fluormethyläthyl)ester,

2-Chlor-4-fluor-5-[2-methoxy-6-oxo-4-trifluormethyl-1(6H)-pyrimidinyl]-benzoesäure-(2-chloräthyl)ester,

2-Chlor-5-[3,6-dihydro-2,6-dioxo-3-methyl-4-trifluormethyl-1(2H)-pyrimidinyl]-4-fluorbenzoesäure-(2-fluoräthyl)ester,

2-Chlor-4-fluor-5-[2-methoxy-6-oxo-4-trifluormethyl-1(6H)-pyrimidinyl]-benzoesäure-(3-chlor-2-butenyl) ester,

2-Chlor-5-[3,6-dihydro-2,6-dioxo-3-methyl-4-trifluormethyl-1(2H)-pyrimidinyl]-4-fluorbenzoesäure-(3-chlor-2-butenyl)ester,

2-Chlor-4-fluor-5-[2-methoxy-6-oxo-4-trifluormethyl-1(6H)-pyrimidinyl]-benzoesäure-(2-fluoräthyl)ester,

2-Chlor-5-[3,6-dihydro-2,6-dioxo-3-methyl-4-trifluormethyl-1(2H)-pyrimidinyl]-4-fluor-thiobenzoesäure-S-isopropylester,

2-Chlor-5-[3,6-dihydro-2,6-dioxo-3-methyl-4-trifluormethyl-1(2H)-pyrimidinyl]-4-fluorthiobenzoesäure-S-äthylester,

2-Chlor-5-[3,6-dihydro-2,6-dioxo-3-methyl-4-trifluormethyl-1(2H)-pyrimidinyl]-4-fluorthiobenzoesäure-S-(n-butyl)ester,

2-Chlor-5-[3,6-dihydro-2,6-dioxo-3-methyl-4-trifluormethyl-1(2H)-pyrimidinyl]-4-fluorthiobenzoesäure-S-allylester,

2-Chlor-5-[3,6-dihydro-2,6-dioxo-3-methyl-4-trifluormethyl-1(2H)-pyrimidinyl]-4-fluorthiobenzoesäure-S-cyclohexylester,

2-Chlor-5-[3,6-dihydro-2,6-dioxo-3-methyl-4-trifluormethyl-1(2H)-pyrimidinyl]-4-fluorthiobenzoesäure-S-phenylester,

2-Chlor-5-[3,6-dihydro-2,6-dioxo-3-methyl-4-trifluormethyl-1(2H)-pyrimidinyl]-4-fluorthiobenzoesäure-S-benzylester und

2-Chlor-5-[3,6-dihydro-3,4-dimethyl-2,6-dioxo-1(2H)-pyrimidinyl]-4-fluorthiobenzoesäure-S-isopropylester.

Weitere Vertreter von Verbindungen der Formel I sind diejenigen Verbindungen I, in denen $R^1$ und $R^5$ je Methyl, $R^2$ Chlor, $R^3$ Fluor, $R^4$ Wasserstoff, Q eine Gruppe (a), $R^6$ und $R^7$ Wasserstoff, n 1 und $CR^8R^9$ sek. Butyliden, 1-Isopropyläthyliden, 1-Aethylpropyliden, 1-Isopropylpropyliden, 1-Propyläthyliden, 1-Propylpropyliden, 1-Propylbutyliden, 1-Butyläthyliden, 1-Cyclopropyläthyliden, 1-Phenyläthyliden, Cyclopentyliden oder Cyclohexyliden bedeuten; der 2-Chlor-5-[3,6-dihydro-3,4-dimethyl-2,6-dioxo-1(2H)-pyrimidinyl]-4-fluorbenzoesäure-{1-methyl-2-[(isopropylidenamino)oxy]-äthyl}ester; der 5-[4-Aethyl-3,6-dihydro-2,6-dioxo-3-methyl-1(2H)-pyrimidinyl]-2-chlor-4-fluorbenzoesäure-{2-[(isopropylidenamino)oxy]-äthyl}ester; der 2-Chlor-5-[3,6-dihydro-3,4-dimethyl-2,6-dioxo-1(2H)-pyrimidinyl]-benzoesäure-{2-[(isopropylidenamino)oxy]-äthyl}ester; der 2-Chlor-5-[3-difluormethyl-3,6-dihydro-2,6-dioxo-4-methyl-1(2H)-pyrimidinyl]-4-fluorbenzoesäure-{2-[(sek. butylidenamino)oxy]-äthyl}ester; der 2-Chlor-5-[3-difluormethyl-3,6-dihydro-2,6-dioxo-4-methyl-1(2H)-pyrimidinyl]-4-fluorbenzoesäure-{2-[(1-äthylpropylidenamino)oxy]-äthyl}ester; der 2-Chlor-5-[3-difluormethyl-3,6-dihydro-2,6-dioxo-4-methyl-1(2H)-pyrimidinyl]-benzoesäure-{2-[(isopropylidenamino)oxy]-äthyl}ester; diejenigen Verbindungen I, in denen $R^1$ und $R^5$ je Methyl, $R^2$ Chlor, $R^3$ Fluor, $R^4$ Wasserstoff, Q eine Gruppe (b) und $CR^{10}R^{11}$ sek. Butyliden, 1-Isopropyläthyliden, 1-Aethylpropyliden, 1-Isopropylpropyliden, 1-Propyläthyliden, 1-Propylpropyliden, 1-Propylbutyliden, 1-Butyläthyliden, 1-Cyclopropyläthyliden, 1-Phenyläthyliden, Cyclopentyliden oder Cyclohexyliden bedeuten; das Aceton-O-{5-[4-Aethyl-3,6-dihydro-2,6-dioxo-3-methyl-1(2H)-pyrimidinyl]-2-chlor-4-fluorbenzoyl}oxim; das Aceton-O-{2-chlor-5-[3,6-dihydro-3,4-dimethyl-2,6-dioxo-1(2H)-pyrimidinyl]-benzoyl}oxim; das 1,1,1-Trifluoraceton-O-{2-chlor-5-[3,6-dihydro-3,4-dimethyl-2,6-dioxo-1(2H)-pyrimidinyl]-4-fluorbenzoyl}oxim; das Methylacetat-O-{2-chlor-5-[3,6-dihydro-3,4-dimethyl-2,6-dioxo-1(2H)-pyrimidinyl]-4-fluorbenzoyl}oxim; das Aceton-O-{2-chlor-5-[3-difluormethyl-3,6-dihydro-2,6-dioxo-4-methyl-1(2H)-pyrimidinyl]-4-fluorbenzoyl}oxim; das 2-Butanon-O-{2-chlor-5-[3-difluormethyl-3,6-dihydro-2,6-dioxo-4-methyl-1(2H)-pyrimidinyl]-4-fluorbenzoyl}oxim; das Aceton-O-{2-chlor-5-[3-difluormethyl-3,6-dihydro-2,6-dioxo-4-methyl-1(2H)-pyrimidinyl]-benzoyl}oxim; diejenigen Verbindungen I, in denen $R^1$ und $R^5$ je Methyl, $R^2$ Chlor, $R^3$ Fluor, $R^4$ Wasserstoff, Q eine Gruppe (c) und $R^{12}$ 2-Chlor-1-methyläthyl, 2-Fluoräthyl, 2-Bromäthyl, Di(chlormethyl)methyl, Di(fluormethyl)methyl, 3,3-Dibrom-2-propenyl, 3-Chlor-2-butenyl, 4-Chlor-2-butenyl, 4-Brom-2-butenyl, 2,2,2-Trifluoräthyl, 4-Chlorbutyl, 2-Chlor-2-propenyl, Di(trifluormethyl)methyl, 3-Chlor-2-propinyl oder 3-Chlor-2-butenyl bedeuten; der 2-Chlor-5-[3,6-dihydro-3,4-dimethyl-2,6-dioxo-1(2H)-pyrimidinyl]-benzoesäure-(2-chloräthyl)ester; der 5-[4-Aethyl-3,6-dihydro-2,6-dioxo-3-methyl-1(2H)-pyrimidinyl]-2-chlor-4-fluorbenzoesäure-(2-chloräthyl)ester; diejenigen Verbindungen I, in denen $R^1$ Methyl, $R^2$ Chlor, $R^3$ Fluor, $R^4$ Wasserstoff, $R^5$ Trifluormethyl, Q eine Gruppe (d) und $R^{13}$ n-Propyl, sek. Butyl oder Cyclopentyl bedeuten; diejenigen Verbindungen I, in denen $R^1$ und $R^5$ je Methyl, $R^2$ Chlor, $R^3$ Fluor, $R^4$ Wasserstoff, Q eine Gruppe (d) und $R^{13}$ Aethyl, Isopropyl, Allyl, Cyclopentyl oder Cyclohexyl bedeuten; der 2-Chlor-5-[3,6-dihydro-2,6-dioxo-3-methyl-4-trifluormethyl-1(2H)-pyrimidinyl]-thiobenzoesäure-isopropylester; der 2-Chlor-5-[3,6-dihydro-2,6-dioxo-3-methyl-4-trifluormethyl-1(2H)-pyrimidinyl]-thiobenzoesäure-allylester; und diejenigen Verbindungen I, in denen $R^1$ Difluormethyl, $R^2$ Chlor, $R^3$ Fluor, $R^4$ Wasserstoff, $R^5$ Methyl, Q eine Gruppe (d) und $R^{13}$ Aethyl, tert. Butyl, Allyl, Cyclopentyl oder Cyclohexyl bedeuten.

Das erfindungsgemässe Verfahren zur Herstellung der Verbindungen der Formel I und ihrer Enoläther sowie Salze ist dadurch gekennzeichnet, dass man

a) zwecks Herstellung derjenigen Verbindungen der Formel I, in denen $R^1$ Wasserstoff und Q eine Gruppe (c) oder (d) bedeuten, sowie gewünschtenfalls von Metallsalzen dieser Verbindungen, eine Verbindung der allgemeinen Formel

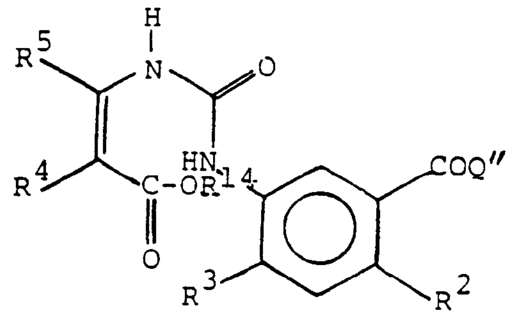

II

worin $R^2$, $R^3$, $R^4$ und $R^5$ die oben angegebenen Bedeutungen besitzen, Q" eine Gruppe (c) oder (d), wie diese oben definiert ist, bedeutet und $R^{14}$ nieder Alkyl, vorzugsweise $C_{1-4}$-Alkyl, bedeutet, einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,

b) zwecks Herstellung derjenigen Verbindungen der Formel I, in denen $R^1$ $C_{1-4}$-Alkyl, $C_{1-4}$-Halogenalkyl, $C_{2-5}$-Alkenyl oder $C_{3-5}$-Alkinyl und Q eine der Gruppen (a) bis (d) bedeuten, und der Enoläther dieser Verbindungen eine Benzoesäure der allgemeinen Formel

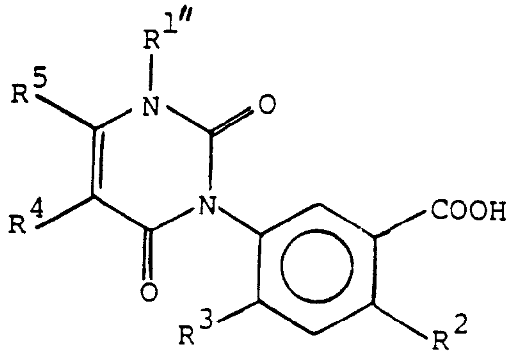

III

worin $R^2$, $R^3$, $R^4$ und $R^5$ die oben angegebenen Bedeutungen besitzen und $R^{1''}$ $C_{1-4}$-Alkyl, $C_{1-4}$-Halogenalkyl, $C_{2-5}$-Alkenyl oder $C_{3-5}$-Alkinyl bedeutet,

oder ein reaktionsfähiges Derivat dieser Benzoesäure mit einer Hydroxy- bzw. Mercaptoverbindung der allgemeinen Formel

H-Q IV

worin Q die oben angegebene Bedeutung besitzt, oder mit einem reaktionsfähigen Derivat dieser Hydroxy- bzw. Mercaptoverbindung verestert bzw. einen Enoläther dieser Benzoesäure, und zwar der allgemeinen Formel

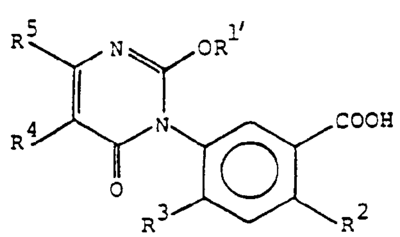

oder

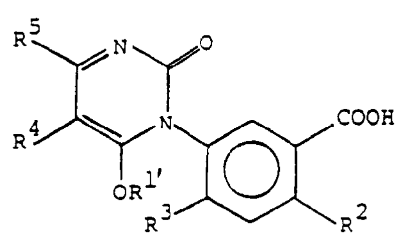

III a

III b

worin $R^{1'}$, $R^2$, $R^3$, $R^4$ und $R^5$ die oben angegebenen Bedeutungen besitzen,

oder ein reaktionsfähiges Derivat dieses Enoläthers mit einer Hydroxy- bzw. Mercaptoverbindung der allgemeinen Formel

H-Q′ IV′

worin Q′ die oben angegebene Bedeutung besitzt,

oder mit einem reaktionsfähigen Derivat dieser Hydroxy- bzw. Mercaptoverbindung verestert,

c) zwecks Herstellung derjenigen Verbindungen der Formel I, in denen $R^1$ $C_{1-4}$-Alkyl, $C_{1-4}$-Halogenalkyl, $C_{2-5}$-Alkenyl oder $C_{3-5}$-Alkinyl und Q eine Gruppe (c) bedeuten, einen Benzoesäureester der allgemeinen Formel

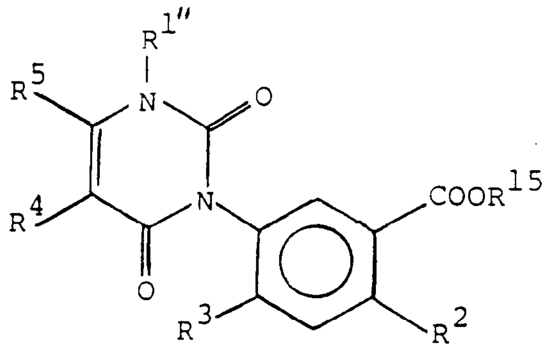

V

worin $R^{1''}$, $R^2$, $R^3$, $R^4$ und $R^5$ die oben angegebenen Bedeutungen besitzen und $R^{15}$ $C_{1-6}$-Alkyl, $C_{2-4}$-Alkenyl, $C_{2-4}$-Alkinyl oder $C_{2-6}$-Alkoxyalkyl bedeutet,

einer Umesterungsreaktion mit einer Hydroxyverbindung der allgemeinen Formel

H-Q‴ IV″

worin Q‴ eine Gruppe (c) bedeutet,

unterwirft, wobei das Reagens IV″ höhersiedend ist als die sich bildende Hydroxyverbindung $R^{15}OH$, oder

d) zwecks Herstellung derjenigen Enoläther der Formel Ia und Ib, in denen Q′ eine Gruppe (c) oder (d) bedeutet, ein Pyrimidinonderivat der allgemeinen Formel

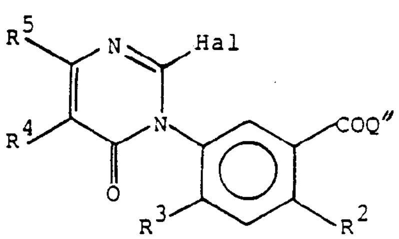

oder

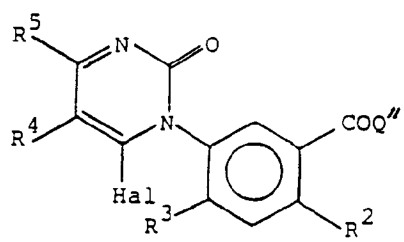

VIa VIb

worin $R^2$, $R^3$, $R^4$, $R^5$ und Q″ die oben angegebenen Bedeutungen besitzen und Hal Chlor oder Brom bedeutet,

mit der deprotonierten Form eines Alkanols, Alkenols oder Alkinols $R^{1'}$ OH, worin $R^{1'}$ die oben angegebene Bedeutung besitzt, behandelt,

und gewünschtenfalls eine so erhaltene Verbindung der Formel I, in der $R^1$ und/oder $R^{13}$ Wasserstoff bedeutet, in ein Salz überführt.

Die Cyclisierung nach Verfahrensvariante a) kann zweckmässigerweise durchgeführt werden, indem man die Verbindung der Formel II in einem inerten protischen organischen Lösungsmittel, wie einem Alkohol, z.B. Methanol, Aethanol oder Isopropanol; einem inerten aprotischen organischen Lösungsmittel, wie einem aliphatischen oder cyclischen Aether, z.B. 1,2-Dimethoxyäthan, Tetrahydrofuran oder Dioxan, oder einem Aromaten, z.B. Benzol oder Toluol; einem inerten aprotischen, polaren organischen Lösungsmittel, z.B. Dimethylformamid oder Dimethylsulfoxid, wobei solche Lösungsmittel gegebenenfalls im Zweiphasen-Gemisch mit einem Kohlenwasserstoff, z.B. n-Hexan oder Toluol, verwendet werden können; oder Wasser mit einer Base bei Temperaturen zwischen -78°C und der Rückflusstemperatur des Reaktionsgemisches behandelt. Als Basen kommen vorzugsweise Natriumalkoholate, Alkalimetallhydroxide, insbesondere Natriumhydroxid und Kaliumhydroxid, Alkalimetallcarbonate, insbesondere Natriumcarbonat und Kaliumcarbonat, und Natriumhydrid in Betracht. Bei der Verwendung von Natriumhydrid als Base ist das Lösungsmittel vorzugsweise ein aliphatischer oder cyclischer Aether, Dimethylformamid oder Dimethylsulfoxid, wobei jedes dieser Lösungsmittel im Gemisch mit Toluol verwendet werden kann.

Nach Beendigung der Cyclisierung liegt das Produkt im Falle der Verwendung einer der obenerwähnten Basen oder dergleichen in Form des entsprechenden Alkalimetallsalzes vor. Dieses kann in an sich bekannter Weise isoliert und gereinigt werden, oder man kann das Gemisch ansäuern, um die jeweilige Verbindung der Formel I an sich zu isolieren. Zu diesem Zwecke verwendet man vorzugsweise eine Mineralsäure, wie Salzsäure, oder eine starke organische Säure, wie Essigsäure oder p-Toluolsulfonsäure.

Bei der Verfahrensvariante b) handelt es sich um eine Veresterung der Benzoesäure III oder des Enoläthers IIIa oder IIIb bzw. eines reaktionsfähigen Derivats der Benzoesäure oder des Enoläthers, die nach an sich bekannten Methoden durchgeführt werden kann. So wird beispielsweise ein Salz einer Benzoesäure der Formel III oder des Enoläthers IIIa oder IIIb mit einem Halogenid, insbesondere Chlorid, Bromid oder Jodid, oder dem Sulfat, Mesylat oder Tosylat der Hydroxy- oder Mercaptoverbindung IV resp. IV′ in einem inerten Verdünnungsmittel bei Temperaturen zwischen dem Raumtemperatur und 100°C, z.B. bei der Rückflusstemperatur des Reaktionsgemisches, vorzugsweise im Temperaturbereich von 40°C bis 70°C, umgesetzt. Es kommen als Salze der Benzoesäure der Formel III oder des entsprechenden Enoläthers insbesondere Alkalimetallsalze, z.B. das Lithium-, Natrium- und Kaliumsalz, Erdalkalimetallsalze, z.B. das Calcium-, Magnesium, und Bariumsalz, und Salze mit organischen Basen, wie tertiäre Amine, z.B. Triäthylamin, 1,5-Diaza-bicyclo[4,3,0]non-5-en, 1,8-Diaza-bicyclo[5,4,0]undec-7-en und 1,4-Diaza-bicyclo[2,2,2]octan, in Frage, wobei die Alkalimetallsalze, insbesondere das Natriumsalz und das Kaliumsalz, bevorzugt sind. Als Verdünnungsmittel werden vorzugsweise inerte organische Lösungsmittel, wie niedere Alkanole, z.B. Aethanol, aliphatische und cyclische Aether, z.B. Diäthyläther, Tetrahydrofuran und Dioxan, Ketone, z.B. Aceton und 2-Butanon, Dimethylformamid, Dimethylsulfoxid und Hexamethylphosphorsäuretriamid verwendet. Das Salz kann in situ hergestellt werden, indem man die Säure mit einer geeigneten anorganischen Base, z.B. einem Alkalimetall- oder Erdalkalimetallcarbonat oder -hydrogencarbonat, oder einem Alkalimetallhydrid, bzw. organischen Base, zum Salz umsetzt und dies anschliessend im gleichen Reaktionsmedium mit dem zweiten Reaktionspartner reagieren lässt.

Im Falle der Verwendung eines Säurehalogenids der Benzoesäure der Formel III oder des Enoläthers IIIa oder IIIb als reaktionsfähigen Derivats wird dies zweckmässigerweise mit der Hydroxy- oder Mercaptoverbindung IV resp. IV′ in einem inerten organischen Lösungsmittel, wie einem aliphatischen oder cyclischen Aether, z.B. Diäthyläther, Tetrahydrofuran oder Dioxan, einem aliphatischen oder aromatischen Kohlenwasserstoff, z.B. n-Hexan, Benzol oder Toluol, oder einem halogenierten, insbesondere chlorierten, Kohlenwasserstoff, z.B. Methylenchlorid, Chloroform oder Tetrachlorkohlenstoff, bei Temperaturen von etwa -20°C bis 100°C, vorzugsweise von 0°C bis 50°C, umgesetzt. Zudem wird zweckmässigerweise in Gegenwart eines säurebindenden Mittels gearbeitet, wie einer organischen Base, z.B. Triäthylamin, Pyridin, 1,5-Diaza-bicyclo[4,3,0]non-5-en, 1,8-Diaza-bicyclo[5,4,0]undec-7-en oder 1,4-Diaza-bicyclo[2,2,2]octan. Das Säurehalogenid ist vorzugsweise das Säurechlorid.

Als weitere in Frage kommende reaktionsfähige Derivate der Benzoesäure III oder des Enoläthers IIIa oder IIIb seien der entsprechende O-Acyl-1,3-dicyclohexylisoharnstoff, der zweckmässigerweise in situ durch Behandlung der Benzoesäure III bzw. des Enoläthers IIIa oder IIIb mit Dicyclohexylcarbodiimid gebildet wird, und das entsprechende N-Acylimidazol oder Säureanhydrid genannt. Solche Derivate können wie das Säurehalogenid mit den Hydroxy- oder Mercaptoverbindungen IV bzw. IV′ umgesetzt werden, um zu den gewünschten Benzoesäureestern zu gelangen. In diesen Fällen erübrigt sich jedoch die Verwendung eines säurebindenden Mittels.

Die Umsetzung nach Verfahrensvariante c) kann zweckmässigerweise durchgeführt werden, indem man den Benzoesäureester der Formel V in überschüssiger Hydroxyverbindung der Formel IV″ in Gegenwart eines basischen Katalysators, z.B. Natriumcyanid, erhitzt, und zwar vorzugsweise bei der Rückflusstemperatur des Reaktionsgemisches. Im Laufe der Reaktion wird der Rest $R^{15}$ des Benzoesäureesters V durch die Gruppe Q‴ (c) der Hydroxyverbindung IV″ ersetzt, wobei das niedriger siedende Alkanol, Alkenol bzw. Alkinol $R^{15}OH$ freigesetzt wird.

Die in der Verfahrensvariante d) verwendete deprotonierte Form des Alkanols, Alkenols oder Alkinols $R^{1'}$ OH entsteht zweckmässigerweise entweder durch Einsatz der Hydroxyverbindung $R^{1'}$ OH in Gegenwart einer organischen Base, insbesondere einer organischen tertiären Base, z.B. Triäthylamin oder Pyridin, oder des entsprechenden Metallalkanolats, -alkenolats oder alkinolats $R^{1'}$ $O^{\ominus}M^{\oplus}$, worin $M^{\oplus}$ ein Aequivalent eines Metallions bedeutet, wie eines Alkalimetallions, z.B. Natrium oder Kalium, oder eines Erdalkalimetallions, z.B. Calcium oder Magnesium. Das Natriumion ist das bevorzugte Metallion.

Die Umsetzung erfolgt zweckmässigerweise in einem Ueberschuss an der entsprechenden Hydroxyverbindung $R^{1'}$ OH als Verdünnungsmittel und bei Temperaturen zwischen 0°C und 50°C, vorzugsweise bei Raumtemperatur.

Sofern sie nicht unmittelbar durch die oben beschriebene unter basischen Bedingungen durchgeführte Cyclisierung herstellbar sind, können die gewünschten Salze der Verbindungen der Formel I, in denen $R^1$ und-/oder $R^{13}$ Wasserstoff bedeutet, sowie der Enoläther der Formeln Ia und Ib, in denen $R^{13}$ Wasserstoff bedeutet, auch aus diesen Verbindungen in an sich bekannter Weise hergestellt werden, wie beispielsweise durch Auflösen der Verbindung I oder des Enoläthers Ia oder Ib in einer Lösung einer diesbezüglichen anorganischen oder organischen Base. Die Salzbildung erfolgt in der Regel innert kurzer Zeit bei Raumtemperatur. In einer Ausführungsform wird das Natriumsalz durch Auflösen der Verbindung I bzw. des Enoläthers Ia oder Ib in wässriger Natriumhydroxidlösung bei Raumtemperatur hergestellt, wobei äquivalente Mengen der Verbindung I bzw. des Enoläthers Ia oder Ib und des Natriumhydroxids verwendet werden. Das feste Salz kann dann durch Fällen mit einem geeigneten inerten Lösungsmittel oder durch Abdampfen des Lösungsmittels isoliert werden. Eine weitere Ausführungsform besteht darin, eine wässrige Lösung eines Alkalimetallsalzes der Verbindung I oder des Enoläthers Ia oder Ib in eine wässrige Lösung eines Salzes, das ein anderes Metallion als ein Alkalimetallion aufweist, einzuführen, wobei das zweite Metallsalz der Verbindung bzw. des Enoläthers hergestellt wird. Diese Ausführungsform dient im allgemeinen zur Herstellung derjenigen Metallsalze, die in Wasser unlöslich sind.

Die erhaltenen Verbindungen der Formel I, Enoläther sowie Salze können nach an sich bekannten Methoden isoliert und gereinigt werden. Sofern keine gezielte Synthese zur Isolierung reiner Isomerer durchgeführt wird, kann das Produkt als Gemisch zweier oder mehrerer Isomerer anfallen. Die Isomeren können nach an sich bekannten Methoden aufgetrennt werden. Gewünschtenfalls können reine optisch aktive Isomere auch durch Synthese aus entsprechenden optisch aktiven Ausgangsmaterialien hergestellt werden.

Die Ausgangsmaterialien der Formel II sind neu und können in an sich bekannter Weise hergestellt werden, z.B. gemäss dem nachfolgenden Reaktionsschema 1 [Methoden aa), bb) und cc)]:

Reaktionsschema 1

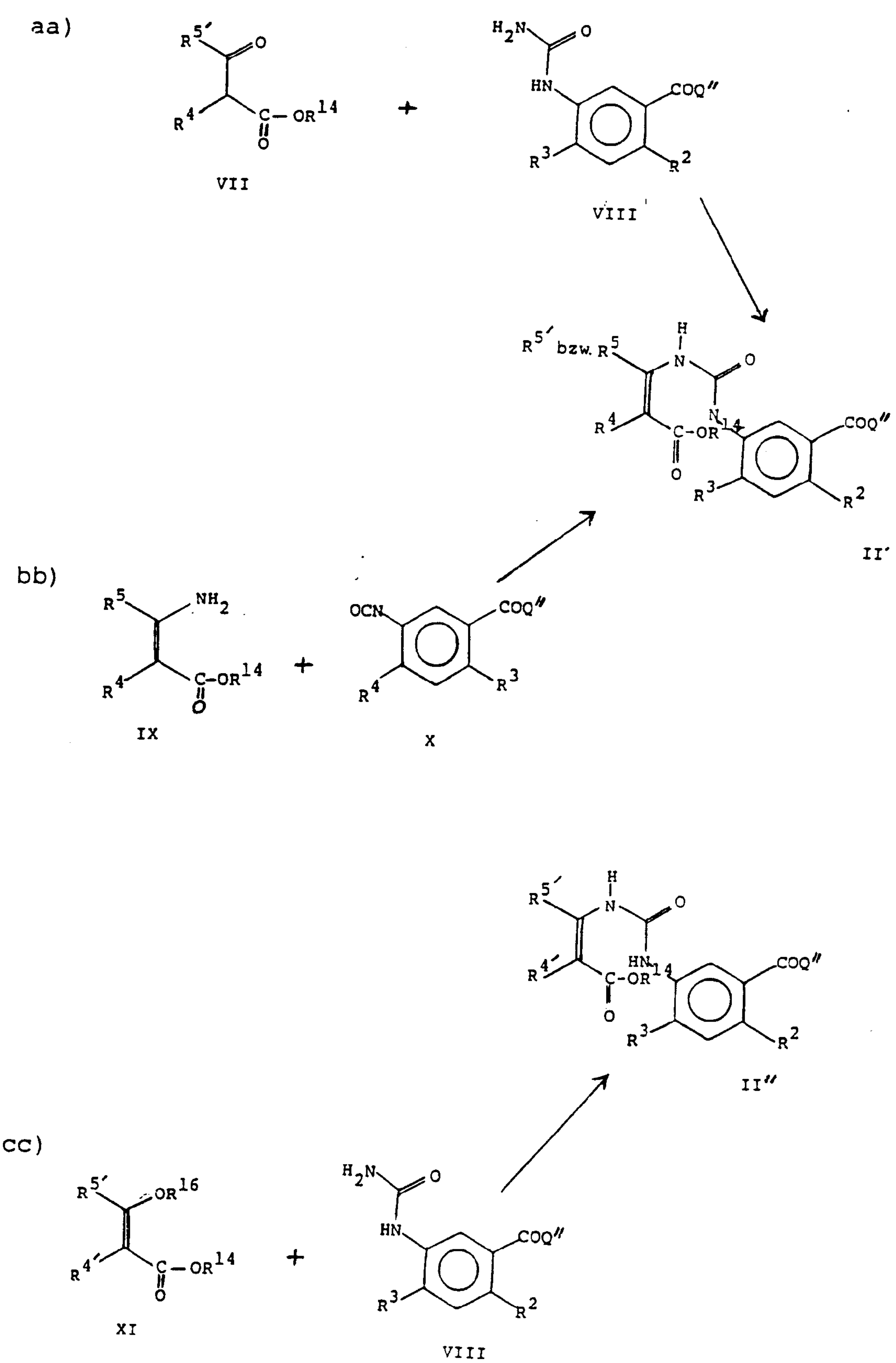

aa) VII + VIII → II'

bb) IX + X → II'

cc) XI + VIII → II''

In den obigen Reaktionsschemata besitzen $R^2$, $R^3$, $R^4$, $R^5$, $R^{14}$ und Q'' die oben angegebenen Bedeutungen; $R^{4'}$ bedeutet Wasserstoff oder $C_{1-4}$-Alkyl; $R^{5'}$ bedeutet $C_{1-4}$-Alkyl; und $R^{16}$ bedeutet nieder Alkyl, vorzugsweise $C_{1-4}$-Alkyl.

Die Methode aa) wird zweckmässigerweise dadurch durchgeführt, dass man die Verbindungen der Formeln VII und VIII in einem im wesentlichen wasserfreien Verdünnungsmittel und in Gegenwart eines sauren Katalysators bei erhöhter Temperatur miteinander reagieren lässt. Als Verdünnungsmittel kommen insbesondere mit Wasser Azeotrope bildende organische Lösungsmittel, wie Aromaten, z.B. Benzol, Toluol und Xylole; halogenierte Kohlenwasserstoffe, wie Methylenchlorid, Chloroform, Tetrachlorkohlenstoff und Chlorbenzol; und aliphatische und cyclische Aether, wie 1,2-Dimethoxyäthan, Tetrahydrofuran und Dioxan, und als saure Katalysatoren insbesondere starke Mineralsäuren, wie Schwefelsäure und Salzsäure; organische Säuren, wie p-Toluolsulfonsäure; Phosphor enthaltende Säuren, wie Orthophosphorsäure und Polyphosphorsäure; und saure Kationenaustauscher, wie "Amberlyst 15" (Fluka), in Frage. Man arbeitet im allgemeinen in einem Temperaturbereich von etwa 70°C bis 120°C, vorzugsweise bei der Rückflusstemperatur des Reaktionsgemisches. Unter diesen Reaktionsbedingungen wird die erwünschte rasche Entfernung des in der Reaktion gebildeten Wassers erzielt.

Die Umsetzung nach der Methode bb) erfolgt zweckmässigerweise in Gegenwart eines im wesentlichen wasserfreien aprotischen organischen Lösungsmittels, wie eines aliphatischen oder cyclischen Aethers, z.B. Diäthyläther, 1,2-Dimethoxyäthan, Tetrahydrofuran oder Dioxan, eines aliphatischen oder aromatischen Kohlenwasserstoffs, z.B. n-Hexan, Benzol, Toluol oder eines Xylols; oder eines halogenierten, aliphatischen Kohlenwasserstoffes, z.B. Methylenchlorid, Chloroform, Tetrachlorkohlenstoff oder 1,2-Dichloräthan, sowie gegebenenfalls in Gegenwart einer Base, insbesondere einer organischen tertiären Base, wie Triäthylamin oder Pyridin, wobei letzteres sowohl als Lösungsmittel wie auch als Base dienen kann, oder eines Metallhydrids, wie Natrium- oder Kaliumhydrid. Die Reaktionstemperaturen sind vorzugsweise im Bereich von etwa -80°C bis 50°C, wobei besonders bevorzugt bei Temperaturen zwischen -30°C und der Raumtemperatur gearbeitet wird.

Die Umsetzung nach der Methode cc) wird zweckmässigerweise in einem inerten, mit Wasser mischbaren, organischen Lösungsmittel, wie einem aliphatischen oder cyclischen Aether, z.B. 1,2-Dimethoxyäthan, Tetrahydrofuran oder Dioxan, oder einem niederen Alkanol, wie Aethanol, bei Temperaturen zwischen 50°C und 100°C, vorzugsweise bei der Rückflusstemperatur des Reaktionsgemisches, oder in einem aromatischen Lösungsmittel, wie Benzol, Toluol oder einem Xylol in Gegenwart eines sauren Katalysators, wie Salzsäure oder p-Toluolsulfonsäure, bei Temperaturen zwischen 50°C und 100°C, vorzugsweise 60°C bis 80°C, durchgeführt.

Die Ausgangsmaterialien der Formeln III und V und deren Herstellung sind grösstenteils in der europäischen Patentpublikation Nr. 195.346 beschrieben. Diejenigen Ausgangsmaterialien III und V, deren Herstellung nicht beschrieben ist, können analog den bekannten Ausgansgmaterialien hergestellt werden. Die ebenfalls als Ausgangsmaterialien verwendbaren reaktionsfähigen Derivate der Benzoesäuren der Formel III können aus dieser Benzoesäuren nach an sich bekannten Methoden hergestellt werden. Neu sind hingegen sämtliche, ebenfalls bei der Verfahrensvariante b) als Ausgangsmaterialien verwendbare Enoläther der Benzoesäuren III, d.h. die Verbindungen der allgemeinen Formeln IIIa und IIIb. Diese können beispielsweise gemäss dem nachfolgenden Reaktionsschema 2, in dem $R^{1'}$, $R^2$, $R^3$, $R^4$, $R^5$, Hal und $M^{\oplus}$ die oben angegebenen Bedeutungen besitzen und $R^{17}$ nieder Alkyl, vorzugsweise $C_{1-4}$-Alkyl, bedeutet, hergestellt werden:

Reaktionsschema 2

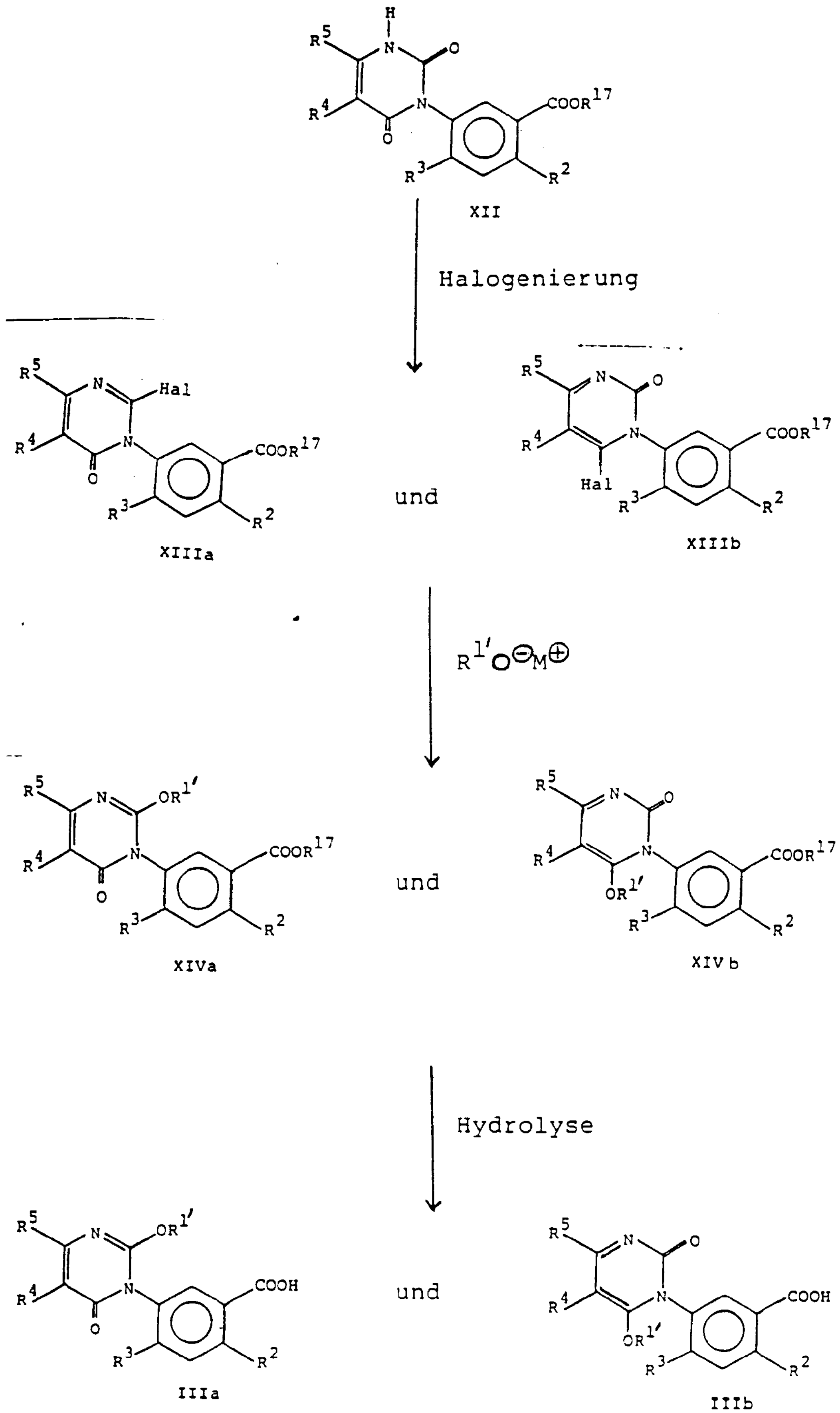


XII

XIIIa und XIIIb

XIVa und XIVb

IIIa und IIIb

Bei der Halogenierung des Benzoesäureesters der Formel XII wird als Halogenierungsmittel insbesondere Thionylchlorid, Phosphorpentachlorid oder Phosphoroxychlorid bzw. Phosphorpentabromid oder Phosphorylbromid verwendet. Gegebenenfalls wird ein Gemisch von Phosphorpentachlorid und Phosphoroxychlorid bzw. von Phosphorpentabromid und Phosphorylbromid eingesetzt, wobei ein Ueberschuss an Phosphoroxychlorid bzw. Phosphorylbromid als Verdünnungsmittel dienen kann. Die Chlorierung bzw. Bromierung kann in Gegenwart eines inerten Verdünnungsmittels, insbesondere eines aprotischen organischen Lösungsmittels, wie eines aliphatischen oder aromatischen Kohlenwasserstoffes, z.B. n-Hexan, Benzol, Toluol oder eines Xylols; eines halogenierten aliphatischen Kohlenwasserstoffes, z.B. Methylenchlorid, Chloroform oder 1,2-Dichloräthan; eines halogenierten aromatischen Kohlenwasserstoffes, z.B. Chlorbenzol, oder eines tertiären Amins, z.B. N,N-Dimethylanilin, durchgeführt werden, jedoch ist dies im Falle der Verwendung von Phosphoroxychlorid bzw. Phosphorylbromid als Halogenierungsmittel nicht notwendig. Bei Verwendung von Thionylchlorid als Halogenierungsmittel erweist es sich als zweckmässig, eine katalytische Menge Dimethylformamid zuzusetzen. Die Reaktionstemperaturen liegen im allgemeinen zwischen 0°C und der Rückflusstemperatur des Reaktionsgemisches, vorzugsweise zwischen 80°C und 120°C.

Auf diese Weise wird üblicherweise ein Gemisch der beiden Verbindungen XIIIa und XIIIb erhalten. Solche Gemische können gegebenenfalls aufgetrennt und die einzelnen Isomeren der Umsetzung mit dem Metallalkanolat, -alkenolat bzw. -alkinolat $R^{1'}O^{\ominus}M^{\oplus}$ unterworfen werden, oder aber man kann, vorzugsweise, das Isomerengemisch XIIIa/XIIIb mit dem Metallalkanolat, -alkenolat bzw. -alkinolat $R^{1'}O^{\ominus}M^{\oplus}$ umsetzen und danach das Produkt gegebenenfalls in die einzelnen Verbindungen XIVa und XIVb auftrennen. Solche Trennungen können nach an sich bekannten Methoden durchgeführt werden. Das besagte Produkt, also das aufzutrennende Gemisch der beiden Isomeren XIVa und XIVb, ist im wesentlichen in reiner Form, also frei von Ausgangsmaterialien und andersartigen Isomeren. Die Umsetzung der Verbindung XIIIa oder XIIIb bzw. des Isomerengemisches XIIIa/XIIIb mit dem Metallalkanolat, -alkenolat bzw. -alkinolat $R^{1'}O^{-}M^{+}$ kann analog der oben beschriebenen Verfahrensvariante d) erfolgen.

Die anschliessende Hydrolyse der Verbindung XIVa oder XIVb kann nach an sich bekannten Methoden durchgeführt werden, insbesondere unter Verwendung einer anorganischen Säure und gegebenenfalls in Gegenwart eines organischen Lösungsmittels und/oder von Wasser. Als Säuren kommen vorzugsweise Salzsäure, Schwefelsäure und Phosphorsäure, als organische Lösungsmittel Alkohole, z.B. Aethanol; aliphatische oder cyclische Aether, z.B. 1,2-Dimethoxyäthan, Tetrahydrofuran und Dioxan, und gegebenenfalls chlorierte aliphatische oder alicyclische Kohlenwasserstoffe, z.B. Methylenchlorid, Tetrachlormethan, n-Hexan und Cyclohexan, in Frage. Die Reaktionstemperaturen liegen im allgemeinen zwischen -20°C und 120°C, vorzugsweise zwischen 0°C und 30°C, insbesondere zwischen 15°C und 20°C.

Die in der Verfahrensvariante d) verwendeten Ausgangsmaterialien der Formel VIa bzw. VIb können durch Halogenierung der entsprechenden Uracilderivate der allgemeinen Formel

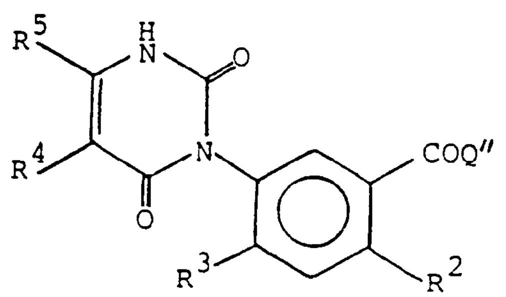

I′

worin $R^2$, $R^3$, $R^4$, $R^5$ und Q″ die oben angegebenen Bedeutungen besitzen,

hergestellt werden, und zwar analog dem oben beschriebenen Verfahren XII→XIIIa und XIIIb (siehe das Reaktionsschema 2 und die darauf folgende Beschreibung der Reaktionsbedingungen). Bei den Uracilderivaten der Formel I′ handelt es sich um eine Untergruppe von Verbindungen der Formel I, die Produkten der Verfahrensvariante a) entsprechen.

Die restlichen in den Verfahrensvarianten b) bis d) sowie in den Reaktionsschemata 1 und 2 involvierten Ausgangsmaterialien bzw. Reagentien sind entweder bekannt oder können nach an sich bekannten Methoden hergestellt werden.

Die Verbindungen der Formel I sowie ihre Enoläther und Salze (im Folgenden insgesamt als erfindungsgemässe Verbindungen oder Wirkstoffe bezeichnet) besitzen herbizide Eigenschaften und eignen sich zur Bekämpfung von Unkräutern, einschliesslich Ungräsern, u.a. Setaria faberii, Digitaria sanguinalis, Poa annua, Chenopodium album, Amaranthus retroflexus, Abutilon theophrasti, Sinapis arvensis und Datura stramonium,

in diversen Nutzpflanzenkulturen, u.a. in Getreide- Soja-, Mais-, Reis- und Baumwollekulturen. Zudem sind die Verbindungen sowohl Vorauflauf- als auch Nachauflauf-Herbizide.

In der Praxis genügt üblicherweise eine Konzentration von 1 g bis 3 kg erfindungsgemässe Verbindung/ha, vorzugsweise 10 bis 500 g erfindungsgemässe Verbindung/ha, um den gewünschten herbiziden Effekt zu erzielen.

Das erfindungsgemässe Unkrautbekämpfungsmittel ist dadurch gekennzeichnet, dass es eine wirksame Menge mindestens einer Verbindung der Formel I, wie oben definiert, oder eines Enoläthers oder Salzes davon, sowie Formulierungshilfsstoffe enthält. Das Mittel enthält zweckmässigerweise zumindest einen der folgenden Formulierungshilfsstoffe aus der Gruppe: feste Trägerstoffe; Lösungs- bzw. Dispersionsmittel; Tenside (Netz- und Emulgiermittel); Dispergatoren (ohne Tensidwirkung); und Stabilisatoren. Unter Verwendung solcher und anderer Hilfsstoffe können diese Verbindungen, also die herbiziden Wirkstoffe, in die üblichen Formulierungen übergeführt werden, wie Stäube, Pulver, Granulate, Lösungen, Emulsionen, Suspensionen, emulgierbare Konzentrate, Pasten und dergleichen.

Die Verbindungen der Formel I und ihre Enoläther sind im allgemeinen wasserunlöslich, die Salze hingegen, insbesondere die Alkalimetallsalze und Ammoniumsalze, im allgemeinen wasserlöslich, und können nach den für wasserunlösliche bzw. wasserlösliche Verbindungen üblichen Methoden unter Verwendung der diesbezüglichen Formulierungshilfsstoffe konfektioniert werden. Die Herstellung der Mittel kann in an sich bekannter Weise durchgeführt werden, z.B. durch Vermischen des jeweiligen Wirkstoffes mit festen Trägerstoffen, durch Auflösen oder Suspendieren in geeigneten Lösungs- bzw. Dispersionsmitteln, eventuell unter Verwendung von Tensiden als Netz- oder Emulgiermitteln und/oder von Dispergatoren, durch Verdünnen bereits vorbereiteter emulgierbarer Konzentrate mit Lösungs- bzw. Dispersionsmitteln usw.

Als feste Trägerstoffe kommen im wesentlichen in Frage: natürliche Mineralstoffe, wie Kreide, Dolomit, Kalkstein, Tonerden und Kieselsäure und deren Salze (beispielsweise Kieselgur, Kaolin, Bentonit, Talkum, Attapulgit und Montmorrillonit); synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Pulver oder als Granulate vorliegen können.

Als Lösungs- bzw. Dispersionsmittel kommen im wesentlichen in Frage: Aromaten, wie Benzol, Toluol, Xylole und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methyläthylketon, Methylisobutylketon und Cyclohexanon; und stark polare Lösungs- bzw. Dispersionsmittel, wie Dimethylformamid, N-Methylpyrrolidon und Dimethylsulfoxid, wobei solche Lösungsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol-Treibgase, wie Halogenkohlenwasserstoffe, z.B. Dichlordifluormethan. Liegt das erfindungsgemässe Unkrautbekämpfungsmittel in Form einer Druckgaspackung vor, so wird zweckmässigerweise zusätzlich zum Treibgas ein Lösungsmittel verwendet.

Die Tenside (Netz- und Emulgiermittel) können nichtionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen sein, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäuren, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose, Polyvinylalkohol, Alginate, Caseinate und Blutalbumin eingesetzt werden.

Beispiele von geeigneten Stabilisatoren sind säurebindende Mittel, z.B. Epichlorhydrin, Phenylglycidäther und Soyaepoxide; Antioxidantien, z.B. Gallussäureester und Butylhydroxytoluol; UV-Absorber, z.B. substituierte Benzophenone, Diphenylacrylonitrilsäureester und Zimtsäureester; und Deaktivatoren, z.B. Salze der Aethylendiaminotetraessigsäure und Polyglykole.

Die erfindungsgemässen Unkrautbekämpfungsmittel können zusätzlich zu den erfindungsgemässen Wirkstoffen Synergisten und andere Wirkstoffe, z.B. Insektizide, Akarizide, Fungizide, Pflanzenwachstumsregulatoren und Düngemittel, enthalten. Solche Kombinationsmittel eignen sich zur Verstärkung der Aktivität bzw. zur Verbreiterung des Wirkungsspektrums.

Die erfindungsgemässen Unkrautbekämpfungsmittel enthalten im allgemeinen zwischen 0,001 und 95 Gewichtsprozent, vorzugsweise zwischen 0,5 und 75 Gewichtsprozent einer bzw. mehrerer erfindungsgemässer Verbindungen als Wirkstoff(e).

Sie können z.B. in einer Form vorliegen, die sich für die Lagerung und den Transport eignet. In solchen Formulierungen, z.B. emulgierbaren Konzentraten, ist die Wirkstoffkonzentration normalerweise im höheren Bereich, vorzugsweise zwischen 1 und 50 Gewichtsprozent, insbesondere zwischen 5 und 30 Gewichtsprozent. Diese Formulierungen können dann, z.B. mit gleichen oder verschiedenen inerten Stoffen, bis zu Wirkstoffkonzentrationen verdünnt werden, die sich für den praktischen Gebrauch eignen, also vorzugsweise ca. 0,001 bis 10 Gewichtsprozent, insbesondere ca. 0,005 bis 5 Gewichtsprozent. Die Wirkstoffkonzentrationen können jedoch auch kleiner oder grösser sein.

Wie oben erwähnt, kann die Herstellung der erfindungsgemässen Unkrautbekämpfungsmittel in an sich bekannter Weise durchgeführt werden.

Zur Herstellung pulverförmiger Präparate kann der Wirkstoff, d.h. mindestens eine erfindungsgemässe Verbindung, mit festem Trägerstoff vermischt werden, z.B. durch Zusammenmahlen; oder man kann den festen Trägerstoff mit einer Lösung oder Suspension des Wirkstoffes imprägnieren und dann das Lösungs- bzw. Dispersionsmittel durch Abdunsten, Erhitzen oder Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Der Wirkstoff kann auch mit einem Tensid und einem festen Trägerstoff zur Bildung eines netzbaren Pulvers vermischt werden, welches in Wasser dispergierbar ist, oder er kann mit einem festen vorgranulierten Trägerstoff zur Bildung eines granulatförmigen Produktes vermischt werden.

Wenn gewünscht, kann der Wirkstoff in einem mit Wasser nicht mischbaren Lösungsmittel, wie beispielsweise einem hochsiedenden Kohlenwasserstoff, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Die Verwendung der erfindungsgemässen Unkrautbekämpfungsmittel, die einen weiteren Gegenstand der vorliegenden Erfindung bildet, kann nach üblichen Applikationsmethoden, wie Spritzen, Sprühen, Stäuben, Giessen oder Streuen, erfolgen. Das erfindungsgemässe Verfahren zur Bekämpfung von Unkräutern ist dadurch gekennzeichnet, dass man das gegen Unkräuter zu schützende Gut und/oder die Unkräuter mit einer erfindungsgemässen Verbindung bzw. mit einem erfindungsgemässen Unkrautbekämpfungsmittel behandelt.

Die nachfolgenden Beispiele dienen zur näheren Erläuterung der Erfindung.

I. Herstellung der Verbindungen der Formel I bzw. ihrer Enoläther:

Beispiel 1

Zu einer Suspension von 3,0 g 2-Chlor-5-[3,6-dihydro-2,6-dioxo-3-methyl-4-trifluormethyl-1(2H)-pyrimidinyl]-4-fluorbenzoesäure in 50 ml Diäthyläther wird bei Raumtemperatur 0,9 g 3-Pentanonoxim zugegeben. Danach wird eine Lösung von 1,9 g Dicyclohexylcarbodiimid und 0,1 g 4-Pyrrolidinopyridin in 15 ml Diäthyläther über 10 Minuten zugetropft. Man rührt das Reaktionsgemisch 12 Stunden bei Raum temperatur nach. Anschliessend wird filtriert und unter vermindertem Druck zur Trockene eingedampft. Der Rückstand wird an Kieselgel mit n-Hexan/Diäthyläther (4:1) als Laufmittel chromatographisch gereinigt. Man erhält auf diese Weise das 3-Pentanon-O-{2-chlor-5-[3,6-dihydro-2,6-dioxo-3-methyl -4-trifluormethyl-1(2H)-pyrimidinyl]-4-fluorbenzoyl}oxim, Smp. 128-130°C.

Beispiele 2-11

Analog dem in Beispiel 1 beschriebenen Verfahren erhält man ausgehend von 2-Chlor-5-[3,6-dihydro-2,6-dioxo-3-methyl-4-trifluormethyl-1(2H)-pyrimidinyl]-4-fluorbenzoesäure und der entsprechenden Hydroxyverbindung der Formel H-Q die in der nachfolgenden Tabelle 1 aufgeführten Verbindungen der Formel I:

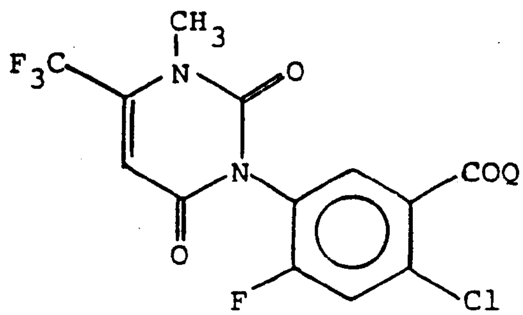

Tabelle 1

| Beispiel | Q | Physikalische Daten |
|---|---|---|
| 2 | $O-N=C(CH_3)($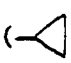$)$ | Smp. 146-148°C |
| 3 | $O-N=C(isoC_3H_7)_2$ | Smp. 113-115°C |
| 4 | $O-N=C(CH_3)(isoC_3H_7)$ | Smp. 50-52°C |

| | | |
|---|---|---|
| 5 | $O-(CH_2)_2-$<br>$ON=C(CH_3)(C_2H_5)$ | $^1H$-NMR ($CDCl_3$, 60 MHz):<br>7,98 ppm (d,1H), 7,42 ppm (d,1H), 6,43 ppm (s,1H), 4,51 ppm (m,4H), 3,62 ppm (d,4H), 2,26 ppm (q,2H), 1,91 ppm (s,3H), 1,20 ppm (m,6H) |
| 6 | 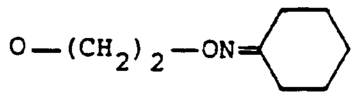 | $^1H$-NMR ($CDCl_3$, 60 MHz):<br>7,99 ppm (d,1H), 7,41 ppm (d,1H), 6,43 ppm (s,1H), 5,50 ppm (m,1H), 4,24 ppm (d,2H), 3,65 ppm (s,3H), 1,91 ppm (s,6H), 1,42 ppm (d,3H) |
| 7 | $O-CH(CH_3)CH_2-$<br>$ON=C(CH_3)_2$ | $^1H$-NMR ($CDCl_3$, 60 MHz):<br>7,96 ppm (d,1H), 7,43 ppm (d,1H), 6,43 ppm (s,1H), 5,50 ppm (m,1H), 4,24 ppm (d,2H), 3,65 ppm (s,3H), 1,91 ppm (s,6H), 1,42 ppm (d,3H) |
| 8 | 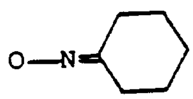 | Smp. 120-122°C |
| 9 | $O-CH(C_2H_5)CH_2-$<br>$ON=C(CH_3)_2$ | $^1H$-NMR ($CDCl_3$, 60 MHz):<br>7,89 ppm (d,1H), 7,40 ppm (d,1H), 6,36 ppm (s,1H), 5,34 ppm (m,1H), 4,22 ppm (d,2H), 3,58 ppm (s,3H), 1,30 ppm (m,11H) |
| 10 | $O-CH(C_6H_5)CH_2-$<br>$ON=C(CH_3)_2$ | Smp. 50-51°C |

| | | |
|---|---|---|
| 11 | O-N=C($CH_3$)($OC_2H_5$) | $^1$H-NMR ($CDCl_3$, 60 MHz): 7,92 ppm (d,1H), 7,46 ppm (d,1H), 6,40 ppm (s,1H), 4,32 ppm (q,2H), 3,60 ppm (d,3H), 2,18 ppm (s,3H), 1,39 ppm (t,3H) |

Beispiele 12-14

Analog dem in Beispiel 1 beschriebenen Verfahren erhält man ausgehend von 2-Chlor-5-[3,6-dihydro-2,6-dioxo-3-methyl bzw. difluormethyl-4-methyl-1(2H)-pyrimidinyl]-4-fluorbenzoesäure und der entsprechenden Hydroxyverbindung der Formel H-Q die in der nachfolgenden Tabelle 2 aufgeführten Verbindungen der Formel I:

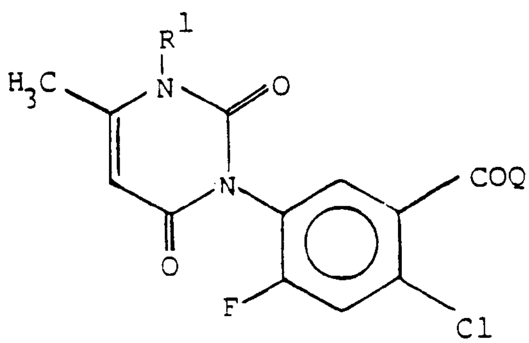

Tabelle 2

| Beispiel | $R^1$ | Q | Physikalische Daten |
|---|---|---|---|
| 12 | $CH_3$ | O-N=C($CH_3$)$_2$ | $^1$H-NMR ($CDCl_3$), 60 MHz): 7,90 ppm (d,1H), 7,43 ppm (d,1H), 5,79 ppm (s,1H), 3,50 ppm (s,3H), 2,37 ppm (s,3H), 2,14 ppm (s,6H). |

| | | | |
|---|---|---|---|
| 13 | $CH_3$ | $O-(CH_2)_2-$ $ON=C(CH_3)_2$ | $^1H$-NMR ($CDCl_3$, 60 MHz): 7,91 ppm (d,1H), 7,36 ppm (d,1H), 5,76 ppm (s,1H), 4,43 ppm (m,4H), 3,48 ppm (s,3H), 2,35 ppm (s,3H), 1,88 ppm (s,6H). |
| 14 | $CHF_2$ | $O-(CH_2)_2-$ $ON=C(CH_3)_2$ | $^1H$-NMR ($CDCl_3$, 60 MHz): 7,90 ppm (d,1H), 7,79 ppm (t,1H), 7,40 ppm (d,1H), 5,88 ppm (s,1H), 4,45 ppm (m,4H), 2,54 ppm (m,3H), 1,89 ppm (s,6H). |

Beispiel 15

Ein Gemisch von 1,5 g 2-Chlor-4-fluor-5-[2-methoxy-6-oxo-4-trifluormethyl-1(6H)-pyrimidinyl]-benzoesäure, 1,37 g 1,1,3-Tribromo-1-propen und 0,52 g Natriumcarbonat in 50 ml wasserfreiem Aceton wird 8 Stunden unter Rühren erhitzt. Anschliessend werden die unlöslichen Anteile abgenutscht, und das Filtrat wird unter vermindertem Druck zur Trockene eingedampft. Man löst den Rückstand in 100 ml Diäthyläther und schüttelt die Lösung dreimal mit je 50 ml Wasser aus. Dann wird die organische Phase über wasserfreiem Natriumsulfat getrocknet und zur Trockene eingedampft, und der Rückstand wird an einer Kieselgel-Säure unter Verwendung von n-Hexan/Diäthyläther (3:1) als Laufmittel chromatographisch gereinigt. Man erhält den 2-Chlor-4-fluor-5-[2-methoxy-6-oxo-4-trifluormethyl-1(6H)-pyrimidinyl]-benzoesäure-(3,3-dibrom-2-propenyl)ester, $^1H$-NMR ($CDCl_3$, 400 MHz): 7,89 ppm (d, 1H), 7,42 ppm (d, 1H), 6,72 ppm (t, 1H), 6,62 ppm (s, 1H), 4,82 ppm (d, 2H), 4,02 ppm (d, 3H).

Als Alternative bei diesem Verfahren können beispielsweise Natriumhydrid als Base und Dimethylformamid als Lösungsmittel verwendet werden.

Beispiele 16-26

Analog dem in Beispiel 15 beschriebenen Verfahren erhält man ausgehend von 2-Chlor-4-fluor-5-[2-methoxy-6-oxo-4-trifluormethyl-1(6H)-pyrimidinyl]-benzoesäure bzw. 2-Chlor-5-[3,6-dihydro-2,6-dioxo -3-methyl-4-trifluormethyl-1(2H)-pyrimidinyl]-4-fluorbenzoesäure und dem entsprechenden Halogenid (reaktionsfähigem Derivat der Hydroxyverbindung H-Q) die in der nachfolgenden Tabelle 3 bzw. 4 aufgeführten Verbindungen der Formel I oder Enoläther:

Tabelle 3

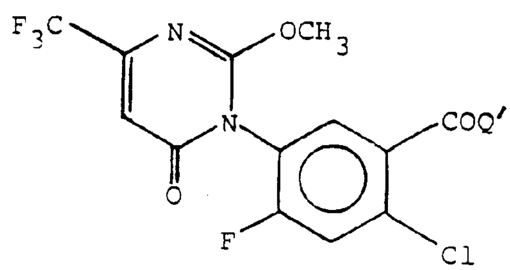

| Beispiel | Q' | Physikalische Daten |
|---|---|---|
| 16 | $O-CH_2CH=CCl_2$ | $^1$H-NMR ($CDCl_3$, 400 MHz): 7,88 ppm (d,1H), 7,42 ppm (d,1H), 6,63 ppm (s,1H), 6,17 ppm (t,1H), 4,92 ppm (d,2H), 4,02 ppm (d,3H) |
| 17 | $O-CH_2CH=C(Cl)(CH_3)$ (E/Z) | $^1$H-NMR ($CDCl_3$, 400 MHz): 7,87 ppm (2d,1H), 7,41 ppm (2d,1H), 6,62 ppm (2s,1H), 5,92-5,74 ppm (m,1H), 4,98--4,76 ppm (m,2H), 4,02 ppm (2s,3H), 2,24-2,14 ppm (m,3H) |
| 18 | $O-CH_2CH=CHCH_2Cl$ (E/Z) | $^1$H-NMR ($CDCl_3$, 400 MHz): 7,88 ppm (2d,1H), 7,42 ppm (2d,1H), 6,62 ppm (2s,1H), 6,04-5,80 ppm (m,2H), 4,96--4,82 ppm (m,2H), 4,22-4,06 ppm (m,2H), 4,02 ppm (2s,3H) |
| 19 | $O-CH_2C{\equiv}CCH_2Br$ | Smp. 113-115°C |

Tabelle 4

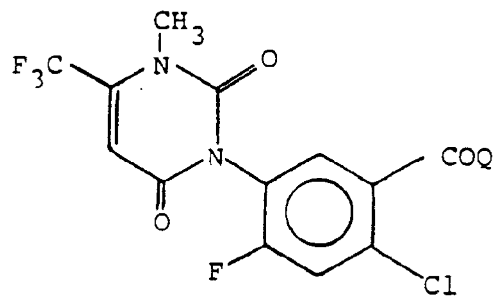

| Beispiel | Q | Physikalische Daten |
|---|---|---|
| 20 | $O-CH_2CH=CBr_2$ | $^1$H-NMR ($CDCl_3$, 400 MHz): 7,92 ppm (d, 1H), 7,40 ppm (d, 1H), 6,71 ppm (t, 1H), 6,38 ppm (s, 1H), 4,81 ppm (d, 2H), 3,57 ppm (m, 3H) |

| | | |
|---|---|---|
| 21 | O-$CH_2$CH=$CCl_2$ | $^1$H-NMR ($CDCl_3$,400 MHz): 7,91 ppm (d,1H), 7,40 ppm (d,1H), 6,38 ppm (s,1H), 6,16 ppm (t,1H), 4,90 ppm (d,2H), 3,57 ppm (m,3H) |
| 22 | O-$CH_2$CH=C(Cl)($CH_3$) (E/Z) | $^1$H-NMR ($CDCl_3$,400 MHz): 7,90 ppm (2d,1H), 7,39 ppm (2d,1H), 6,37 ppm (2s,1H), 5,72-5,92 ppm (2m,1H), 4,76--4,98 ppm (m,2H), 3,56 ppm (m,3H), 2,18 ppm (m,3H) |
| 23 | O-$CH_2$CH=CH$CH_2$Cl (E/Z) | $^1$H-NMR ($CDCl_3$,400 MHz): 7,89 ppm (2d,1H), 7,39 ppm (2d,1H), 6,37 ppm (2s,1H), 6,02-5,80 ppm (m,2H), 4,96--4,80 ppm (m,2H), 4,22-4,04 ppm (m,2H), 3,56 ppm (m,3H) |
| 24 | O-$CH_2$C≡C$CH_2$Br | $^1$H-NMR ($CDCl_3$,400 MHz): 7,95 ppm (d,1H), 7,40 ppm (d,1H), 6,38 ppm (s,1H), 4,97 ppm (t,2H), 3,94 ppm (t,2H), 3,57 ppm (t,3H) |
| 25 | O-$CH_2$C≡CCl | $^1$H-NMR ($CDCl_3$, 400 MHz): 7,94 ppm (d,1H), 7,40 ppm (d,1H), 6,38 ppm (s,1H), 4,89 ppm (s,2H), 3,56 ppm (m,3H) |

| | | |
|---|---|---|
| 26 | $O-CH_2CH=CHCl$ (E/Z) | $^1H$-NMR ($CDCl_3$, 400 MHz): 7,90 ppm (2d,1H), 7,39 ppm (2d,1H), 6,43 und 6,29 ppm (2m,1H), 6,37 ppm (2s,1H), 6,13 und 6,04 ppm (2m,1H), 5,02 und 4,79 ppm (2q,2H), 3,56 ppm (2m,3H) |

Beispiele 27-31

Analog dem in Beispiel 15 beschriebenen Verfahren erhält man ausgehend von der entsprechenden 2-Chlor-5-[3,6-dihydro-2,6-dioxo-3,4-disubstituiertes-1(2H)-pyrimidinyl]-4-fluorbenzoesäure und der entsprechenden Hydroxyverbindung der Formel H-Q die in der nachfolgenden Tabelle 5 aufgeführten Verbindungen der Formel I:

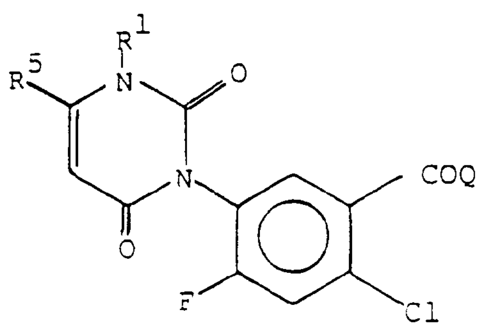

Tabelle 5

| Beispiel | $R^1$ | Q | $R^5$ | Physikalische Daten |
|---|---|---|---|---|
| 27 | $CH_3$ | $O-CH_2C(Cl)=CH_2$ | $C_2F_5$ | $^1H$-NMR ($CDCl_3$, 400 MHz): 7,95 ppm (d,1H), 7,41 ppm (d,1H), 6,33 ppm (s,1H), 5,57 ppm (m,1H), 5,47 ppm (d,1H), 4,89 ppm (d,2H), 3,57 ppm (m,3H) |

| | | | | |
|---|---|---|---|---|
| 28 | $CH_3$ | $O-CH_2-C{\equiv}CCl$ | $CH_3$ | $^1$H-NMR ($CDCl_3$, 60 MHz): 7,91 ppm (d,1H), 7,36 ppm (d,1H), 5,74 ppm (s,1H), 4,89 ppm (s,2H), 3,45 ppm (s,3H), 2,32 ppm (s,3H) |
| 29 | $CH_3$ | $O-CH_2-CH{=}CCl_2$ | $CH_3$ | Smp. 131-133°C |
| 30 | $CH_3$ | $O-CH_2CH_2Cl$ | $CH_3$ | $^1$H-NMR ($CDCl_3$, 60 MHz): 7,92 ppm (d,1H), 7,36 ppm (d,1H), 5,74 ppm (s,1H), 4,55 ppm (t,2H), 3,78 ppm (t,2H), 3,45 ppm (s,3H), 2,32 ppm (s,3H) |
| 31 | $CHF_2$ | $O-CH_2CH_2Cl$ | $CH_3$ | $^1$H-NMR ($CDCl_3$, 60 MHz): 7,92 ppm (d,1H), 7,72 ppm (t,1H), 7,39 ppm (d,1H), 5,84 ppm (s,1H), 4,57 ppm (t,2H), 3,78 ppm (t,2H), 2,50 ppm (s,3H) |

Beispiel 32

2,5 g 2-Chlor-5-[3,6-dihydro-2,6-dioxo-3-methyl-4-trifluormethyl-1(2H)-pyrimidinyl]-4-fluorbenzoesäure werden in 20 ml Benzol und 2,4 ml Thionylchlorid zusammen mit einem Tropfen Dimethylformamid 3 Stunden bei Rückflusstemperatur erhitzt. Anschliessend wird das Reaktionsgemisch zur Trockene eingedampft und in 15 ml Dioxan gelöst. Diese Lösung, die hauptsächlich aus dem Säurechlorid der obenerwähnten Benzoesäure und dem Lösungsmittel besteht, wird bei Raumtemperatur zu einer Lösung von 0,7 g Isopropylmercaptan und 0,8 g Pyridin in 10 ml Dioxan getropft. Dann wird das Reaktionsgemisch 2,5 Stunden bei Raumtemperatur gerührt, mit 300 ml Wasser versetzt und zweimal mit je 300 ml Aethylacetat extrahiert. Man wäscht die vereinigten organischen Phasen zweimal mit je 150 ml 1N Salzsäure und einmal mit 150 ml gesättigter Natriumchloridlösung, trocknet die über wasserfreiem Natriumsulfat und dampft sie ein. Der Rückstand wird an Kieselgel mit Aethylacetat/n-Hexan (2:3) chromatographisch gereinigt. Auf diese Weise erhält man den 2-Chlor-5-[3,6-dihydro-2,6-dioxo -3-methyl-4-trifluormethyl-1(2H)-pyrimidinyl] -4-fluorthiobenzoesäure-S-isopropylester als zähes Oel.

Massenspektrum (m/e): $M^+$ 424 (0,6); $^1$H-NMR ($CDCl_3$, 400MHz): 7,96 ppm (d,1H), 7,91 ppm (d,1H), 6,61 ppm (s,1H), 3,78 ppm (Septett,1H), 3,42 ppm (s,3H), 1,36 ppm (d,6H).

Als Alternative bei diesem Verfahren kann beispielsweise Methylenchlorid als Lösungsmittel verwendet werden.

Beispiele 33-60

Analog dem in Beispiel 32 beschriebenen Verfahren erhält man ausgehend von 2-Chlor-5-[3,6-dihydro-2,6-dioxo-3-methyl-4-substituiertes-1(2H)-pyrimidinyl]-4-fluorbenzoesäure über deren Säurechlorid und der entsprechenden Hydroxy- oder Mercaptoverbindung H-Q die in der nachfolgenden Tabelle 6 aufgeführten Verbindungen der Formel I:

Tabelle 6

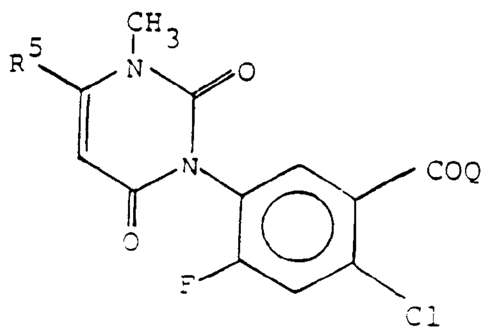

| Beispiel | $R^5$ | Q | Physikalische Daten |
|---|---|---|---|
| 33 | $CF_3$ | $O-N=C(CH_3)_2$ | $^1H$-NMR ($CDCl_3$,400 MHz): 7,87 ppm (d,1H), 7,40 ppm (d,1H), 6,37 ppm (s,1H), 3,56 ppm (m,3H), 2,11 ppm (s,3H), 2,13 ppm (s,3H) |
| 34 | $CF_3$ | $O-N=C(CH_3)($ 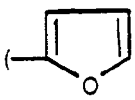 ) | Smp. 73-75°C |
| 35 | $CF_3$ | $O-N=C(CH_3)(COOCH_3)$ | $^1H$-NMR ($CDCl_3$, 60 MHz): 7,96 ppm (d,1H), 7,50 ppm (d,1H), 6,44 ppm (s,1H), 3,61 ppm (s,3H), 2,60 ppm (s,3H), 2,20 ppm (s,3H) |
| 36 | $CF_3$ | $O-(CH_2)_2-ON=C(CH_3)_2$ | $^1H$-NMR ($CDCl_3$,250 MHz): 7,92 ppm (d,1H), 7,39 ppm (d,1H), 6,38 ppm (s,1H), 4,53 ppm (m,2H), 4,31 ppm (m,2H), 3,57 ppm (m,1H), 1,85 ppm (s,3H), 1,84 ppm (s,3H) |
| 37 | $CF_3$ | $O-CH_2CH_2Cl$ | $^1H$-NMR ($CDCl_3$, 400 MHz): 7,93 ppm (d,1H), 7,41 ppm (d,1H), 6,38 ppm (s,1H), 4,57 ppm (m,2H), 3,80 ppm (m,2H), 3,57 ppm (m,3H) |

| | | | | |
|---|---|---|---|---|
| 38 | $CF_3$ | $O-CH_2CH_2F$ | | $^1H$-NMR ($CDCl_3$,400 MHz): 7,95 ppm (d,1H), 7,41 ppm (d,1H), 6,38 ppm (s,1H), 4,71 ppm (m,2H), 4,56 ppm (m,2H), 3,57 ppm (m,3H) |
| 39 | $CF_3$ | $O-CH_2CH_2Br$ | | $^1H$-NMR ($CDCl_3$,400 MHz): 7,94 ppm (d,1H), 7,41 ppm (d,1H), 6,38 ppm (s,1H), 4,63 ppm (t,2H), 3,63 ppm (t,2H), 3,58 ppm (m,3H) |
| 40 | $CF_3$ | $O-CH(CH_2Cl)_2$ | | $^1H$-NMR ($CDCl_3$,400 MHz): 7,91 ppm (d,1H), 7,42 ppm (d,1H), 6,39 ppm (s,1H), 5,42 ppm (m,1H), 3,87 ppm (q,4H), 3,57 ppm (m,3H) |
| 41 | $CF_3$ | $O-CH(CH_2F)_2$ | | $^1H$-NMR ($CDCl_3$,400 MHz): 7,93 ppm (d,1H), 7,42 ppm (d,1H), 6,38 ppm (s,1H), 5,45 ppm (m, 1H), 4,71 ppm (m,4H), 3,57 ppm (m,3H) |
| 42 | $CF_3$ | $S-CH_2C_6H_5$ | | Oel; Massenspektrum (m/e): $M^{+\cdot}$ 472 (4,4); $^1H$-NMR($D_6$-DMSO,400 MHz): 7,98 ppm (d,1H), 7,93 ppm (d,1H), 7,41-7,25 ppm (m,5H), 6,60 ppm (s,1H), 4,37 ppm (s,2H), 3,40 ppm (s,3H) |
| 43 | $CF_3$ | $S-C_4H_9n$ | | Smp. 83-84°C |

| | | | |
|---|---|---|---|
| 44 | $CF_3$ | $S-C_2H_5$ | Smp. 88-89°C |
| 45 | $CF_3$ | $S-CH_2CH=CH_2$ | Oel;<br>Massenspektrum (m/e): $M^+$ 422 (1,1);<br>$^1H$-NMR($D_6$-DMSO,400 MHz):<br>8,00 ppm (d,1H), 7,92 ppm (d,1H), 6,61 ppm (s,1H), 5,94-5,72 ppm (m,1H), 5,35 ppm (m,1H), 5,17 ppm (m,1H), 3,77 ppm (m,2H), 3,42 ppm (s,3H) |
| 46 | $CF_3$ | 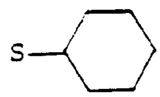 | Oel;<br>$^1H$-NMR ($D_6$-DMSO,400 MHz):<br>7,96 ppm (d,1H), 7,90 ppm (d,1H), 6,61 ppm (s,1H), 3,73-3,62 ppm (m,1H), 3,42 ppm (s,3H), 2,00-1,90 ppm (m,2H), 1,72-1,38 ppm (m,7H), 1,36-1,24 ppm (m,1H);<br>Mikroanalyse:<br>C% H% N% S% Cl%<br>berechnet 49,09 3,69 6,03 6,90 7,63<br>gefunden 49,09 3,62 5,79 7,08 7,55 |
| 47 | $CF_3$ | 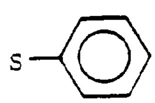 | Smp. 153-154°C |
| 48 | $CF_3$ | $O-CH_2CF_3$ | $^1H$-NMR ($CDCl_3$, 400 MHz): 7,94 ppm (d,1H), 7,43 ppm (d,1H), 6,38 ppm (s,1H), 4,68 ppm (m,2H), 3,57 ppm (m,3H) |
| 49 | $CF_3$ | $O-CH(CF_3)_2$ | $^1H$-NMR ($CDCl_3$, 400 MHz): 7,96 ppm (d,1H), 7,47 ppm (d,1H), 6,39 ppm (s,1H), 5,99 ppm (m,1H), 3,57 ppm (m,3H) |

| | | | |
|---|---|---|---|
| 50 | $CF_3$ | $O-N=C(CH_2COOC_2H_5)_2$ | $^1H$-NMR ($CDCl_3$, 60 MHz): 7,95 ppm (d,1H), 7,46 ppm (d,1H), 6,41 ppm (s,1H), 4,22 ppm (m,4H), 3,79 ppm (m,4H), 1,78 ppm (m,6H) |
| 51 | $CF_3$ | $O-N=C(CH_3)(CH_2COOC_2H_5)$ | $^1H$-NMR ($CDCl_3$, 60 MHz): 7,94 ppm (d,1H), 7,48 ppm (d,1H), 6,42 ppm (s,1H), 4,24 ppm (q,2H), 3,60 ppm (m,3H), 3,41 ppm (s,2H), 2,25 ppm (s,3H), 1,30 ppm (t,3H) |
| 52 | $CF_3$ | $O-N=C(CH_3)(C_6H_5)$ | $^1H$-NMR ($CDCl_3$, 60 MHz): 7,91 ppm (d,1H), 7,58 ppm (m,5H), 7,45 ppm (d,1H), 6,40 ppm (s,1H), 3,64 ppm (m,3H), 2,53 ppm (s,3H) |
| 53 | $CF_3$ | $O-N=C(CH_3)(COCH_3)$ | $^1H$-NMR ($CDCl_3$, 60 MHz): 7,96 ppm (d,1H), 7,50 ppm (d,1H), 6,44 ppm (s,1H), 3,61 ppm (m,3H), 2,60 ppm (s,3H), 2,20 ppm (s,3H) |
| 54 | $CF_3$ | $O-N=C(CH_3)(COOC_2H_5)$ | $^1H$-NMR ($CDCl_3$, 60 MHz): 7,96 ppm (d,1H), 7,50 ppm (d,1H), 6,43 ppm (s,1H), 4,46 ppm (q,2H), 3,65 ppm (m,3H), 2,39 ppm (s,3H), 1,47 ppm (t,3H) |

| | | | |
|---|---|---|---|
| 55 | $CF_3$ | $O-N=C(CH_3)(CH_2OCH_3)$ | $^1$H-NMR ($CDCl_3$, 60 MHz): 7,93 ppm (d,1H), 7,49 ppm (d,1H), 6,41 ppm (s,1H), 4,24 ppm (m,2H), 3,61 ppm (m,3H), 3,45 ppm (s,3H), 2,25 ppm (s,3H) |
| 56 | $CF_3$ | $O-N=C(CH_3)(CF_3)$ | $^1$H-NMR ($CDCl_3$, 60 MHz): 7,90 ppm (d,1H), 7,45 ppm (d,1H), 6,40 ppm (s,1H), 3,62 ppm (m,3H), 2,35 ppm (s,3H) |
| 57 | $C_2F_5$ | $O-CH_2CF_3$ | $^1$H-NMR ($CDCl_3$, 400 MHz): 7,95 ppm (d,1H), 7,44 ppm (d,1H), 6,34 ppm (s,1H), 4,69 ppm (m,2H), 3,58 ppm (m,3H) |
| 58 | $C_2F_5$ | $O-(CH_2)_4Cl$ | $^1$H-NMR ($CDCl_3$, 400 MHz): 7,89 ppm (d,1H), 7,39 ppm (d,1H), 6,33 ppm (s,1H), 4,36 ppm (m,2H), 3,58 ppm (m,5H), 1,93 ppm (m,4H) |
| 59 | $C_2F_5$ | $O-CH(CF_3)_2$ | $^1$H-NMR ($CDCl_3$, 400 MHz): 7,96 ppm (d,1H), 7,48 ppm (d,1H), 6,35 ppm (s,1H), 5,99 ppm (m,1H), 3,59 ppm (m,3H) |

| | | | |
|---|---|---|---|
| 60 | $CH_3$ | $S-CH(CH_3)_2$ | $^1H$-NMR ($D_6$-DMSO, 400 MHz): 7,86-7,83 ppm (m,2H), 5,80 ppm (s,1H), 3,76 ppm (Septett,1H), 3,35 ppm (s,3H), 2,33 ppm (s,3H), 1,36 ppm (d,6H) Massenspektrum (m/e): $M^+$ 370(2) |

Beispiele 61-66

Analog dem in Beispiel 32 beschriebenen Verfahren erhält man ausgehend von 2-Chlor-4-fluor-5-[2-methoxy-6-oxo-4-trifluormethyl bzw. pentafluoräthyl-1(6H)-pyrimidinyl]-benzoesäure über deren Säurechlorid und der entsprechenden Hydroxyverbindung H-Q' die in der nachfolgenden Tabelle 7 aufgeführten Enoläther der Verbindungen der Formel I:

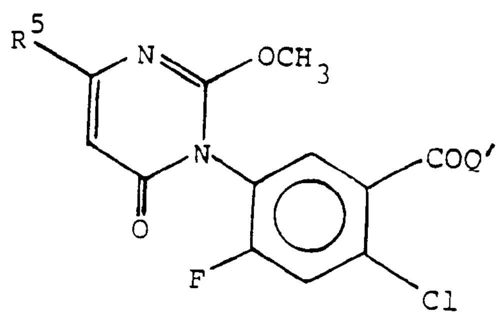

Tabelle 7

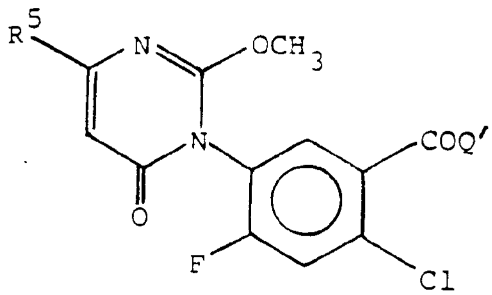

| Beispiel | $R^5$ | Q' | Physikalische Daten |
|---|---|---|---|
| 61 | $CF_3$ | $O-CH_2CH_2Cl$ | $^1H$-NMR ($CDCl_3$,400 MHz): 7,91 ppm (d,1H), 7,43 ppm (d,1H), 6,63 ppm (s,1H), 4,59 ppm (m,2H), 4,03 ppm (s,3H), 3,81 ppm (t,2H) |
| 62 | $CF_3$ | $O-CH(CH_2F)_2$ | $^1H$-NMR ($CDCl_3$,400 MHz): 7,90 ppm (d,1H), 7,44 ppm (d,1H), 6,63 ppm (s,1H), 5,47 ppm (m,1H), 4,73 ppm (m,4H), 4,03 ppm (s,3H) |
| 63 | $CF_3$ | $O-(CH_2)_2-O-N=C(CH_3)_2$ | $^1H$-NMR ($CDCl_3$, 400 MHz): 7,89 ppm (d,1H), 7,40 ppm (d,1H), 6,62 ppm (s,1H), 4,56 ppm (m,2H), 4,32 ppm (m,2H), 4,02 ppm (s,3H), 1,85 ppm (s,3H), 1,84 ppm (s,3H) |

| | | | |
|---|---|---|---|
| 64 | $C_2F_5$ | $O-CH_2CF_3$ | $^1H$-NMR ($CDCl_3$, 400 MHz): 7,93 ppm (d,1H), 7,46 ppm (d,1H), 6,68 ppm (s,1H), 4,71 ppm (m,2H), 4,01 ppm (s,3H) |
| 65 | $C_2F_5$ | $O-(CH_2)_5Cl$ | $^1H$-NMR ($CDCl_3$, 400 MHz): 7,86 ppm (d,1H), 7,41 ppm (d,1H), 6,67 ppm (s,1H), 4,35 ppm (t,2H), 3,99 ppm (s,3H), 3,56 ppm (t,2H), 1,82 ppm (m,4H), 1,61 ppm (m,2H) |
| 66 | $C_2F_5$ | $O-CH(CF_3)_2$ | $^1H$-NMR ($CDCl_3$, 400 MHz): 7,95 ppm (d,1H), 7,50 ppm (d,1H), 6,69 ppm (s,1H), 6,00 ppm (m,1H), 4,02 ppm (s,3H) |

II. Herstellung der Ausgangsmaterialien der Formeln III und IIIa:

Beispiel 67

Die als Ausgangsmaterial in den Beispielen 15-19 und 61-63 verwendete 2-Chlor-4-fluor-5-[2-methoxy-6-oxo-4-trifluormethyl-1(6H)-pyrimidinyl]-benzoesäure kann wie folgt hergestellt werden:

Eine Lösung von 3,55 g 3-Amino-4,4,4-trifluorcrotonsäure-äthylester in 50 ml n-Hexan wird unter Rühren bei 0-3°C während 15 Minuten zu 0,85 g einer 55%igen Natriumhydrid-Dispersion in 50 ml Dimethylformamid zugetropft, und das Gemisch wird 30 Minuten nachgerührt. Anschliessend wird während 5 Minuten unter Rühren und Kühlen eine Lösung von 5,0 g 2-Chlor-4-fluor-5-isocyanatobenzoesäure-isopropylester in 100 ml n-Hexan zugetropft. Die Temperatur des Reaktionsgemisches steigt auf 10°C an, und das Gemisch wird danach eine Stunde bei Raumtemperatur nachgerührt. Das dabei anfallende Zwischenprodukt 2-Chlor-4-fluor-5-{3-[2-(äthoxycarbonyl)-1-trifluormethyl-vinyl]ureido}-benzoesäure-isopropylester wird nicht isoliert.

Man bringt durch Zusatz von konzentrierter Essigsäure das Gemisch auf pH 4, giesst es auf 750 ml Wasser und extrahiert das wässrige Gemisch mit 300 ml Aethylacetat. Die organische Phase wird über wasserfreiem Natriumsulfat getrocknet und anschliessend unter vermindertem Druck zur Trockene eingedampft und der Rückstand aus Diäthyläther/n-Hexan umkristallisiert. Man erhält auf diese Weise den 2-Chlor-5-[3,6-dihydro-4-trifluormethyl -2,6-dioxo-1(2H)-pyrimidinyl]-4-fluorbenzoesäure-isopropylester, Smp. 127-129°C.

Zu einer Suspension von 38,8 g 2-Chlor-5-[3-,6-dihydro-2,6-dioxo-4-trifluormethyl-1(2H)-pyrimidinyl]-4-fluorbenzoesäure-isopropylester in 45,2 g Phosphoroxychlorid und 40 ml Toluol werden bei Raumtemperatur unter Rühren und Kühlen 23,3 g Pyridin zugefügt. Es bildet sich rasch eine Lösung. Die Temperatur wird zwischen 30 bis 35°C gehalten, und nach 15 Minuten beginnt sich ein farbloser Niederschlag abzuscheiden. Man rührt das Reaktionsgemisch 45 Minuten bei 30-35°C nach und giesst es dann auf 300 g Eis. Anschliessend wird mit 200 ml Essigsäure-äthylester ausgeschüttelt, und die organische Phase wird dreimal mit je 50 ml Wasser gewaschen, über wasserfreiem Natriumsulfat getrocknet und unter vermindertem Druck zur Trockene eingedampft. Der Rückstand wird aus n-Hexan umkristallisiert. Man erhält den 2-Chlor-5-[2-chlor-6-oxo-4-trifluormethyl-1(6H)-pyrimidinyl]-4-fluorbenzoesäure-isopropylester, Smp. 72-75°C.

Zu einer Lösung von 42,4 g 2-Chlor-5-[2-chlor-6-oxo-4-trifluormethyl-1(6H)-pyrimidinyl]-4-fluorbenzoesäure-isopropylester in 100 ml absolutem Methanol werden unter Rühren und Kühlen bei 0°C während 5 Minuten 51,3 ml einer 2N-Lösung von Natriummethylat in Methanol zugetropft. Das Reaktionsgemisch wird 10 Minuten bei 0°C nachgerührt und mit konzentrierter Essigsäure auf ca. pH 4 eingestellt. Anschliessend wird das Reaktionsgemisch unter vermindertem Druck zur Trockene eingedampft. Der Rückstand wird in 300 ml

Diäthyläther gelöst und die Lösung zweimal mit je 100 ml Wasser gewaschen. Die organische Phase wird über wasserfreiem Natriumsulfat getrocknet und zur Trockene eingedampft. Der Rückstand wird an einer Kieselgel-Säule unter Verwendung von n-Hexan/Diäthyläther (3:1) als Laufmittel chromatographisch gereinigt und anschliessend aus n-Hexan/Diäthyläther umkristallisiert. Man erhält den 2-Chlor-4-fluor-5-[2-methoxy-6-oxo-4-trifluormethyl -1(6H)-pyrimidinyl]-benzoesäure-isopropylester, Smp. 121-123°C.

Eine Lösung von 5 g 2-Chlor-4-fluor-5-[2-methoxy-6-oxo-4-trifluormethyl -1(6H)-pyrimidinyl]-benzoesäure-isopropylester in 20 ml Methylenchlorid wird unter Rühren und Kühlen bei 20-25°C mit 25 ml konzentrierter Schwefelsäure versetzt. Das Reaktionsgemisch wird 20 Minuten bei Raumtemperatur nachgerührt und auf 100 g Eis gegossen. Die organische Phase wird abgetrennt und die wässrige Phase zweimal mit je 15 ml Aethylacetat ausgeschüttelt,und die vereinigten organischen Phasen werden mit Wasser neutral gewaschen. Anschliessend wird die Lösung über wasserfreiem Natriumsulfat getrocknet und unter vermindertem Druck zur Trockene eingedampft. Der harzige Rückstand wird aus Diäthyläther/n-Hexan umkristallisiert. Man erhält die 2-Chlor-4-fluor-5-[2-methoxy-6-oxo-4-trifluormethyl-1(6H)-pyrimidinyl]-benzoesäure, Smp. 205-210°C.

Beispiele 68-71

Analog zum 4. Teil des Beispiels 67 wird der 2-Chlor-5-[3,6-dihydro-2,6-dioxo-3-methyl-4-substituiertes -1(2H)-pyrimidinyl]-4-fluorbenzoesäure-isopropylester bzw. 2-Chlor-4-fluor-5-[2-methoxy-4-oxo-4-substituiertes-1(6H)-pyrimidinyl]-benzoesäure-isopropylester hydrolysiert, um die in der nachfolgenden Tabelle 8 aufgeführten Ausgangsmaterialien der Formel III und IIIa (Benzoesäure) herzustellen. Die diesbezüglichen Benzoesäure-isopropylester sind bereits in der europäischen Patentpublikation Nr. 195.346 bzw. 260.621 veröffentlicht.

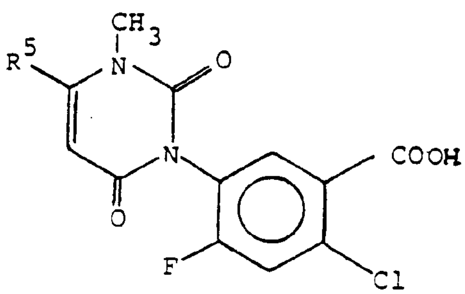

III′

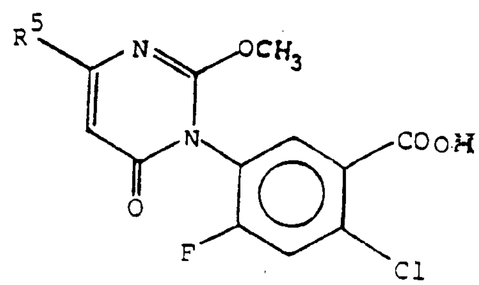

IIIa′

Tabelle 8

| Beispiel | Beispiel-Nr(n) des Endproduktes I bzw. Ia | Formel | $R^5$ | Physikalische Daten |
|---|---|---|---|---|
| 68 | 1-11, 20-26, 32-56 | III' | $CF_3$ | Smp. 239-242°C |
| 69 | 12, 13, 28-30, 60 | III' | $CH_3$ | Smp. 236-239°C |
| 70 | 27, 57-59 | III' | $C_2F_5$ | Smp. 229-231°C |
| 71 | 64-66 | IIIa' | $C_2F_5$ | Smp. 197-199°C |

Beispiel 72

Die als Ausgangsmaterial in den Beispielen 14 und 31 verwendete 2-Chlor-5-[3-difluormethyl-3,6-dihydro-2,6-dioxo-4-methyl-1(2H)-pyrimidinyl]-4-fluorbenzoesäure kann wie folgt hergestellt werden:

In eine Suspension von 145,0 g 2-Chlor-4-fluor-5-[3,6-dihydro-4-methyl-2,6-dioxo-1(2H)-pyrimidinyl]-benzoesäure-isopropylester und 59,0 g wasserfreiem, fein pulverisiertem Kaliumcarbonat in 1 l Dimethylformamid werden unter Rühren bei 80°C während 6 Stunden 103 g Chlordifluormethan eingeleitet. Nach Abkühlen wird der feste Anteil abgenutscht und mit 100 ml Dimethylformamid nachgewaschen. Das Filtrat wird unter vermindertem Druck bei 55°C weitgehend eingeengt, der Rückstand auf 2 l Wasser gegossen und das wässrige Gemisch mit konzentrierter Salzsäure auf pH 3 eingestellt. Das Gemisch wird mit 1,5 l Essigsäure-äthylester ausgeschüttelt und die organische Phase zweimal mit je 1 l Wasser gewaschen, über wasserfreiem Natriumsulfat getrocknet und unter vermindertem Druck zur Trockene eingedampft. Der harzige Rückstand wird an einer Kieselgel-Säule unter Verwendung von Essigsäure-äthylester/n-Hexan (1:1) als Laufmittel chromatographisch gereinigt. Das Produkt wird in 1500 ml heissem n-Hexan gelöst und die Lösung mit Kohle behandelt, filtriert und bei erhöhter Temperatur auf 700 ml eingeengt. Anschliessend wird geimpft und unter Rühren auf 5°C abgekühlt. Die resultierenden Kristalle werden abgenutscht, mit n-Hexan gewaschen und getrocknet. Man erhält den 2-Chlor-5-[3-difluormethyl-3,6-dihydro-4-methyl-2,6-dioxo-1(2H)-pyrimidinyl]-4-fluorbenzoesäure-isopropylester, Smp. 90-93°C.

Eine Lösung von 18,0 g 2-Chlor-5-[3-difluormethyl-3,6-dihydro-4-methyl-2,6-dioxo-1(2H)-pyrimidinyl]-4-fluorbenzoesäure-isopropylester in 60 ml Methylenchlorid wird mit 100 ml konzentrierter Schwefelsäure 5 Minuten intensiv gerührt und anschliessend auf 1 kg Eis gegossen. Die resultierenden Kristalle werden abgenutscht, zweimal mit je 50 ml Wasser gewaschen und in 300 ml Methanol gelöst. Die Mutterlauge wird zweimal mit je 100 ml Methylenchlorid ausgeschüttelt und die organische Phase mit Wasser neutral gewaschen und mit der methanolischen Lösung vereinigt. Diese Lösung wird über wasserfreiem Natriumsulfat getrocknet und zur Trockene eingedampft. Der kristalline Rückstand wird mit 100 ml Essigsäure-äthylester bei 70°C aufgeschlämmt und auf Raumtemperatur abgekühlt, und die Kristalle werden abgenutscht und mit Diäthyläther gewaschen. Man erhält die 2-Chlor-5-[3-difluormethyl-3,6-dihydro-4-methyl-2,6-dioxo-1(2H)-pyrimidinyl]-4-fluorbenzoesäure, Smp. 247-248°C.

III. Formulierungsbeispiele:

Beispiel 73

Ein emulgierbares Konzentrat enthält folgende Bestandteile:

| | |
|---|---|
| Erfindungsgemässe Verbindung (Wirkstoff) | 50 g/l |
| N-Methylpyrrolidon (1. Lösungsmittel) | 200 g/l |
| Nonylphenol-(10)äthoxylat (nichtionogener Emulgator) | 50 g/l |
| Calcium-dodecylbenzolsulfonat (anionischer Emulgator) | 25 g/l |
| Gemisch von Alkylbenzolen (2. Lösungsmittel) | ad 1000 ml |

Der Wirkstoff und die Emulgatoren werden unter Rühren im 1. Lösungsmittel gelöst, und die Lösung wird mit dem 2. Lösungsmittel auf 1 Liter ergänzt.

Das resultierende emulgierbare Konzentrat lässt sich in Wasser emulgieren und ergibt so eine gebrauchsfertige Spritzbrühe mit der gewünschten Konzentration.

Beispiel 74

Ein emulgierbares Konzentrat für gut lösliche erfindungsgemässe Verbindungen enthält folgende Bestandteile:

| | |
|---|---|
| Erfindungsgemässe Verbindung (Wirkstoff) | 250 g/l |
| Oleylalkohol-polyäthoxylat (ca. 1 Mol Alkohol: 10 Mol $C_2H_4O$; nichtionogener Emulgator) | 50 g/l |
| Calcium-dodecylbenzolsulfonat (anionischer Emulgator) | 25 g/l |
| Gemisch von Alkylbenzolen (Lösungsmittel) ad | 1000 ml |

Der Wirkstoff und die Emulgatoren werden unter Rühren in einem Teil des Lösungsmittels gelöst, und die Lösung wird mit dem restlichen Lösungsmittel auf 1 Liter ergänzt.

Das resultierende emulgierbare Konzentrat lässt sich in Wasser emulgieren und ergibt so eine gebrauchsfertige Spritzbrühe mit der gewünschten Konzentration.

Beispiel 75

Zur Herstellung eines 25% Spritzpulvers werden die nachstehend aufgeführten Bestandteile miteinander vermischt:

| | |
|---|---|
| Erfindungsgemässe Verbindung (Wirkstoff) | 25 g |
| Kieselsäure, hydratisiert (Trägerstoff, Mahlhilfsmittel) | 5 g |
| Natrium-laurylsulfat (Netzmittel) | 1 g |
| Natrium-lignosulfonat (Dispergator) | 2 g |
| Kaolin (Trägerstoff) | 67 g |
| | 100 g |

Anschliessend wird das Gemisch unter Verwendung einer Stiftmühle oder vergleichbaren Mahleinrichtung fein gemahlen.

Das resultierende Spritzpulver ergibt beim Einrühren in Wasser eine feine Suspension, die sich als gebrauchsfertige Spritzbrühe eignet.

## Patentansprüche

1. Verbindungen der allgemeinen Formel

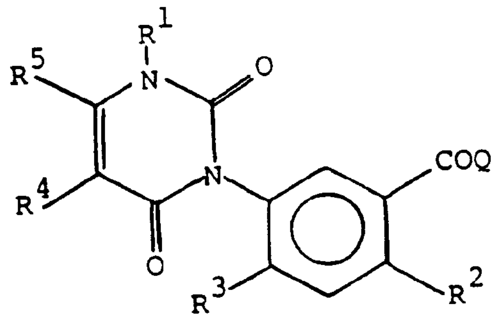

I

worin

$R^1$ Wasserstoff, $C_{1-4}$-Alkyl, $C_{1-4}$-Halogenalkyl, $C_{2-5}$-Alkenyl oder $C_{3-5}$-Alkinyl,
$R^2$ Halogen oder Cyano,
$R^3$ Wasserstoff oder Halogen,
$R^4$ Wasserstoff, Halogen oder $C_{1-4}$-Alkyl,
$R^5$ $C_{1-4}$-Alkyl oder, im Falle, dass $R^1$ verschieden von $C_{1-4}$-Halogenalkyl ist, auch $C_{1-4}$-Halogenalkyl

und

Q eine der Gruppen (a) bis (d) (falls $R^1$ verschieden von Wasserstoff ist) bzw. eine Gruppe (c) oder (d) (falls $R^1$ für Wasserstoff steht) bedeuten

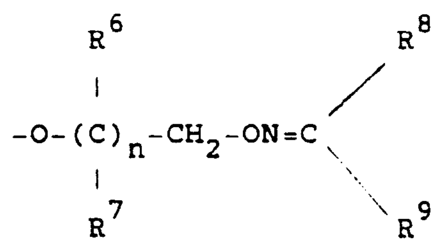

(a)

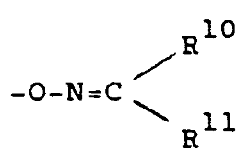

(b)

$-O-R^{12}$ (c)
$-S-R^{13}$ (d)

worin

$R^6$ Wasserstoff oder $C_{1-4}$-Alkyl,
$R^7$ Wasserstoff, $C_{1-4}$-Alkyl, Phenyl oder Benzyl,
$R^8$ Wasserstoff oder $C_{1-6}$-Alkyl,
$R^9$ $C_{1-6}$-Alkyl, $C_{3-6}$-Cycloalkyl oder Phenyl,

oder

$R^8$ und $R^9$ zusammen Tri-, Tetra-, Penta- oder Hexamethylen,
n 0 oder 1,
$R^{10}$ $C_{1-6}$-Alkyl, $C_{1-6}$-Alkylthio, $C_{2-7}$-Alkoxycarbonyl oder $C_{2-7}$-Alkoxycarbonyl-$C_{1-4}$-alkyl,
$R^{11}$ $C_{1-6}$-Alkyl, Trifluormethyl, $C_{1-6}$-Alkoxy-$C_{1-4}$-alkyl, $C_{2-7}$-Alkoxycarbonyl-$C_{1-4}$-alkyl, Di($C_{2-7}$-alkoxycarbonyl)-$C_{1-4}$-alkyl, $C_{3-6}$-Cycloalkyl, $C_{1-6}$-Alkoxy, $C_{1-6}$-Alkylthio, $C_{2-7}$-Alkanoyl, $C_{2-7}$-Alkoxycarbonyl, Phenyl oder 2-Furyl,

oder

$R^{10}$ und $R^{11}$ zusammen mit dem Kohlenstoffatom, an das sie geknüpft sind, einen gegebenenfalls mit 1 bis 3 $C_{1-4}$-Alkylgruppen substituierten Cyclopentan- oder Cyclohexanring,

$R^{12}$ $C_{1-8}$-Halogenalkyl, $C_{3-5}$-Halogenalkenyl oder $C_{3-5}$-Halogenalkinyl,

und

$R^{13}$ Wasserstoff, $C_{1-8}$-Alkyl, $C_{3-8}$-Alkenyl, $C_{3-8}$-Alkinyl, $C_{2-6}$-Alkoxyalkyl oder gegebenenfalls mit 1 bis 3 $C_{1-4}$-Alkylgruppen substituiertes $C_{3-8}$-Cycloalkyl, Phenyl oder Benzyl bedeuten,

und die Enoläther derjenigen Verbindungen der Formel I, in denen $R^1$ $C_{1-4}$-Alkyl, $C_{2-5}$-Alkenyl oder $C_{3-5}$-Alkinyl und Q eine Gruppe (b), (c) oder (d) bedeuten, sowie Salze derjenigen Verbindungen der Formel I bzw. Enoläther, in denen $R^1$ und/oder $R^{13}$ Wasserstoff bedeutet.

2. Verbindungen nach Anspruch 1, worin $R^9$ verschieden von $C_{3-6}$-Cycloalkyl und $R^{11}$ verschieden von Phenyl sind.

3. Verbindungen nach Anspruch 1 oder 2, worin $R^1$ geradkettiges $C_{1-4}$-Alkyl, insbesondere Methyl, oder Difluormethyl bedeutet.

4. Verbindungen nach einem der Ansprüche 1 bis 3, worin $R^2$ Chlor oder Brom und $R^3$ Wasserstoff oder Fluor bedeuten.

5. Verbindungen nach einem der Ansprüche 1 bis 4, worin $R^4$ Wasserstoff, Fluor oder Methyl bedeutet.

6. Verbindungen nach einem der Ansprüche 1 bis 5, worin $R^5$ Methyl, Trifluormethyl oder Pentafluoräthyl bedeutet.

7. Eine Verbindung nach Anspruch 1, ausgewählt aus

2-Chlor-5-[3,6-dihydro-2,6-dioxo-3-methyl-4-trifluormethyl-1(2H)-pyrimidinyl]-4-fluorbenzoesäure-{2-[(isopropylidenamino)oxy]-äthyl}ester,

2-Chlor-5-[3,6-dihydro-2,6-dioxo-3-methyl-4-trifluormethyl-1(2H)-pyrimidinyl]-4-fluorbenzoesäure-{2-[(cyclohexylidenamino)oxy]-äthyl}ester,

2-Chlor-5-[3,6-dihydro-2,6-dioxo-3-methyl-4-trifluormethyl-1(2H)-pyrimidinyl]-4-fluorbenzoesäure-[α-{[(isopropylidenamino)oxy]methyl}-benzyl]ester,

1-Cyclopropyl-1-äthanon-O-{2-chlor-5-[3,6-dihydro-2,6-dioxo-3-methyl-4-trifluormethyl-1(2H)-pyrimidinyl]-4-fluorbenzoyl}oxim,

1-[4-Chlor-2-fluor-5-{[(isopropylidenamino)oxy]carbonyl}-phenyl]-3-methyl-4-trifluormethyl-2,6(1H, 3H)-pyrimidindion,

2-Furylmethylketon-O-{2-chlor-5-[3,6-dihydro-2,6-dioxo-3-methyl-4-trifluormethyl-1(2H)-pyrimidinyl]-4-fluorbenzoyl}oxim,

2,3-Butandion-2-[O-{2-Chlor-5-[3,6-dihydro-2,6-dioxo-3-methyl-4-trifluormethyl-1(2H)-pyrimidinyl]-4-fluorbenzoyl}]oxim,

2-Chlor-4-fluor-5-[2-methoxy-6-oxo-4-trifluormethyl-1(6H)-pyrimidinyl]-benzoesäure-(2-fluor-1-fluormethyläthyl)ester,

2-Chlor-4-fluor-5-[2-methoxy-6-oxo-4-trifluormethyl-1(6H)-pyrimidinyl]-benzoesäure-(2-chloräthyl) ester,

2-Chlor-5-[3,6-dihydro-2,6-dioxo-3-methyl-4-trifluormethyl-1(2H)-pyrimidinyl]-4-fluorbenzoesäure-(2-fluoräthyl)ester,

2-Chlor-4-fluor-5-[2-methoxy-6-oxo-4-trifluormethyl-1(6H)-pyrimidinyl]-benzoesäure-(3-chlor-2-butenyl)ester,

2-Chlor-5-[3,6-dihydro-2,6-dioxo-3-methyl-4-trifluormethyl-1(2H)-pyrimidinyl]-4-fluorbenzoesäure-(3-chlor-2-butenyl)ester,

2-Chlor-4-fluor-5-[2-methoxy-6-oxo-4-trifluormethyl-1(6H)-pyrimidinyl]-benzoesäure-(2-fluoräthyl) ester,

2-Chlor-5-[3,6-dihydro-2,6-dioxo-3-methyl-4-trifluormethyl-1(2H)-pyrimidinyl]-4-fluor-thiobenzoesäure-S-isopropylester,

2-Chlor-5-[3,6-dihydro-2,6-dioxo-3-methyl-4-trifluormethyl-1(2H)-pyrimidinyl]-4-fluorthiobenzoesäure-S-äthylester,

2-Chlor-5-[3,6-dihydro-2,6-dioxo-3-methyl-4-trifluormethyl-1(2H)-pyrimidinyl]-4-fluorthiobenzoesäure-S-(n-butyl)ester,

2-Chlor-5-[3,6-dihydro-2,6-dioxo-3-methyl-4-trifluormethyl-1(2H)-pyrimidinyl]-4-fluorthiobenzoesäure-S-allylester,

2-Chlor-5-[3,6-dihydro-2,6-dioxo-3-methyl-4-trifluormethyl-1(2H)-pyrimidinyl]-4-fluorthiobenzoesäure-S-cyclohexylester,

2-Chlor-5-[3,6-dihydro-2,6-dioxo-3-methyl-4-trifluormethyl-1(2H)-pyrimidinyl]-4-fluorthiobenzoesäure-S-phenylester,

2-Chlor-5-[3,6-dihydro-2,6-dioxo-3-methyl-4-trifluormethyl-1(2H)-pyrimidinyl]-4-fluorthiobenzoesäure-S-benzylester und

2-Chlor-5-[3,6-dihydro-3,4-dimethyl-2,6-dioxo-1(2H)-pyrimidinyl]-4-fluorthiobenzoesäure-S-isopropylester.

8. Unkrautbekämpfungsmittel, dadurch gekennzeichnet, dass es eine wirksame Menge mindestens einer Verbindung der allgemeinen Formel

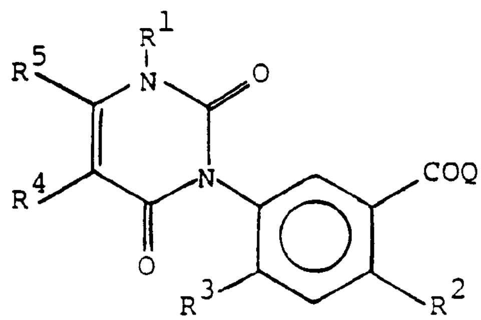

I

worin

$R^1$ Wasserstoff, $C_{1-4}$-Alkyl, $C_{1-4}$-Halogenalkyl, $C_{2-5}$-Alkenyl oder $C_{3-5}$-Alkinyl,

$R^2$ Halogen oder Cyano,

$R^3$ Wasserstoff oder Halogen,

$R^4$ Wasserstoff, Halogen oder $C_{1-4}$-Alkyl,

$R^5$ $C_{1-4}$-Alkyl oder, im Falle, dass $R^1$ verschieden von $C_{1-4}$-Halogenalkyl ist, auch $C_{1-4}$-Halogenalkyl

und

Q eine der Gruppen (a) bis (d) (falls $R^1$ verschieden von Wasserstoff ist) bzw. eine Gruppe (c) oder (d) (falls $R^1$ für Wasserstoff steht) bedeuten

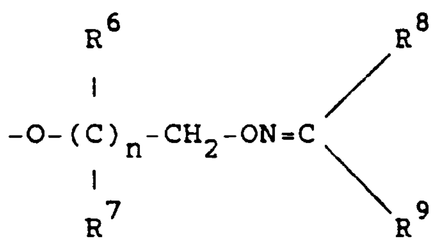

(a)

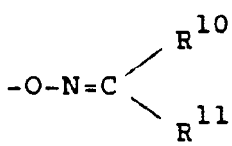

(b)

$-O-R^{12}$ (c)

$-S-R^{13}$ (d)

worin

$R^6$ Wasserstoff oder $C_{1-4}$-Alkyl,

$R^7$ Wasserstoff, $C_{1-4}$-Alkyl, Phenyl oder Benzyl,

$R^8$ Wasserstoff oder $C_{1-6}$-Alkyl,

$R^9$ $C_{1-6}$-Alkyl, $C_{3-6}$-Cycloalkyl oder Phenyl,

oder

$R^8$ und $R^9$ zusammen Tri-, Tetra-, Penta- oder Hexamethylen,

n 0 oder 1,

$R^{10}$ $C_{1-6}$-Alkyl, $C_{1-6}$-Alkylthio, $C_{2-7}$-Alkoxycarbonyl oder $C_{2-7}$-Alkoxycarbonyl-$C_{1-4}$-alkyl,

$R^{11}$ $C_{1-6}$-Alkyl, Trifluormethyl, $C_{1-6}$-Alkoxy-$C_{1-4}$-alkyl, $C_{2-7}$-Alkoxycarbonyl-$C_{1-4}$-alkyl, Di($C_{2-7}$-alkoxycarbonyl)-$C_{1-4}$-alkyl, $C_{3-6}$-Cycloalkyl, $C_{1-6}$-Alkoxy, $C_{1-6}$-Alkylthio, $C_{2-7}$-Alkanoyl, $C_{2-7}$-Alkoxycarbonyl, Phenyl oder 2-Furyl,

oder

$R^{10}$ und $R^{11}$ zusammen mit dem Kohlenstoffatom, an das sie geknüpft sind, einen gegebenenfalls mit 1 bis 3 $C_{1-4}$-Alkylgruppen substituierten Cyclopentan- oder Cyclohexanring,

$R^{12}$ $C_{1-8}$-Halogenalkyl, $C_{3-5}$-Halogenalkenyl oder $C_{3-5}$-Halogenalkinyl,

und

$R^{13}$ Wasserstoff, $C_{1-8}$-Alkyl, $C_{3-8}$-Alkenyl, $C_{3-8}$-Alkinyl, $C_{2-6}$-Alkoxyalkyl oder gegebenenfalls mit 1 bis 3 $C_{1-4}$-Alkylgruppen substituiertes $C_{3-8}$-Cycloalkyl, Phenyl oder Benzyl bedeuten,

oder eines Enoläthers einer solchen Verbindung der Formel I, in der $R^1$ $C_{1-4}$-Alkyl, $C_{2-5}$-Alkenyl oder $C_{3-5}$-Alkinyl und Q eine Gruppe (b), (c) oder (d) bedeuten, oder eines Salzes einer solchen Verbindung der Formel I bzw. eines solchen Enoläthers, in der bzw. dem $R^1$ und/oder $R^{13}$ Wasserstoff bedeutet,

sowie Formulierungshilfsstoffe enthält.

9. Unkrautbekämpfungsmittel nach Anspruch 8, worin $R^9$ verschieden von $C_{3-6}$-Cycloalkyl und $R^{11}$ verschieden von Phenyl sind.

10. Unkrautbekämpfungsmittel, dadurch gekennzeichnet, dass es eine wirksame Menge mindestens einer aus der Gruppe

2-Chlor-5-[3,6-dihydro-2,6-dioxo-3-methyl-4-trifluormethyl-1(2H)-pyrimidinyl]-4-fluorbenzoesäure-{2-[(isopropylidenamino)oxy]-äthyl}ester,

2-Chlor-5-[3,6-dihydro-2,6-dioxo-3-methyl-4-trifluormethyl-1(2H)-pyrimidinyl]-4-fluorbenzoesäure-{2-[(cyclohexylidenamino)oxy]-äthyl}ester,

2-Chlor-5-[3,6-dihydro-2,6-dioxo-3-methyl-4-trifluormethyl-1(2H)-pyrimidinyl]-4-fluorbenzoesäure-[α-{[(isopropylidenamino)oxy]-methyl}-benzyl]ester,

1-Cyclopropyl-1-äthanon-O-{2-chlor-5-[3,6-dihydro-2,6-dioxo-3-methyl-4-trifluormethyl-1(2H)-pyrimidinyl]-4-fluorbenzoyl}oxim,

1-[4-Chlor-2-fluor-5-{[(isopropylidenamino)oxy]carbonyl}-phenyl]-3-methyl-4-trifluormethyl-2,6(1H, 3H)-pyrimidindion,

2-Furylmethylketon-O-{2-chlor-5-[3,6-dihydro-2,6-dioxo-3-methyl-4-trifluormethyl-1(2H)-pyrimidinyl]-4-fluorbenzoyl}oxim,

2,3-Butandion-2-[O-{2-chlor-5-[3,6-dihydro-2,6-dioxo-3-methyl-4-trifluormethyl-1(2H)-pyrimidinyl]-4-fluorbenzoyl}]oxim,

2-Chlor-4-fluor-5-[2-methoxy-6-oxo-4-trifluormethyl-1(6H)-pyrimidinyl]-benzoesäure-(2-fluor-1-fluormethyläthyl)ester,

2-Chlor-4-fluor-5-[2-methoxy-6-oxo-4-trifluormethyl-1(6H)-pyrimidinyl]-benzoesäure-(2-chloräthyl) ester,

2-Chlor-5-[3,6-dihydro-2,6-dioxo-3-methyl-4-trifluormethyl-1(2H)-pyrimidinyl]-4-fluorbenzoesäure-(2-fluoräthyl)ester,

2-Chlor-4-fluor-5-[2-methoxy-6-oxo-4-trifluormethyl-1(6H)-pyrimidinyl]-benzoesäure-(3-chlor-2-butenyl)ester,

2-Chlor-5-[3,6-dihydro-2,6-dioxo-3-methyl-4-trifluormethyl-1(2H)-pyrimidinyl]-4-fluorbenzoesäure-(3-chlor-2-butenyl)ester,

2-Chlor-4-fluor-5-[2-methoxy-6-oxo-4-trifluormethyl-1(6H)-pyrimidinyl]-benzoesäure-(2-fluoräthyl) ester,

2-Chlor-5-[3,6-dihydro-2,6-dioxo-3-methyl-4-trifluormethyl-1(2H)-pyrimidinyl]-4-fluor-thiobenzoesäure-S-isopropylester,

2-Chlor-5-[3,6-dihydro-2,6-dioxo-3-methyl-4-trifluormethyl-1(2H)-pyrimidinyl]-4-fluorthiobenzoesäure-S-äthylester,

2-Chlor-5-[3,6-dihydro-2,6-dioxo-3-methyl-4-trifluormethyl-1(2H)-pyrimidinyl]-4-fluorthiobenzoesäure-S-(n-butyl)ester,

2-Chlor-5-[3,6-dihydro-2,6-dioxo-3-methyl-4-trifluormethyl-1(2H)-pyrimidinyl]-4-fluorthiobenzoesäure-S-allylester,

2-Chlor-5-[3,6-dihydro-2,6-dioxo-3-methyl-4-trifluormethyl-1(2H)-pyrimidinyl]-4-fluorthiobenzoesäure-S-cyclohexylester,

2-Chlor-5-[3,6-dihydro-2,6-dioxo-3-methyl-4-trifluormethyl-1(2H)-pyrimidinyl]-4-fluorthiobenzoesäure-S-phenylester,

2-Chlor-5-[3,6-dihydro-2,6-dioxo-3-methyl-4-trifluormethyl-1(2H)-pyrimidinyl]-4-fluorthiobenzoesäure-S-benzylester und

2-Chlor-5-[3,6-dihydro-3,4-dimethyl-2,6-dioxo-1(2H)-pyrimidinyl]-4-fluorthiobenzoesäure-S-isopropylester

ausgewählten Verbindung sowie Formulierungshilfsstoffe enthält.

**11.** Verfahren zur Herstellung von Verbindungen der allgemeinen Formel

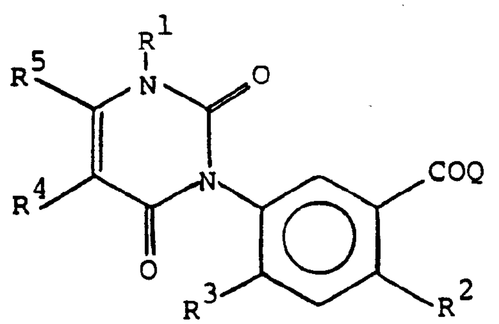

I

worin

$R^1$ Wasserstoff, $C_{1-4}$-Alkyl, $C_{1-4}$-Halogenalkyl, $C_{2-5}$-Alkenyl oder $C_{3-5}$-Alkinyl,

$R^2$ Halogen oder Cyano,

$R^3$ Wasserstoff oder Halogen,

$R^4$ Wasserstoff, Halogen oder $C_{1-4}$-Alkyl,

$R^5$ $C_{1-4}$-Alkyl oder, im Falle, dass $R^1$ verschieden von $C_{1-4}$-Halogenalkyl ist, auch $C_{1-4}$-Halogenalkyl

und

Q eine der Gruppen (a) bis (d) (falls $R^1$ verschieden von Wasserstoff ist) bzw. eine Gruppe (c) oder (d) (falls $R^1$ für Wasserstoff steht) bedeuten

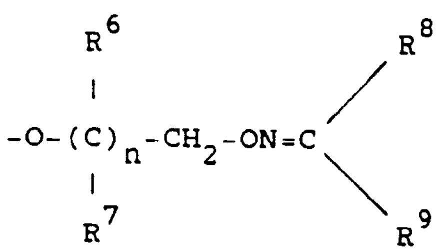

(a)

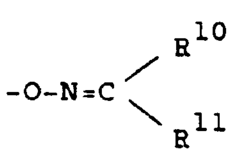

(b)

$-O-R^{12}$ (c)

$-S-R^{13}$ (d)

worin

$R^6$ Wasserstoff oder $C_{1-4}$-Alkyl,

$R^7$ Wasserstoff, $C_{1-4}$-Alkyl, Phenyl oder Benzyl,

$R^8$ Wasserstoff oder $C_{1-6}$-Alkyl,

$R^9$ $C_{1-6}$-Alkyl, $C_{3-6}$-Cycloalkyl oder Phenyl,

oder

$R^8$ und $R^9$ zusammen Tri-, Tetra-, Penta- oder Hexamethylen,

n 0 oder 1,

$R^{10}$ $C_{1-6}$-Alkyl, $C_{1-6}$-Alkylthio, $C_{2-7}$-Alkoxycarbonyl oder $C_{2-7}$-Alkoxycarbonyl-$C_{1-4}$-alkyl,

$R^{11}$ $C_{1-6}$-Alkyl, Trifluormethyl, $C_{1-6}$-Alkoxy-$C_{1-4}$-alkyl, $C_{2-7}$-Alkoxycarbonyl-$C_{1-4}$-alkyl, Di($C_{2-7}$-alkoxycarbonyl)-$C_{1-4}$-alkyl, $C_{3-6}$-Cycloalkyl, $C_{1-6}$-Alkoxy, $C_{1-6}$-Alkylthio, $C_{2-7}$-Alkanoyl, $C_{2-7}$-Alkoxycarbonyl, Phenyl oder 2-Furyl,

oder

$R^{10}$ und $R^{11}$ zusammen mit dem Kohlenstoffatom, an das sie geknüpft sind, einen gegebenenfalls mit 1 bis 3 $C_{1-4}$-Alkylgruppen substituierten Cyclopentan- oder Cyclohexanring,

$R^{12}$ $C_{1-8}$-Halogenalkyl, $C_{3-5}$-Halogenalkenyl oder $C_{3-5}$-Halogenalkinyl,

und

$R^{13}$ Wasserstoff, $C_{1-8}$-Alkyl, $C_{3-8}$-Alkenyl, $C_{3-8}$-Alkinyl, $C_{2-6}$-Alkoxyalkyl oder gegebenenfalls mit 1 bis 3 $C_{1-4}$-Alkylgruppen substituiertes $C_{3-8}$-Cycloalkyl, Phenyl oder Benzyl bedeuten,

und von den entsprechenden Enoläthern derjenigen Verbindungen der Formel I, in denen $R^1$ $C_{1-4}$-Alkyl, $C_{2-5}$-Alkenyl oder $C_{3-5}$-Alkinyl und Q eine Gruppe (b), (c) oder (d) bedeuten, sowie von Salzen derjenigen Verbindungen der Formel I oder Enoläther, in denen $R^1$ und/oder $R^{13}$ Wasserstoff bedeutet, dadurch gekennzeichnet, dass man

a) zwecks Herstellung derjenigen Verbindungen der Formel I, in denen $R^1$ Wasserstoff und Q eine Gruppe (c) oder (d) bedeuten, sowie gewünschtenfalls von Metallsalzen dieser Verbindungen, eine Verbindung der allgemeinen Formel

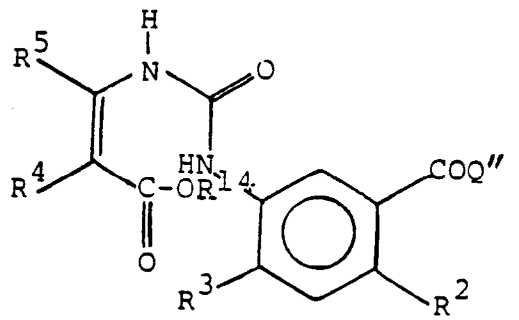

II

worin $R^2$, $R^3$, $R^4$ und $R^5$ die oben angegebenen Bedeutungen besitzen, Q″ eine Gruppe (c) oder (d), wie diese oben definiert ist, bedeutet und $R^{14}$ nieder Alkyl bedeutet,

einer Cyclisierung unter basischen Bedingungen unterwirft und gewünschtenfalls ein allfällig erhaltenes Metallsalz des Uracilderivats der Formel I durch Behandlung mit einer Säure in die saure Form ($R^1$ = Wasserstoff) überführt,

b) zwecks Herstellung derjenigen Verbindungen der Formel I, in denen $R^1$ $C_{1-4}$-Alkyl, $C_{1-4}$-Halogenalkyl, $C_{2-5}$-Alkenyl oder $C_{3-5}$-Alkinyl und Q eine der Gruppen (a) bis (d) bedeutet, und der Enoläther dieser Verbindungen eine Benzoesäure der allgemeinen Formel

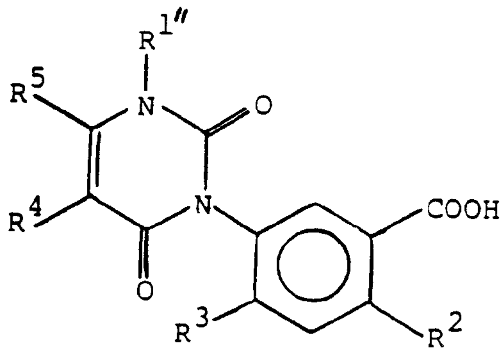

III

worin $R^2$, $R^3$, $R^4$ und $R^5$ die oben angegebenen Bedeutungen besitzen und $R^{1''}$ $C_{1-4}$-Alkyl, $C_{1-4}$-Halogenalkyl, $C_{2-5}$-Alkenyl oder $C_{3-5}$-Alkinyl bedeutet,
oder ein reaktionsfähiges Derivat dieser Benzoesäure mit einer Hydroxy- bzw. Mercaptoverbindung der allgemeinen Formel

H-Q IV

worin Q die oben angegebene Bedeutung besitzt, oder mit einem reaktionsfähigen Derivat dieser Hydroxy- bzw. Mercaptoverbindung verestert bzw. einen Enoläther dieser Benzoesäure, und zwar der allgemeinen Formel

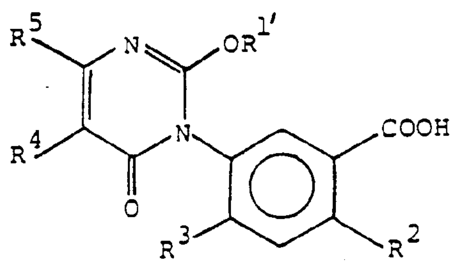

oder

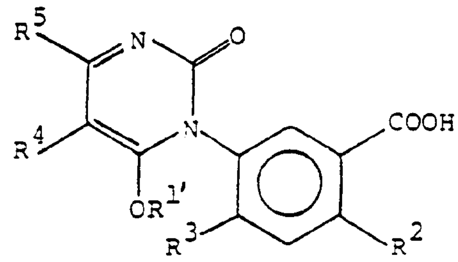

III a III b

worin $R^{1'}$, $R^2$, $R^3$, $R^4$ und $R^5$ die oben angegebenen Bedeutungen besitzen,
oder ein reaktionsfähiges Derivat dieses Enoläthers mit einer Hydroxy- bzw. Mercaptoverbindung der allgemeinen Formel

H-Q' IV'

worin Q' eine Gruppe (b), (c) oder (d), wie diese oben definiert ist, bedeutet,
oder mit einem reaktionsfähigen Derivat dieser Hydroxy- bzw. Mercaptoverbindung verestert,

c) zwecks Herstellung derjenigen Verbindungen der Formel I, in denen $R^1$ $C_{1-4}$-Alkyl, $C_{1-4}$-Halogenalkyl, $C_{2-5}$-Alkenyl oder $C_{3-5}$-Alkinyl und Q eine Gruppe (c) bedeuten, einen Benzoesäureester der allgemeinen Formel

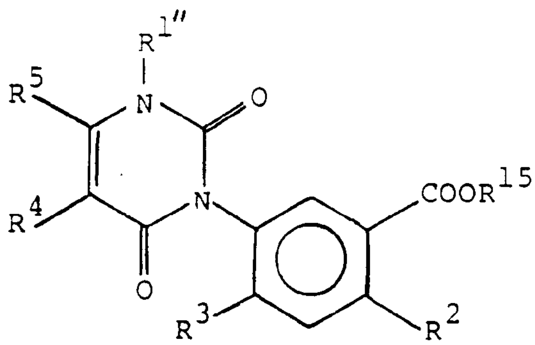

V

worin $R^{1''}$, $R^2$, $R^3$, $R^4$ und $R^5$ die oben angegebenen Bedeutungen besitzen und $R^{15}$ $C_{1-6}$-Alkyl, $C_{2-4}$-Alkenyl, $C_{2-4}$-Alkinyl oder $C_{2-6}$-Alkoxyalkyl bedeutet,
einer Umesterungsreaktion mit einer Hydroxyverbindung der allgemeinen Formel

H-Q''' IV''

worin Q''' eine Gruppe (c) bedeutet,
unterwirft, wobei das Reagens IV'' höhersiedend ist als die sich bildende Hydroxyverbindung $R^{15}OH$, oder

d) zwecks Herstellung der Enoläther derjenigen Verbindungen der Formel I, in denen $R^1$ $C_{1-4}$-Alkyl, $C_{2-5}$-Alkenyl oder $C_{3-5}$-Alkinyl und Q eine Gruppe (c) oder (d) bedeuten, eine Pyrimidinonderivat der allgemeinen Formel

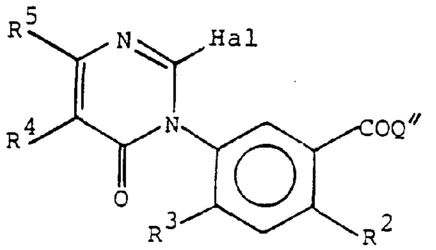

oder

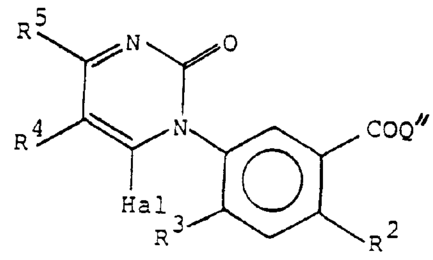

VIa

VIb

worin $R^2$, $R^3$, $R^4$, $R^5$ und Q″ die oben angegebenen Bedeutungen besitzen und Hal Chlor oder Brom bedeutet,
mit der deprotonierten Form eines Alkanols, Alkenols oder Alkinols $R^{1\prime}OH$, worin $R^{1\prime}$ die oben angegebene Bedeutung besitzt, behandelt,
und gewünschtenfalls eine so erhaltene Verbindung der Formel I, in der $R^1$ und/oder $R^{13}$ Wasserstoff bedeutet, in ein Salz überführt.

**12.** Verfahren nach Anspruch 11, worin $R^9$ verschieden von $C_{3-6}$-Cycloalkyl und $R^{11}$ verschieden von Phenyl sind.

**13.** Verfahren zur Bekämpfung von Unkräutern, dadurch gekennzeichnet, dass man das gegen Unkräuter zu schützende Gut und/oder die Unkräuter mit einer wirksamen Menge einer Verbindung gemäss einem der Ansprüche 1 bis 7 bzw. eines Mittels gemäss Anspruch 8, 9 oder 10 behandelt.

**14.** Verwendung einer Verbindung gemäss einem der Ansprüche 1 bis 7 bzw. eines Mittels gemäss Anspruch 8, 9 oder 10 zur Bekämpfung von Unkräutern.

## Claims

**1.** A compound of the general formula

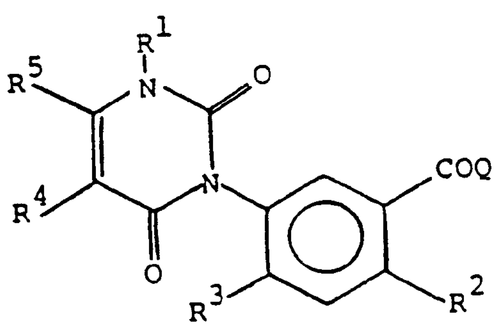

I

wherein
$R^1$ signifies hydrogen, $C_{1-4}$alkyl, $C_{1-4}$haloalkyl, $C_{2-5}$alkenyl or $C_{3-5}$alkynyl, $R^2$ signifies halogen or cyano, $R^3$ signifies hydrogen or halogen, $R^4$ signifies hydrogen, halogen or $C_{1-4}$alkyl, $R^5$ signifies $C_{1-4}$alkyl or, when R′ is different from $C_{1-4}$haloalkyl, also $C_{1-4}$haloalkyl and Q signifies one of the groups (a) to (d) (where $R^1$ is different from hydrogen) or a group (c) or (d) (where $R^1$ stands for hydrogen)

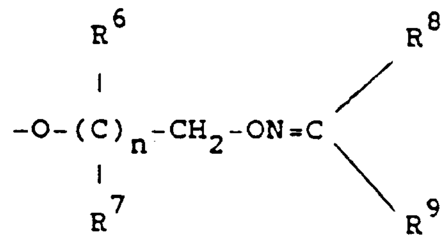

(a)

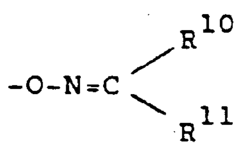

(b)

$-O-R^{12}$ (c)

$-S-R^{13}$ (d)

wherein

$R^6$ signifies hydrogen or $C_{1-4}$alkyl,

$R^7$ signifies hydrogen, $C_{1-4}$alkyl, phenyl or benzyl,

$R^8$ signifies hydrogen or $C_{1-6}$alkyl,

$R^9$ signifies $C_{1-6}$alkyl, $C_{3-6}$cycloalkyl or phenyl,

or

$R^8$ and $R^9$ together signify tri-, tetra-, penta- or hexamethylene,

n signifies 0 or 1,

$R^{10}$ signifies $C_{1-6}$alkyl, $C_{1-6}$alkylthio, $C_{2-7}$alkoxycarbonyl or $C_{2-7}$alkoxycarbonyl-$C_{1-4}$alkyl,

$R^{11}$ signifies $C_{1-6}$alkyl, trifluoromethyl, $C_{1-6}$alkoxy- $C_{1-4}$alkyl, $C_{2-7}$alkoxycarbonyl-$C_{1-4}$alkyl, di($C_{2-7}$alkoxycarbonyl)-$C_{1-4}$alkyl, $C_{3-6}$cycloalkyl, $C_{1-6}$alkoxy, $C_{1-6}$alkylthio, $C_{2-7}$alkanoyl, $C_{2-7}$alkoxycarbonyl, phenyl or 2-furyl,

or

$R^{10}$ and $R^{11}$ together with the carbon atom to which they are attached signify a cyclopentane or cyclohexane ring optionally substituted with 1 to 3 $C_{1-4}$alkyl groups,

$R^{12}$ signifies $C_{1-8}$haloalkyl, $C_{3-5}$haloalkenyl or $C_{3-5}$haloalkynyl,

and

$R^{13}$ signifies hydrogen, $C_{1-8}$alkyl, $C_{3-8}$alkenyl, $C_{3-8}$alkynyl or $C_{2-6}$alkoxyalkyl, or $C_{3-8}$cycloalkyl, phenyl or benzyl optionally substituted with 1 to 3 $C_{1-4}$alkyl groups, or an enol ether of those compounds of formula I in which $R^1$ signifies $C_{1-4}$alkyl, $C_{2-5}$alkenyl or $C_{3-5}$alkynyl and Q signifies a group (b), (c) or (d) or else a salt of those compounds of formula I and, respectively, enol ethers in which $R^1$ and/or $R^{13}$ signifies hydrogen.

**2.** A compound according to claim 1, wherein $R^9$ is different from $C_{3-6}$cycloalkyl and $R^{11}$ is different from phenyl.

**3.** A compound according to claim 1 or 2, wherein $R^1$ signifies straight-chain $C_{1-4}$alkyl, especially methyl, or difluoromethyl.

**4.** A compound according to any one of claims 1 to 3, wherein $R^2$ signifies chlorine or bromine and $R^3$ signifies hydrogen or fluorine.

**5.** A compound according to any one of claims 1 to 4, wherein $R^4$ signifies hydrogen, fluorine or methyl.

**6.** A compound according to any one of claims 1 to 5, wherein $R^5$ signifies methyl, trifluoromethyl or pentafluoroethyl.

**7.** A compound according to claim 1, selected from
2-[(isopropylideneamino)oxy]-ethyl 2-chloro-5-[3,6-dihydro-2,6-dioxo-3-methyl-4-trifluoromethyl-1(2H)-pyrimidinyl]-4-fluorobenzoate,

2-[(cyclohexylideneamino)oxy]-ethyl 2-chloro-5-[3,6-dihydro-2,6-dioxo-3-methyl-4-trifluoromethyl-1(2H)-pyrimidinyl]-4-fluorobenzoate,
α-{[(isopropylideneamino)oxy]methyl}-benzyl]2-chloro-5-[3,6-dihydro-2,6-dioxo-3-methyl-4-trifluoromethyl-1(2H)-pyrimidinyl]-4-fluorobenzoate,
1-cyclopropyl-1-ethanone O-{2-chloro-5-[3,6-dihydro-2,6-dioxo-3-methyl-4-trifluoromethyl-1(2H)-pyrimidinyl]-4-fluorobenzoyl} oxime,
1-[4-chloro-2-fluoro-5-{[(isopropylideneamino)oxy]-carbonyl}-phenyl]-3-methyl-4-trifluoromethyl-2,6-(1H, 3H)-pyrimidinedione,
2-furyl methyl ketone O-{2-chloro-5-[3,6-dihydro-2,6-dioxo-3-methyl-4-trifluoromethyl-1(2H)pyrimidinyl]-4-fluorobenzoyl} oxime,
2,3-butanedione 2-[O-{2-chloro-5-[3,6-dihydro-2,6-dioxo-3-methyl-4-trifluoromethyl-1(2H)-pyrimidinyl]-4-fluorobenzoyl}] oxime,
2-fluoro-1-fluoromethylethyl 2-chloro-4-fluoro-5-[2-methoxy-6-oxo-4-trifluoromethyl-1(6H)-pyrimidinyl]-benzoate,
2-chloroethyl 2-chloro-4-fluoro-5-[2-methoxy-6-oxo-4-trifluoromethyl-1(6H)-pyrimidinyl]-benzoate,
2-fluoroethyl 2-chloro-5-[3,6-dihydro-2,6-dioxo-3-methyl-4-trifluoromethyl-1(2H)-pyrimidinyl]-4-fluorobenzoate,
3-chloro-2-butenyl 2-chloro-4-fluoro-5-[2-methoxy-6-oxo-4-trifluoromethyl-1(6H)-pyrimidinyl]-benzoate,
3-chloro-2-butenyl 2-chloro-5-[3,6-dihydro-2,6-dioxo-3-methyl-4-trifluoromethyl-1(2H)-pyrimidinyl]-4-fluorobenzoate,
2-fluoroethyl 2-chloro-4-fluoro-5-[2-methoxy-6-oxo-4-trifluoromethyl-1(6H)-pyrimidinyl]-benzoate,
S-isopropyl 2-chloro-5-[3,6-dihydro-2,6-dioxo-3-methyl-4-trifluoromethyl-1(2H)-pyrimidinyl]-4-fluorothiobenzoate,
S-ethyl 2-chloro-5-[3,6-dihydro-2,6-dioxo-3-methyl-4-trifluoromethyl-1(2H)-pyrimidinyl]-4-fluorothiobenzoate,
S-(n-butyl) 2-chloro-5-[3,6-dihydro-2,6-dioxo-3-methyl-4-trifluoromethyl-1(2H)-pyrimidinyl]-4-fluorothiobenzoate, S-allyl 2-chloro-5-[3,6-dihydro-2,6-dioxo-3-methyl-4-trifluoromethyl-1(2H)-pyrimidinyl]-4-fluorothiobenzoate,
S-cyclohexyl 2-chloro-5-[3,6-dihydro-2,6-dioxo-3-methyl-4-trifluoromethyl-1(2H)-pyrimidinyl]-4-fluorothiobenzoate,
S-phenyl 2-chloro-5-[3,6-dihydro-2,6-dioxo-3-methyl-4-trifluoromethyl-1(2H)-pyrimidinyl]-4-fluorothiobenzoate,
S-benzyl 2-chloro-5-[3,6-dihydro-2,6-dioxo-3-methyl-4-trifluoromethyl-1(2H)-pyrimidinyl]-4-fluorothiobenzoate and
S-isopropyl 2-chloro-5-[3,6-dihydro-3,4-dimethyl-2,6-dioxo-1(2H)-pyrimidinyl]-4-fluorothiobenzoate.

8. A weed control composition, which contains an effective amount of at least one compound of the general formula

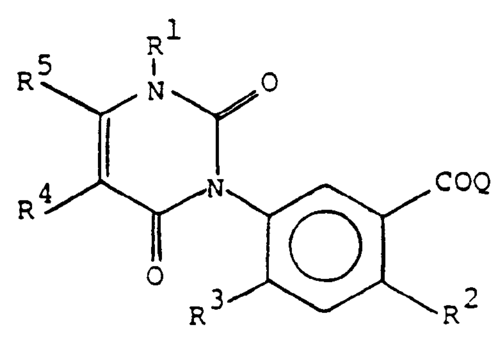

I

wherein
$R^1$ signifies hydrogen, $C_{1-4}$alkyl, $C_{1-4}$haloalkyl, $C_{2-5}$alkenyl or $C_{3-5}$alkynyl, $R^2$ signifies halogen or cyano, $R^3$ signifies hydrogen or halogen, $R^4$ signifies hydrogen, halogen or $C_{1-4}$alkyl, $R^5$ signifies $C_{1-4}$alkyl or, where R′ is different from $C_{1-4}$haloalkyl, also $C_{1-4}$haloalkyl and Q signifies one of the groups (a) to (d) (where $R^1$ is different from hydrogen) or a group (c) or (d) (where $R^1$ stands for hydrogen)

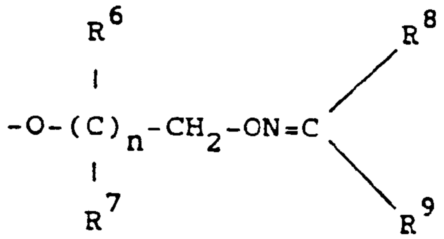

(a)

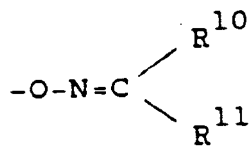

(b)

$-O-R^{12}$ (c)

$-S-R^{13}$ (d)

wherein

$R^6$ signifies hydrogen or $C_{1-4}$alkyl,

$R^7$ signifies hydrogen, $C_{1-4}$alkyl, phenyl or benzyl,

$R^8$ signifies hydrogen or $C_{1-6}$alkyl,

$R^9$ signifies $C_{1-6}$alkyl, $C_{3-6}$cycloalkyl or phenyl,

or

$R^8$ and $R^9$ together signify tri-, tetra-, penta- or hexamethylene,

n signifies 0 or 1,

$R^{10}$ signifies $C_{1-6}$alkyl, $C_{1-6}$alkylthio, $C_{2-7}$alkoxycarbonyl or $C_{2-7}$alkoxycarbonyl-$C_{1-4}$alkyl,

$R^{11}$ signifies $C_{1-6}$alkyl, trifluoromethyl, $C_{1-6}$alkoxy-$C_{1-4}$alkyl, $C_{2-7}$alkoxycarbonyl-$C_{1-4}$alkyl, di($C_{2-7}$alkoxycarbonyl)-$C_{1-4}$alkyl, $C_{3-6}$cycloalkyl, $C_{1-6}$alkoxy, $C_{1-6}$alkylthio, $C_{2-7}$alkanoyl, $C_{2-7}$alkoxycarbonyl, phenyl or 2-furyl,

or

$R^{10}$ and $R^{11}$ together with the carbon atom to which they are attached signify a cyclopentane or cyclohexane ring optionally substituted with 1 to 3 $C_{1-4}$alkyl groups,

$R^{12}$ signifies $C_{1-8}$haloalkyl, $C_{3-5}$haloalkenyl or $C_{3-5}$haloalkynyl,

and

$R^{13}$ signifies hydrogen, $C_{1-8}$alkyl, $C_{3-8}$alkenyl, $C_{3-8}$alkynyl or $C_{2-6}$alkoxyalkyl, or $C_{3-8}$cycloalkyl, phenyl or benzyl optionally substituted with 1 to 3 $C_{1-4}$alkyl groups, or an enol ether of such a compound of formula I in which $R^1$ signifies $C_{1-4}$alkyl, $C_{2-5}$alkenyl or $C_{3-5}$alkynyl and Q signifies a group (b), (c) or (d) or of a salt of such a compound of formula I or of such an enol ether in which $R^1$ and/or $R^{13}$ signifies hydroge n, as well as formulation adjuvants.

**9.** A weed control composition according to claim 8, in which $R^9$ is different from $C_{3-6}$cycloalkyl and $R^{11}$ is different from phenyl.

**10.** A weed control composition, which contains an effective amount of at least one compound selected from the group.

2-[(isopropylideneamino)oxy]-ethyl 2-chloro-5-[3,6-dihydro-2,6-dioxo-3-methyl-4-trifluoromethyl-1(2H)-pyrimidinyl]-4-fluorobenzoate,

2-[(cyclohexylideneamino)oxy]-ethyl 2-chloro-5-[3,6-dihydro-2,6-dioxo-3-methyl-4-trifluoromethyl-1(2H)-pyrimidinyl]-4-fluorobenzoate,

α-{[(isopropylideneamino)oxy]methyl}-benzyl]2-chloro-5-[3,6-dihydro-2,6-dioxo-3-methyl-4-trifluoromethyl-1(2H)-pyrimidinyl]-4-fluorobenzoate,

1-cyclopropyl-1-ethanone O-{2-chloro-5-[3,6-dihydro-2,6-dioxo-3-methyl-4-trifluoromethyl-1(2H)-pyrimidinyl]-4-fluorobenzoyl} oxime,

1-[4-chloro-2-fluoro-5-{[(isopropylideneamino)oxy]-carbonyl}-phenyl]-3-methyl-4-trifluoromethyl-2,6-(1H, 3H)-pyrimidinedione,

2-furyl methyl ketone O-{2-chloro-5-[3,6-dihydro-2,6-dioxo-3-methyl-4-trifluoromethyl-1(2H)pyrimidinyl]-4-fluorobenzoyl} oxime,

2,3-butanedione 2-[O-{2-chloro-5-[3,6-dihydro-2,6-dioxo-3-methyl-4-trifluoromethyl-1(2H)-pyrimidinyl]-4-fluorobenzoyl}] oxime,
2-fluoro-1-fluoromethylethyl 2-chloro-4-fluoro-5-[2-methoxy-6-oxo-4-trifluoromethyl-1(6H)-pyrimidinyl]-benzoate,
2-chloroethyl 2-chloro-4-fluoro-5-[2-methoxy-6-oxo-4-trifluoromethyl-1(6H)-pyrimidinyl]-benzoate,
2-fluoroethyl 2-chloro-5-[3,6-dihydro-2,6-dioxo-3-methyl-4-trifluoromethyl-1(2H)-pyrimidinyl]-4-fluoOrobenzoate,
3-chloro-2-butenyl 2-chloro-4-fluoro-5-[2-methoxy-6-oxo-4-trifluoromethyl-1(6H)-pyrimidinyl]-benzoate,
3-chloro-2-butenyl 2-chloro-5-[3,6-dihydro-2,6-dioxo-3-methyl-4-trifluoromethyl-1(2H)-pyrimidinyl]-4-fluorobenzoate,
2-fluoroethyl 2-chloro-4-fluoro-5-[2-methoxy-6-oxo-4-trifluoromethyl-1(6H)-pyrimidinyl]-benzoate,
S-isopropyl 2-chloro-5-[3,6-dihydro-2,6-dioxo-3-methyl-4-trifluoromethyl-1(2H)-pyrimidinyl]-4-fluorothiobenzoate,
S-ethyl 2-chloro-5-[3,6-dihydro-2,6-dioxo-3-methyl-4-trifluoromethyl-1(2H)-pyrimidinyl]-4-fluorothiobenzoate,
S-(n-butyl) 2-chloro-5-[3,6-dihydro-2,6-dioxo-3-methyl-4-trifluoromethyl-1(2H)-pyrimidinyl]-4-fluorothiobenzoate,
S-allyl 2-chloro-5-[3,6-dihydro-2,6-dioxo-3-methyl-4-trifluoromethyl-1(2H)-pyrimidinyl]-4-fluorothiobenzoate,
S-cyclohexyl 2-chloro-5-[3,6-dihydro-2,6-dioxo-3-methyl-4-trifluoromethyl-1(2H)-pyrimidinyl]-4-fluorothiobenzoate,
S-phenyl 2-chloro-5-[3,6-dihydro-2,6-dioxo-3-methyl-4-trifluoromethyl-1(2H)-pyrimidinyl]-4-fluorothiobenzoate,
S-benzyl 2-chloro-5-[3,6-dihydro-2,6-dioxo-3-methyl-4-trifluoromethyl-1(2H)-pyrimidinyl]-4-fluorothiobenzoate and
S-isopropyl 2-chloro-5-[3,6-dihydro-3,4-dimethyl-2,6-dioxo-1(2H)-pyrimidinyl]-4-fluorothiobenzoate as well as formulation adjuvants.

**11.** A process for the preparation of a compound of the general formula

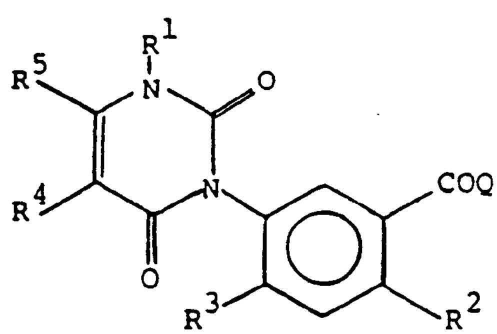

I

wherein
$R^1$ signifies hydrogen, $C_{1-4}$alkyl, $C_{1-4}$haloalkyl, $C_{2-5}$alkenyl or $C_{3-5}$alkynyl, $R^2$ signifies halogen or cyano, $R^3$ signifies hydrogen or halogen, $R^4$ signifies hydrogen, halogen or $C_{1-4}$alkyl, $R^5$ signifies $C_{1-4}$alkyl or, where R′ is different from $C_{1-4}$haloalkyl, also $C_{1-4}$haloalkyl and Q signifies one of the groups (a) to (d) (where $R^1$ is different from hydrogen) or a group (c) or (d) (where $R^1$ stands for hydrogen)

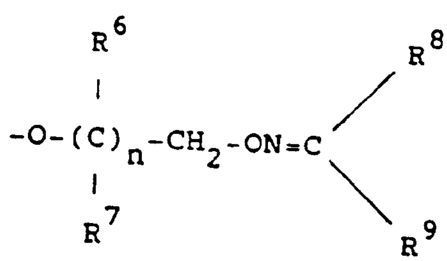

(a)

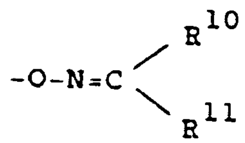 (b)

-O-$R^{12}$ (c)

-S-$R^{13}$ (d)

wherein

$R^6$ signifies hydrogen or $C_{1-4}$alkyl,

$R^7$ signifies hydrogen, $C_{1-4}$alkyl, phenyl or benzyl,

$R^8$ signifies hydrogen or $C_{1-6}$alkyl,

$R^9$ signifies $C_{1-6}$alkyl, $C_{3-6}$cycloalkyl or phenyl,

or

$R^8$ and $R^9$ together signify tri-, tetra-, penta- or hexamethylene,

n signifies 0 or 1,

$R^{10}$ signifies $C_{1-6}$alkyl, $C_{1-6}$alkylthio, $C_{2-7}$alkoxycarbonyl or $C_{2-7}$alkoxycarbonyl-$C_{1-4}$alkyl,

$R^{11}$ signifies $C_{1-6}$alkyl, trifluoromethyl, $C_{1-6}$alkoxy- $C_{1-4}$alkyl, $C_{2-7}$alkoxycarbonyl-$C_{1-4}$alkyl, di($C_{2-7}$alkoxycarbonyl)-$C_{1-4}$alkyl, $C_{3-6}$cycloalkyl, $C_{1-6}$alkoxy, $C_{1-6}$alkylthio, $C_{2-7}$alkanoyl, $C_{2-7}$alkoxycarbonyl, phenyl or 2-furyl,

or

$R^{10}$ and $R^{11}$ together with the carbon atom to which they are attached signify a cyclopentane or cyclohexane ring optionally substituted with 1 to 3 $C_{1-4}$alkyl groups,

$R^{12}$ signifies $C_{1-8}$haloalkyl, $C_{3-5}$haloalkenyl or $C_{3-5}$haloalkynyl,

and

$R^{13}$ signifies hydrogen, $C_{1-8}$alkyl, $C_{3-8}$alkenyl, $C_{3-8}$alkynyl or $C_{2-6}$alkoxyalkyl, or $C_{3-8}$cycloalkyl, phenyl or benzyl optionally substituted with 1 to 3 $C_{1-4}$alkyl groups,

or of a corresponding enol ether of those compounds of formula I in which $R^1$ signifies $C_{1-4}$alkyl, $C_{2-5}$alkenyl or $C_{3-5}$alkynyl and Q signifies a group (b), (c) or (d) or else of a salt of those compounds of formula I or enol ethers in which $R^1$ and/or $R^{13}$ signifies hydrogen, which process comprises

a) for the preparation of those compounds of formula I in which $R^1$ signifies hydrogen and Q signifies a group (c) or (d) as well as, if desired, of metal salts of these compounds, subjecting a compound of the general formula

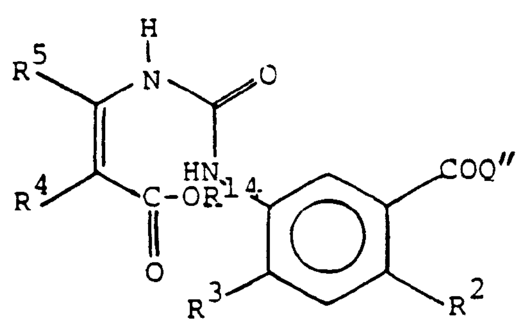 II

wherein $R^2$, $R^3$, $R^4$ and $R^5$ have the significations given above, Q″ signifies a group (c) or (d), as defined above, and $R^{14}$ signifies lower alkyl, preferably $C_{1-4}$alkyl,

to a cyclization under basic conditions and, if desired, converting a metal salt of the uracil derivative of formula I which may be obtained into the acidic form ($R^1$ = hydrogen) by treatment with an acid.

b) for the preparation of those compounds of formula I in which $R^1$ signifies $C_{1-4}$alkyl, $C_{1-4}$haloalkyl, $C_{2-5}$alkenyl or $C_{3-5}$alkynyl and Q signifies one of the groups (a) to (d) and of the enol ethers of these compounds, esterifying a benzoic acid of the general formula

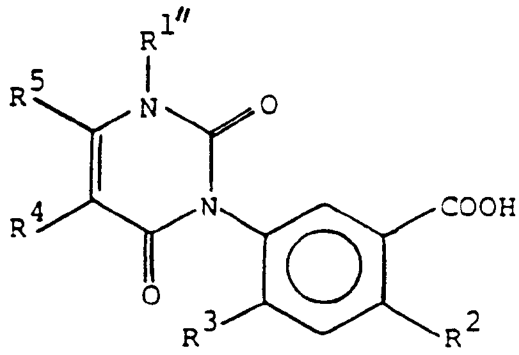

III

wherein $R^2$, $R^3$, $R^4$ and $R^5$ have the significations given above and $R^{1''}$ signifies $C_{1-4}$alkyl, $C_{1-4}$haloalkyl, $C_{2-5}$alkenyl or $C_{3-5}$alkynyl, or a reactive derivative of this benzoic acid with a hydroxy or mercapto compound of the general formula

H-Q IV

wherein Q has the signification give above, or with a reactive derivative of this hydroxy or mercapto compound or esterifying an enol ether of this benzoic acid, namely of the general formula

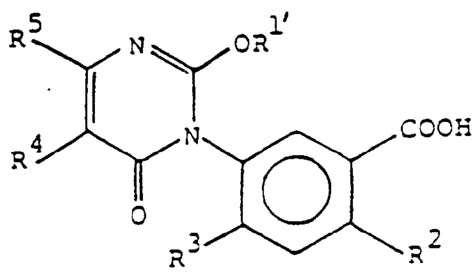

IIIa

or

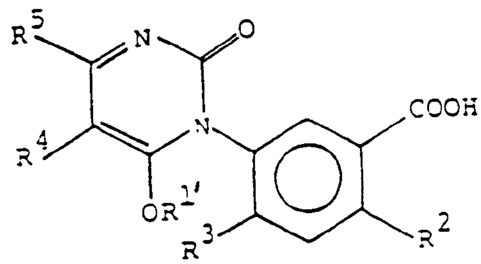

IIIb

wherein $R^{1'}$, $R^2$, $R^3$, $R^4$ and $R^5$ have the significations give above, or a reactive derivative of this enol ether with a hydroxy or mercapto compound of the general formula

H-Q′ IV′

wherein Q′ has the signification give above,
or with a reactive derivative of this hydroxy or mercapto compound,
c) for the preparation of those compounds of formula I in which $R^1$ signifies $C_{1-4}$alkyl, $C_{1-4}$haloalkyl, $C_{2-5}$alkenyl or $C_{3-5}$alkynyl and Q signifies a group (c), subjecting a benzoic acid ester of the general formula

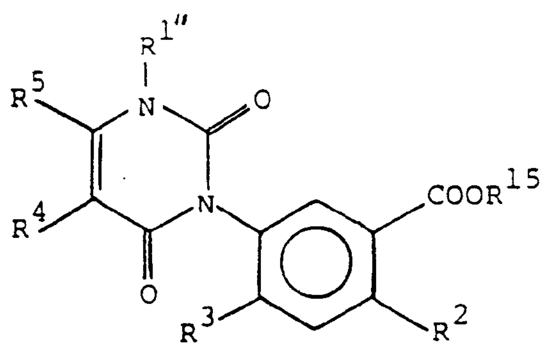

V

wherein $R^{1''}$, $R^2$, $R^3$, $R^4$ and $R^5$ have the significations given above and $R^{15}$ signifies $C_{1-6}$alkyl, $C_{2-4}$alkenyl, $C_{2-4}$alkynyl or $C_{2-6}$alkoxyalkyl,
to a transesterification reaction with a hydroxy compound of the general formula

H-Q‴ IV″

wherein Q‴ signifies a group (c), the reagent IV″ having a higher boiling point than the hydroxy compound $R^{15}OH$ formed, or

d) for the preparation of those enol ethers of formula Ia and Ib in which Q′ signifies a group (c) or (d), treating a pyrimidinone derivative of the general formula

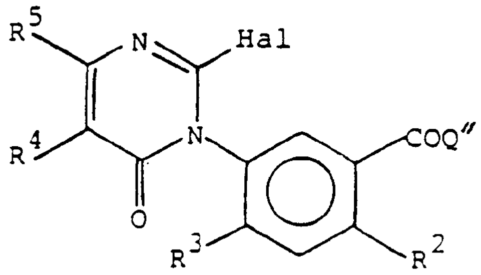

VIa

or

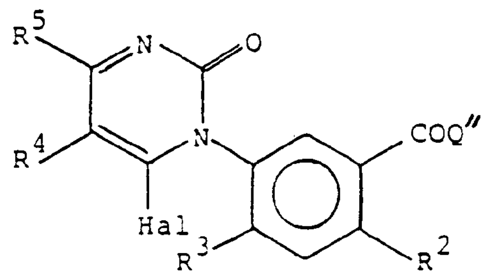

VIb

wherein $R^2$, $R^3$, $R^4$, $R^5$ and Q″ have the significations given above and Hal signifies chlorine or bromine, with the deprotonized form of an alkanol, alkenol or alkynol $R^{1'}OH$ in which $R^{1'}$ has the signification given above,

and, if desired, converting a compound thus obtained of formula I in which $R^1$ and/or $R^{13}$ signifies hydrogen into a salt.

**12.** A process according to claim 11, wherein $R^9$ is different from $C_{3-6}$cycloalkyl and $R^{13}$ is different from phenyl.

**13.** A method for the control of weeds, which method comprises treating the locus to be protected against weeds and/or the weeds with an effective amount of a compound in accordance with any one of claims 1 to 7 or of a composition in accordance with claim 8, 9 or 10.

**14.** The use of a compound in accordance with any one of claims 1 to 7 or of a composition in accordance with claim 8, 9 or 10 for the control of weeds.

## Revendications

**1.** Composés de formule générale I

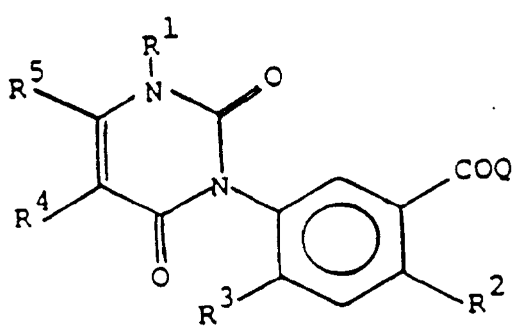

I

dans laquelle

$R^1$ représente un hydrogène, un alcoyle en $C_{1-4}$, un halogènealcoyle en $C_{1-4}$, un alcényle en $C_{2-5}$ ou un alcynyle en $C_{3-5}$,

$R^2$ représente un halogène ou un cyano,

$R^3$ représente un hydrogène ou un halogène,

$R^4$ représente un hydrogène, un halogène ou un alcoyle en $C_{1-4}$,

$R^5$ représente un alcoyle en $C_{1-4}$ ou encore, dans le cas où $R^1$ est différent d'un halogènalcoyle en $C_{1-4}$, un halogènealcoyle en $C_{1-4}$,

Q représente l'un des groupes (a) à (d) (si $R^1$ est différent d'un hydrogène) ou un groupe (c) ou (d) (où $R^1$ représente un hydrogène)

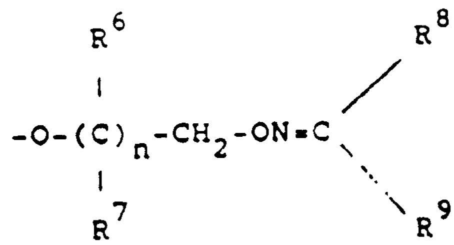

(a)

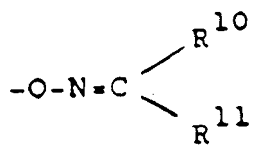

(b)

-O-$R^{12}$ (c)

-S-$R^{13}$ (d)

où

$R^6$ représente un hydrogène ou un alcoyle en $C_{1-4}$,

$R^7$ représente un hydrogène, un alcoyle en $C_{1-4}$, un phényle ou un benzyle,

$R^8$ représente un hydrogène ou un alcoyle en $c_{1-6}$,

$R^9$ représente un alcoyle en $C_{1-6}$, un cycloalcoyle en $C_{3-6}$ ou un phényle,

ou

$R^8$ et $R^9$ représentent ensemble un tri-, tétra, penta-, ou hexaméthylène,

n vaut 0 ou 1,

$R^{10}$ représente un alcoyle en $C_{1-6}$, alcoylthio en $C_{1-6}$, alcoxycarbonyle en $C_{2-7}$ ou alcoxy en $C_{2-7}$-carbonyle-alcoyle en $C_{1-4}$,

$R^{11}$ représente un alcoyle en $C_{1-6}$, un trifluoro-méthyle, alcoxy en $C_{1-6}$-alcoyle en $C_{1-4}$, un alcoxy en $C_{2-7}$-carbonyle-alcoyle en $C_{1-4}$, un di(alcoxy en $C_{2-7}$-carbonyle)-alcoyle en $C_{1-4}$, un cycloalcoyle en $C_{3-6}$, un alcoxy en $C_{1-6}$, un alcoylthio en $C_{1-6}$, un alcanoyle en $C_{2-7}$, un alcoxycarbonyle en $C_{2-7}$, un phényle ou un 2-furyle,

ou

$R^{10}$ et $R^{11}$ représentent ensemble avec l'atome de carbone auquel ils sont liés un noyau cyclo-pentane ou cyclohexane éventuellement substitué par de 1 à 3 groupes alcoyles en $C_{1-4}$,

$R^{12}$ représente un halogènalcoyle en $C_{1-8}$, un halogènalcényle en $C_{3-5}$ ou un halogènalcynyle en $C_{3-5}$,

et

$R^{13}$ représente un hydrogène, un alcoyle en $C_{1-8}$, un alcényle en $C_{3-8}$, un alcynyle en $C_{3-8}$, un alcoxyalcoyle en $C_{2-6}$ ou un cyclalcoyle en $C_{3-8}$ éventuellement substitué par de 1 à 3 groupes alcoyles en $C_{1-4}$, un phényle ou un benzyle,

et les énoléthers des composés de formule I dans lesquels $R^1$ représente un alcoyle en $C_{1-4}$, un alcényle en $C_{2-5}$ ou un alcynyle en $C_{3-5}$,

et Q un groupe (b), (c) ou (d), ainsi que les sels des composés de formule I ou les énoléthers dans lesquels $R^1$ et/ou $R^{13}$ représentent un hydrogène.

2. Composés selon la revendication 1, où $R^9$ est différent d'un cycloalcoyle en $C_{3-6}$ et $R^{11}$ est différent d'un phényle.

3. Composés selon la revendication 1 ou 2, où $R^1$ est un alcoyle en $C_{1-4}$ à chaîne droite, en particulier un méthyle ou un difluorométhyle.

4. Composés selon l'une des revendications 1 à 3, où $R^2$ est un chlore ou un brome et $R^3$ un hydrogène ou un fluor.

5. Composés selon l'une quelconque des revendications 1 à 4, où $R^4$ est un hydrogène, un fluor ou un méthyle.

6. Composés selon l'une des revendications 1 à 5, où $R^5$ est un méthyle, un trifluorométhyle ou un pentafluoroéthyle.

7. Composé selon la revendication, choisi parmi les composés suivants :
{2-[(isopropylidènamino)oxy]-éthyl}ester de l'acide 2-chloro-5-[3,6-dihydro-2,6-dioxo-3-méthyl-4-trifluoro-méthyl-1(2H)-pyrimidinyl]-4-fluorobenzoïque,
{2-[(cyclohexylidènamino)oxy]-éthyl}ester de l'acide 2-chloro-5-[3,6-dihydro-2,6-dioxo-3-méthyl-4-trifluorométhyl-1(2H)-pyrimidinyl]-4-fluorobenzoïque,
[α-{[(isopropylidènamino)oxy]méthyl}-benzyl]-ester de l'acide 2-chloro-5-[3,6-dihydro-2,6-dioxo-3-méthyl-4-trifluorométhyl-1(2H)-pyrimidinyl]-4-fluorobenzoïque,
1-cyclopropyl-1-éthanon-O-{2-chloro-5-[3,6-dihydro-2,6-dioxo-3-méthyl-4-trifluorométhyl-1(2H)-pyrimidinyl]-4-fluorobenzoyl}oxime,
1-[4-chloro-2-fluoro-5-{[(isopropylidènamino)oxy]carbonyl}-phényl]-3-méthyl-4-trifluorométhyl-2,6(1H,3H)-pyrimidinedione,
2-furylméthylcéton-O-{2-chloro-5-[3,6-dihydro-2,6-dioxo-3-méthyl-4-trifluorométhyl-1(2H)-pyrimidinyl]-4-fluorobenzoyl}oxime,
2,3-butanedione-2-[O-{2-chloro-5-[3,6-dihydro-2,6-dioxo-3-méthyl-4-trifluorométhyl-1(2H)-pyrimidinyl]-4-fluoro-benzoyl}]oxime,
(2-fluoro-1-fluorométhyl-éthyl)ester de l'acide 2-chloro-4-fluoro-5-[2-méthoxy-6-oxo-4-trifluorométhyl-1(6H)-pyrimidinyl]benzoïque,
(2-chloroéthyl)ester de l'acide 2-chloro-4-fluoro-5-[2-méthoxy-6-oxo4-trifluorométhyl-1(6H)-pyrimidinyl]-benzoïque,
(2-fluoroéthyl)-ester de l'acide 2-chloro-5-[3,6-dihydro-2,6-dioxo-3-méthyl-4-trifluorométhyl-1(2H)-pyrimidinyl]-4-fluorobenzoïque,
(3-chloro-2-butényl)ester de l'acide 2-chloro-4-fluoro-5-[2-méthoxy-6-oxo-4-trifluorométhyl-1(6H)-pyrimidinyl]benzoïque,
(3-chloro-2-butényl)ester de l'acide 3-chloro-5-[3,6-dihydro-2,6-dioxo-3-méthyl-4-trifluorométhyl-1(2H)-pyrimidinyl]-4-fluorobenzoïque,
(2-fluoroéthyl)ester de l'acide 2-chloro-4-fluoro-5-[2-méthoxy-6-oxo-4-trifluorométhyl-1(6H)-pyrimidinyl]benzoïque,
S-isopropylester de l'acide 2-chloro-5-[3,6-dihydro-2,6-dioxo-3-méthyl-4-trifluorométhyl-1(2H)-pyrimidinyl]-4-fluoro-trhiobenzoïque,
S-éthylester de l'acide 2-chloro-5-[3,6-dihydro-2,6-dioxo-3-méthyl-4-trifluorométhyl-1(2H)-pyrimidinyl]-4-fluorothiobenzoïque,
S-(n-butyl)-ester de l'acide 2-chloro-5-[3,6-dihydro-2,6-dioxo-3-méthyl-4-trifluorométhyl-1(2H)-pyrimidinyl]-4-fluorothiobenzoïque,
S-allylester de l'acide 2-chloro-5è[3,6-dihydro-2,6-dioxo-3-méthyl-4-trifluorométhyl-1(2H)-pyrimidinyl]-4-fluorothiobenzoïque,
S-cyclohexylester de l'acide 2-chloro-5-[3,6-dihydro-2,6-dioxo-3-méthyl-4-trifluorométhyl-1(2H)-pyrimidinyl]-4-fluorothiobenzoïque,
S-phénylester de l'acide 2-chloro-4-[3,6-dihydro-2,6-dioxo-3-méthyl-4-trifluorométhyl-1(2H)-pyrimidinyl]-4-fluorothiobenzoïque,
S-benzylester de l'acide 2-chloro-5-[3,6-dihydro-2,6-dioxo-3-méthyl-4-trifluorométhyl-1(2H)-pyrimidinyl]-4-fluorothiobenzoïque,
S-isopropylester de l'acide 2-chloro-5-[3,6-dihydro-3,4-diméthyl-2,6-dioxo-1(2H)-pyrimidinyl]-4-fluorothiobenzoïque.

8. Agent de lutte contre les mauvaises herbes, caractérisé en ce qu'il contient une quantité efficace d'au moins un composé de formule générale

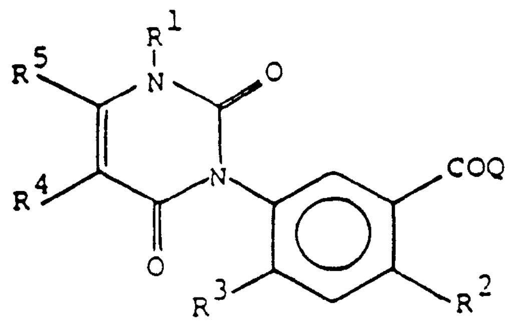

I

dans laquelle

$R^1$ représente un hydrogène, un alcoyle en $C_{1-4}$, un halogènealcoyle en $C_{1-4}$, un alcényle en $C_{2-5}$ ou un alcynyle en $C_{3-5}$,

$R^2$ représente un halogène ou un cyano,

$R^3$ représente un hydrogène ou un halogène,

$R^4$ représente un hydrogène, un halogène ou un alcoyle en $C_{1-4}$,

$R^5$ représente un alcoyle en $C_{1-4}$ ou encore, dans le cas où $R^1$ est différent d'un halogènalcoyle en $C_{1-4}$, un halogènealcoyle en $C_{1-4}$,

Q représente l'un des groupes (a) à (d) (si $R^1$ est différent d'un hydrogène) ou un groupe (c) ou (d) (où $R^1$ représente un hydrogène)

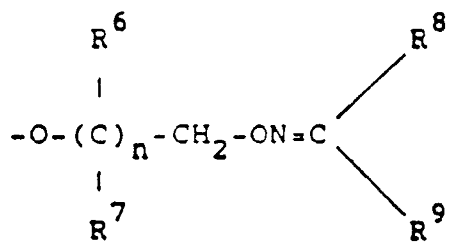

(a)

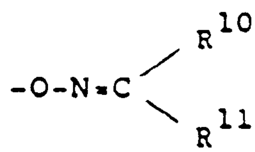

(b)

$-O-R^{12}$ (c)

où

$R^6$ représente un hydrogène ou un alcoyle en $C_{1-4}$,

$R^7$ représente un hydrogène, un alcoyle en $C_{1-4}$, un phényle ou un benzyle,

$R^8$ représente un hydrogène ou un alcoyle en $C_{1-6}$,

$R^9$ représente un alcoyle en $C_{1-6}$, un cycloalcoyle en $C_{3-6}$ ou un phényle,

ou

$R^8$ et $R^9$ représentent ensemble un tri-, tétra, penta-, ou hexaméthylène,

n vaut 0 ou 1,

$R^{10}$ représente un alcoyle en $C_{1-6}$, alcoylthio en $C_{1-6}$, alcoxycarbonyle en $C_{2-7}$ ou alcoxy en $C_{2-7}$-carbonyle-alcoyle en $C_{1-4}$,

$R^{11}$ représente un alcoyle en $C_{1-6}$, un trifluorométhyle, alcoxy en $C_{1-6}$-alcoyle en $C_{1-4}$, un alcoxy en $C_{2-7}$-carbonyle-alcoyle en $C_{1-4}$, un di(alcoxy en $C_{2-7}$-carbonyle)-alcoyle en $C_{1-4}$, un cycloalcoyle en $C_{3-6}$, un alcoxy en $C_{1-6}$, un alcoylthio en $C_{1-6}$, un alcanoyle en $C_{2-7}$, un alcoxycarbonyle en $C_{2-7}$, un phényle ou un 2-furyle,

ou

$R^{10}$ et $R^{11}$ représentent ensemble avec l'atome de carbone auquel ils sont liés un noyau cyclopentane ou cyclohexane éventuellement substitué par de 1 à 3 groupes alcoyles en $C_{1-4}$,

$R^{12}$ représente un halogènalcoyle en $C_{1-8}$, un halogènalcényle en $C_{3-5}$ ou un halogènalcynyle en $C_{3-5}$,

et

$R^{13}$ représente un hydrogène, un alcoyle en $C_{1-8}$, un alcényle en $C_{3-8}$, un alcynyle en $C_{3-8}$, un alcoxyalcoyle en $C_{2-6}$ ou un cyclalcoyle en $C_{3-8}$ éventuellement substitué par de 1 à 3 groupes alcoyles en $C_{1-4}$, un phényle ou un benzyle,

et les énoléthers des composés de formule I dans lesquels $R^1$ représente un alcoyle en $C_{1-4}$, un alcényle en $C_{2-5}$ ou un alcynyle en $C_{3-5}$,

et Q un groupe (b), (c) ou (d), ainsi que les sels des composés de formule I ou les énoléthers dans lesquels $R^1$ et/ou $R^{13}$ représentent un hydrogène,

ainsi que des additifs de formulation.

9. Agent de lutte contre les mauvaises herbes selon la revendication 8, où $R^9$ est différent d'un cycloalcoyle en $C_{3-6}$ et $R^{11}$ est différent d'un phényle.

10. Agent de lutte contre les mauvaises herbes, caractérisé en ce qu'il contient une quantité efficace d'au moins un des composés choisis dans le groupe :

{2-[(isopropylidènamino)oxy]-éthyl}ester de l'acide 2-chloro-5-[3,6-dihydro-2,6-dioxo-3-méthyl-4-trifluoro-méthyl-1(2H)-pyrimidinyl]-4-fluorobenzoïque,

{2-[(cyclohexylidènamino)oxy]-éthyl}ester de l'acide 2-chloro-5-[3,6-dihydro-2,6-dioxo-3-méthyl-4-trifluorométhyl-1(2H)-pyrimidinyl]-4-fluorobenzoïque,

[α-{[(isopropylidènamino)oxy]méthyl}-benzyl]-ester de l'acide 2-chloro-5-[3,6-dihydro-2,6-dioxo-3-méthyl-4-trifluorométhyl-1(2H)-pyrimidinyl]-4-fluorobenzoïque,

1-cyclopropyl-1-éthanon-O-{2-chloro-5-[3,6-dihydro-2,6-dioxo-3-méthyl-4-trifluorométhyl-1(2H)-pyrimidinyl]-4-fluorobenzoyl}oxime,

1-[4-chloro-2-fluoro-5-{[(isopropylènamino)oxy]carbonyl}-phényl]-3-méthyl-4-trifluorométhyl-2,6(1H,3H)-pyrimidinedione,

2-furylméthylcéton-O-{2-chloro-5-[3,6-dihydro-2,6-dioxo-3-méthyl-4-trifluorométhyl-1(2H)-pyrimidinyl]-4-fluorobenzoyl}oxime,

2,3-butanedione-2-[O-{2-chloro-5-[3,6-dihydro-2,6-dioxo-3-méthyl-4-trifluorométhyl-1(2H)-pyrimidinyl]-4-fluoro-benzoyl}]oxime,

(2-fluoro-1-fluorométhyl-éthyl)ester de l'acide 2-chloro-4-fluoro-5-[2-méthoxy-6-oxo-4-trifluorométhyl-1(6H)-pyrimidinyl]benzoïque,

(2-chloroéthyl)ester de l'acide 2-chloro-4-fluoro-5-[2-méthoxy-6-oxo4-trifluorométhyl-1(6H)-pyrimidinyl]-benzoïque,

(2-fluoroéthyl)-ester de l'acide 2-chloro-5-[3,6-dihydro-2,6-dioxo-3-méthyl-4-trifluorométhyl-1(2H)-pyrimidinyl]-4-fluorobenzoïque,

(3-chloro-2-buténył)ester de l'acide 2-chloro-4-fluoro-5-[2-méthoxy-6-oxo-4-trifluorométhyl-1(6H)-pyrimidinyl]benzoïque,

(3-chloro-2-buténył)ester de l'acide 3-chloro-5-[3,6-dihydro-2,6-dioxo-3-méthyl-4-trifluorométhyl-1(2H)-pyrimidinyl]-4-fluorobenzoïque,

(2-fluoroéthyl)ester de l'acide 2-chloro-4-fluoro-5-[2-méthoxy-6-oxo-4-trifluorométhyl-1(6H)-pyrimidinyl]benzoïque,

S-isopropylester de l'acide 2-chloro-5-[3,6-dihydro-2,6-dioxo-3-méthyl-4-trifluorométhyl-1(2H)-pyrimidinyl]-4-fluoro-trhiobenzoïque,

S-éthylester de l'acide 2-chloro-5-[3,6-dihydro-2,6-dioxo-3-méthyl-4-trifluorométhyl-1(2H)-pyrimidinyl]-4-fluorothiobenzoïque,

S-(n-butyl)-ester de l'acide 2-chloro-5-[3,6-dihydro-2,6-dioxo-3-méthyl-4-trifluorométhyl-1(2H)-pyrimidinyl]-4-fluorothiobenzoïque,

S-allylester de l'acide 2-chloro-5è[3,6-dihydro-2,6-dioxo-3-méthyl-4-trifluorométhyl-1(2H)-pyrimidinyl]-4-fluorothiobenzoïque,

S-cyclohexylester de l'acide 2-chloro-5-[3,6-dihydro-2,6-dioxo-3-méthyl-4-trifluorométhyl-1(2H)-pyrimidinyl]-4-fluorothiobenzoïque,

S-phénylester de l'acide 2-chloro-4-[3,6-dihydro-2,6-dioxo-3-méthyl-4-trifluorométhyl-1(2H)-pyrimidinyl]-4-fluorothiobenzoïque,

S-benzylester de l'acide 2-chloro-5-[3,6-dihydro-2,6-dioxo-3-méthyl-4-trifluorométhyl-1(2H)-pyrimidinyl]-4-fluorothiobenzoïque,

S-isopropylester de l'acide 2-chloro-5-[3,6-dihydro-3,4-diméthyl-2,6-dioxo-1(2H)-pyrimidinyl]-4-fluorothiobenzoïque,

ainsi que des additifs de formulation.

**11.** Procédé de préparation de composés de formule générale

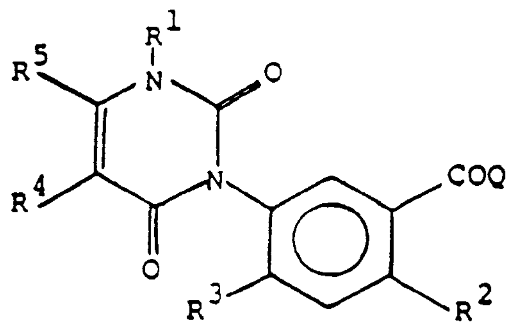

I

$R^1$ représente un hydrogène, un alcoyle en $C_{1-4}$, un halogènealcoyle en $C_{1-4}$, un alcényle en $C_{2-5}$ ou un alcynyle en $C_{3-5}$,

$R^2$ représente un halogène ou un cyano,

$R^3$ représente un hydrogène ou un halogène,

$R^4$ représente un hydrogène, un halogène ou un alcoyle en $C_{1-4}$,

$R^5$ représente un alcoyle en $C_{1-4}$ ou encore, dans le cas où $R^1$ est différent d'un halogènalcoyle en $C_{1-4}$, un halogènealcoyle en $C_{1-4}$,

Q représente l'un des groupes (a) à (d) (si $R^1$ est différent d'un hydrogène) ou un groupe (c) ou (d) (où $R^1$ représente un hydrogène)

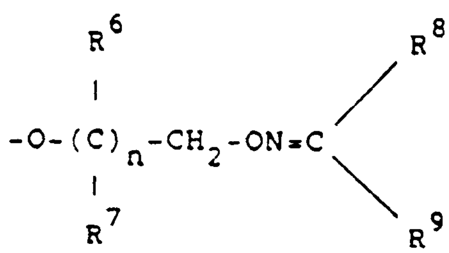

(a)

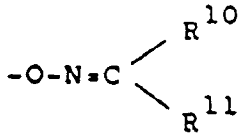

(b)

$-O-R^{12}$ (c)

$-S-R^{13}$ (d)

où

$R^6$ représente un hydrogène ou un alcoyle en $C_{1-4}$,

$R^7$ représente un hydrogène, un alcoyle en $C_{1-4}$, un phényle ou un benzyle,

$R^8$ représente un hydrogène ou un alcoyle en $C_{1-6}$,

$R^9$ représente un alcoyle en $C_{1-6}$, un cycloalcoyle en $C_{3-6}$ ou un phényle,

ou

$R^8$ et $R^9$ représentent ensemble un tri-, tétra, penta-, ou hexaméthylène,

n vaut 0 ou 1,

$R^{10}$ représente un alcoyle en $C_{1-6}$, alcoylthio en $C_{1-6}$, alcoxycarbonyle en $C_{2-7}$ ou alcoxy en $C_{2-7}$-

carbonyle-alcoyle en $C_{1-4}$,

$R^{11}$ représente un alcoyle en $C_{1-6}$, un trifluorométhyle, alcoxy en $C_{1-6}$-alcoyle en $C_{1-4}$, un alcoxy en $C_{2-7}$-carbonyle-alcoyle en $C_{1-4}$, un di(alcoxy en $C_{2-7}$-carbonyle)-alcoyle en $C_{1-4}$, un cycloalcoyle en $C_{3-6}$, un alcoxy en $C_{1-6}$, un alcoylthio en $C_{1-6}$, un alcanoyle en $C_{2-7}$, un alcoxycarbonyle en $C_{2-7}$, un phényle ou un 2-furyle,

ou

$R^{10}$ et $R^{11}$ représentent ensemble avec l'atome de carbone auquel ils sont liés un noyau cyclopentane ou cyclohexane éventuellement substitué par de 1 à 3 groupes alcoyles en $C_{1-4}$,

$R^{12}$ représente un halogènalcoyle en $C_{1-8}$, un halogènalcényle en $C_{3-5}$ ou un halogènalcynyle en $C_{3-5}$,

et

$R^{13}$ représente un hydrogène, un alcoyle en $C_{1-8}$, un alcényle en $C_{3-8}$, un alcynyle en $C_{3-8}$, un alcoxyalcoyle en $C_{2-6}$ ou un cyclalcoyle en $C_{3-8}$ éventuellement substitué par de 1 à 3 groupes alcoyles en $C_{1-4}$, un phényle ou un benzyle,

et les énoléthers des composés de formule I dans lesquels $R^1$ représente un alcoyle en $C_{1-4}$, un alcényle en $C_{2-5}$ ou un alcynyle en $C_{3-5}$,

et Q un groupe (b), (c) ou (d), ainsi que les sels des composés de formule I ou les énoléthers dans lesquels $R^1$ et/ou $R^{13}$ représentent un hydrogène, caractérisé en ce que

a) pour préparer les composés de formule I dans lesquels $R^1$ représente un hydrogène et Q un groupe (c) ou (d), ainsi que si on le désire les sels métalliques de ces composés, on soumet un composé de formule générale

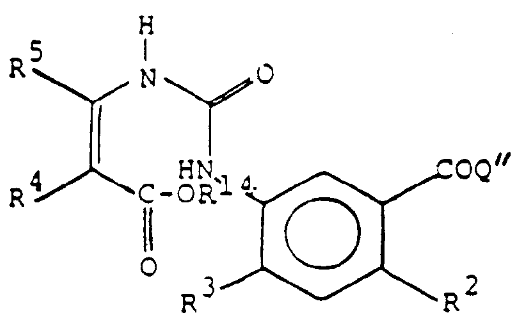

II

dans laquelle $R^2$, $R^3$, $R^4$ et $R^5$ ont les significations données ci-dessus, Q″ représente un groupe (c) ou (d), tel que défini ci-dessus, et $R^{14}$ représente un alcoyle inférieur, de préférence un alcoyle en $C_{1-4}$, à une cyclisation dans des conditions basiques, et si on le désire on transforme un sel métallique du dérivé d'uracile de formule I éventuellement obtenu par traitement avec un acide en la forme acide ($R^1$=hydrogène),

b) pour préparer des composés de formule I dans lesquels $R^1$ représente un alcoyle en $C_{1-4}$, halogènalcoyle en $C_{1-4}$, alcényle en $C_{2-5}$ ou alcynyle en $C_{3-5}$ et Q représente l'un des groupes (a) à (d), et l'énoléther de ces composés, on estérifie un acide benzoïque de formule générale

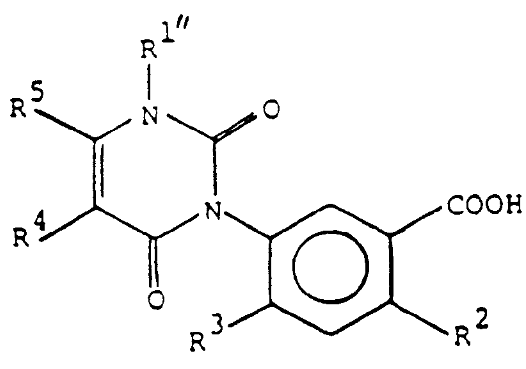

III

dans laquelle $R^2$, $R^3$, $R^4$ et $R^5$ ont les significations données ci-dessus et $R^{1″}$ représente un alcoyle en

$C_{1-4}$, un halogènalcoyle en $C_{1-4}$, un alcényle en $C_{2-5}$ ou un alcynyle en $C_{3-5}$,

ou un dérivé réactif de cet acide benzoïque, avec un composé hydroxy ou mercapto de formule générale

H-Q IV

où Q a la signification donnée ci-dessus, ou avec un dérivé réactif de ce composé hydroxy ou mercapto ou selon les cas un énoléther de cet acide benzoïque, à savoir de formule générale

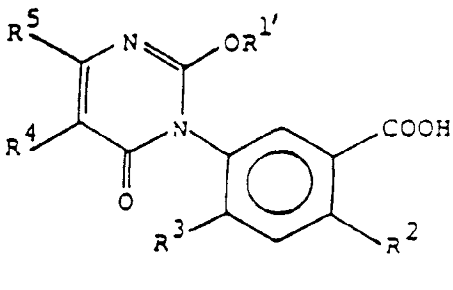

III a

ou

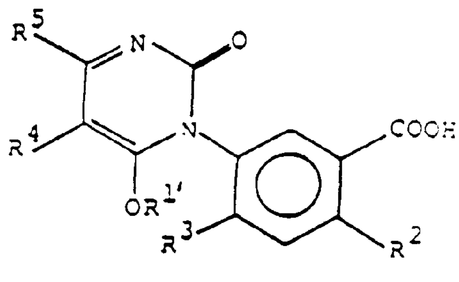

III b

où $R^{1'}$, $R^2$, $R^3$, $R^4$ et $R^5$ ont les significations données ci-dessus,

ou un dérivé réactif de cet énoléther avec un composé hydroxy ou mercapto de formule générale

H-Q′ IV′

où Q a la signification donnée ci-dessus,

ou avec un dérivé réactif de ce composé hydroxy ou mercapto,

c) pour préparer les composés de formule I dans laquelle $R^1$ représente un alcoyle en $C_{1-4}$, un halogènalcoyle en $C_{1-4}$, un alcényle en $C_{2-5}$ ou un alcynyle en $C_{3-5}$ et Q un groupe (c), on soumet un ester d'acide benzoïque de formule générale :

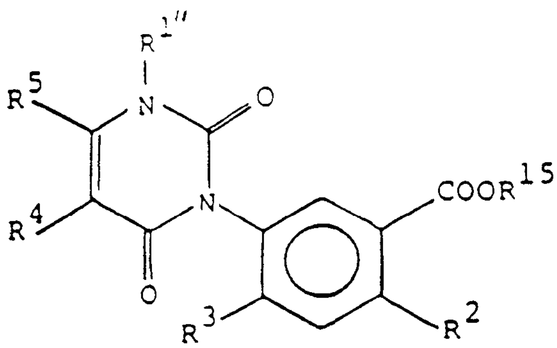

V

dans laquelle $R^{1''}$, $R^2$, $R^3$, $R^4$ et $R^5$ ont les significations données ci-dessus et $R^{15}$ est un alcoyle en $C_{1-6}$, alcényle en $C_{2-4}$, alcynyle en $C_{2-4}$ ou un alcoxyalcoyle en $C_{2-6}$,

à une réaction de transestérification avec un composé hydroxy de formule générale

H-Q‴ IV″

où Q‴ représente un groupe (c),

où le réactif IV″ a un plus haut point d'ébullition que le composé hydroxy $R^{15}OH$ qui se forme, ou

d) pour préparer les énoléthers de formules Ia et Ib dans lesquels Q′ représente un groupe (c) ou (d), on traite un dérivé de pyrimidinone de formule générale

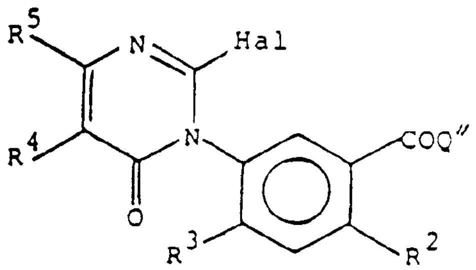

VIa

ou

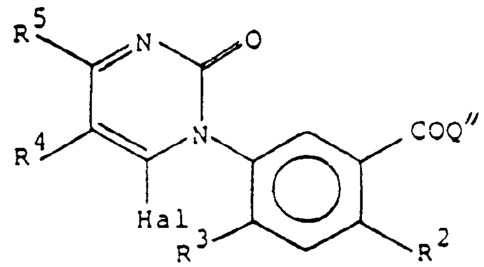

VIb

dans laquelle $R^2$, $R^3$, $R^4$, $R^5$ et Q″ ont les significations données ci-dessus et Hal représente un chlore ou un brome,

avec la forme déprotonée d'un alcanol, alcénol ou alcynol, $R^{1'}OH$, où $R^{1'}$ a la signification donnée ci-dessus,

et si on le désire en ce qu'on transforme un composé ainsi obtenu de formule I dans laquelle $R^1$ et/ou $R^{13}$ représentent un hydrogène, en un sel.

**12.** Procédé selon la revendication 11, dans lequel $R^9$ est différent d'un cycloalcoyle en $C_{3-6}$ et $R^{11}$ est différent d'un phényle.

**13.** Procédé de lutte contre les mauvaises herbes, caractérisé en ce qu'on traite la matière à protéger contre les mauvaises herbes et/ou les mauvaises herbes avec une quantité efficace d'un composé selon l'une des revendications 1 à 7, ou d'un agent selon la revendication 8, 9 ou 10.

**14.** Application d'un composé selon l'une des revendications 1 à 7 ou d'un agent selon la revendication 8, 9 ou 10, à la lutte contre les mauvaises herbes.